# EUROPEAN PATENT APPLICATION

(11) **EP 4 799 641 A1**
(43) Date of publication of application: **02.09.2026**
(21) Application number: 25161153.9
(22) Date of filing: 28.02.2025
(51) Int. Cl.: A61K 41/00, A61K 31/197, A61K 49/00, A61P 35/00, A61K 9/20, A61K 31/366, A61K 45/06

(54) **A BRAIN IMPLANT AND USE THEREOF**

(71) Applicant: JLP Health GmbH, 1130 Wien (AT)
(72) Inventor: The designation of the inventor has not yet been filed
(74) Representative: Arth, Hans-Lothar

(57) **Abstract**

The present invention relates to brain implant for linear release of 5-aminolevulinic acid comprising:
a) a core comprising:
5-aminolevulinic acid and a core polymer,
wherein the core polymer is selected from the group consisting of poly(lactic acid) (PLA), poly(ethylene oxide) (PEO), poly(ethylene glycol) (PEG), and poly(lactic-co-glycolic acid) (PLGA);
and

b) a shell consisting of an upper membrane, a spacer, and a lower membrane, wherein the upper membrane, the spacer, and the lower membrane each comprise a shell polymer, wherein the shell polymer is poly(lactic acid) (PLA);
characterized in that
the shell polymer is in a range from 20 wt.% to 60 wt.% based on the total weight of the brain implant;
and
when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core;
or
when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
a preparation method of said brain implant,
and
a medical use thereof, in particular in the prophylaxis and/or treatment of brain cancer.

## Description

### Field of the Invention

The present invention relates to a brain implant comprising
a) a core comprising:
   5-aminolevulinic acid and a core polymer,
   wherein the core polymer is selected from the group consisting of poly(lactic acid) (PLA), poly(ethylene oxide) (PEG), poly(ethylene glycol) (PEG), and poly(lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEG), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core, a preparation method of said brain implant, and a medical use thereof, in particular in the prophylaxis and/or treatment of brain cancer.

### Background of the Invention

There have been many therapeutic methods developed to treat brain cancer, including surgery, radiotherapy and chemotherapy. The present invention relates to pharmaceutical compositions that have activity as anti-cancer agents and to the methods for the treatment of cancer in patients.

Glioblastoma is an aggressive cancer that grows from the supportive cells in the brain and is diffusely infiltrative. The current standard treatment is aggressive surgical debulking followed by combined modality therapy of chemotherapy and radiation.

The only FDA-approved local delivery implant for GBM is the Gliadel^{®} wafer, a polyanhydride implant incorporating the anti-cancer agent carmustine (BCNU) into a 20:80 molar ratio copolymer matrix of 3-bis(p-carboxyphenoxy) propane (CPP) and sebacic acid (SA) (pCPP:SA (20:80)) (Dang et al., Pharmaceutical Research 1996, 13, 5, 683). While the wafers initially deliver a significant amount of BCNU, the rate of release declines rapidly within 5-7 days (Bastiancich et al., Journal of Controlled Release 2021, 337, 296-305). This decline, occurring even when the wafer remains in the cavity for months suggests the potential rapid diffusion of BCNU through the partially degraded polymer network. Given that the effectiveness of any anti-tumour agent is time-sensitive and depends upon the duration of exposure, the treatment outcome may be suboptimal (Tabet et al., Adv. Healthcare Mater. 2019, 8, 1801391). Other limitations include poor drug diffusion and fast initial release, a one-drug system, potential implant dislodgement, and the need for large resection cavities (Bastiancich et al., Journal of Controlled Release, 2016, 243, 29-42). Attempts to improve the efficacy of polymer-based implants involved loading wafers with various drugs like doxorubicin, temozolomide, epirubicin, z-butylidenephthalide, riluzole and memantine, and 3-bromopyruvate (3-BrPA) and dichloroacetate (DCA) (Bastiancich et al., 2021). However, the clinical significance of these formulations requires further investigation.

A combination of artemisinin derivatives and 5-aminolevulinic acid (5-ALA) has been shown to be effective for the treatment of brain cancer in preclinical models. While 5-ALA is used as imaging agent to mark malignant tissue during glioblastoma resection following oral application of a 20 mg/kg solution, multiple dosing has shown significant adverse events including strong phototoxicity and reduced liver function (Journal of Hepatology, 2003, 38, 476-482). These side effects limit the application of systemic 5-ALA delivery combined with artemisinin derivatives, particularly since a large amount of 5-aminolevulinic acid is needed to pass through the blood-brain barrier and reach the brain tumor tissues. Long-term local delivery of 5-ALA to the brain is complicated by 5-ALAs physiochemical properties: 5-ALA is very small and strongly hydrophilic. Therefore, in existing formulations, 5-aminolevulinic acid is rapidly released and/or shows a strong burst release within the first hours post hydration. Therefore, it is necessary to construct an implant with a continuous, slow release of 5-aminolevulinic acid (5-ALA) to reach constant 5-ALA level around the tumor tissues for up to several weeks

It is the objective of the present invention to provide a brain implant for linear release of 5-aminolevulinic acid (5-ALA) and a method for producing said brain implant.

The present invention provides a brain implant for linear release of 5-aminolevulinic acid (5-ALA), comprising a core comprising 5-aminolevulinic acid (5-ALA), a core polymer, and optionally an excipient in a core, and a shell consisting of an upper membrane, a spacer, and a lower membrane, and the upper membrane and the spacer, and the lower membrane each comprise a shell polymer, and optionally the upper and lower membranes each further comprise 5-aminolevulinic acid (5-ALA), or an excipient such as PEG.

This objective of the present invention is solved by the teachings of the independent claims. Further advantageous features, aspects and details of the invention are evident from the dependent claims, the description, the figures, and the examples of the present application.

### Description of the invention

The present invention provides a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

In another aspect, the present invention provides the brain implant as described herein, wherein the shell further comprises 5-aminolevulinic acid in a range from 1 wt.% to 15 wt.% based on the total weight of the shell.

In one embodiment, the core and/or the shell and preferably only the core of the brain implant comprises at least one excipient. In one embodiment, said at least one excipient is an antioxidant selected from ascorbic acid, acetylcysteine, cysteine, thioglycerol, sodium hydrogen sulfite (sodium bisulfite), butylated hydroxyanisole (BHA), butylated hydroxytoluene (BHT), α-tocopherol acetate (vitamin E acetate), methionine, citric acid, ethylenediaminetetraacetic acid (EDTA), tartaric acid, gallic acid and its esters, glutathione, uric acid, carotenoids, and polyphenols. Preferably, the antioxidant is selected from, but not limited to, ascorbic acid, acetylcysteine, cysteine, thioglycerol, sodium hydrogen sulfite, butyl-hydroxyanisole, butyl-hydroxytoluene, α-tocopherol acetate, methionine, and chelators. Further suitable excipients which could be present in the intracranial drug delivery system are organic acids selected from citric acid, fumaric acid, glycolic acid, lactic acid, malic acid, tartaric acid, maleic acid, succinic acid and mixtures of two or more of these organic acids.

In further aspect, the present invention provides the brain implant as described herein, further comprising an excipient in the core, or in the shell, wherein
the excipient in the core is selected from the group consisting of citric acid, fumaric acid, glycolic acid, lactic acid, malic acid, tartaric acid, maleic acid, succinic acid and a mixture thereof; and/or
the excipient in the shell is selected from polyethylene glycol (PEG).

In further aspect, the present invention provides the brain implant as described herein, wherein
the excipient in the core is in a range from 5 wt.% to 15 wt.%, based on the total weight of the core; or
the excipient in the shell is in a range from 35 wt.% to 45 wt.%, based on the total weight of the shell.

In further aspect, the present invention provides the brain implant as described herein, wherein the excipient in the shell is only contained in the upper membrane and/or the lower membrane of the shell.

In further aspect, the present invention provides the brain implant as described herein, the core is in a range from 40 wt.% to 75 wt. %, or 45 wt.% to 75 wt. %, or 45 wt.% to 70 wt.% based on the total weight of the brain implant.

In further aspect, the present invention provides the brain implant as described herein, consisting of:
a) the core consisting of:
   5-aminolevulinic acid in a range from 25 wt.% to 75 wt.%,
   the core polymer in a range from 75 wt.% to 25 wt.%, and
   optionally the excipient in the core in a range of 5 wt.% to 15 wt.%, based on the total weight of the core,
   wherein the excipient in the core is citric acid, fumaric acid or a mixture thereof; and
b) the shell consisting of:
   the upper membrane, the spacer, and the lower membrane each comprising a shell polymer in a range from 55 wt.% to 100 wt.%, and
   optionally, 5-aminolevulinic acid in a range from 1 wt.% to 15 wt.%, or
   the excipient in the shell in a range from 35 wt.% to 45 wt.% based on the total weight of the shell,
   wherein the excipient in the shell is PEG 400.

In further aspect, the present invention provides the brain implant as described herein, wherein
the brain implant consists of:
   a) the core consisting of:
      65 wt.% to 75 wt.% of 5-aminolevulinic acid, and 35 wt.% to 25 wt.% of poly (lactic-co-glycolic acid) (PLGA), based on a total weight of the core; and
   b) the shell consisting of poly(lactic acid) (PLA);
      wherein in the brain implant
      poly(lactic acid) (PLA) is in a range from 40 wt.% to 50 wt.%,
      based on a total weight of the brain implant;
         or
the brain implant consists of:
   a) the core consisting of:
      25 wt.% to 45 wt.% of 5-aminolevulinic acid and 75 wt.% to 55 wt.% of poly(lactic acid) (PLA), based on a total weight of the core; and
   b) the shell consisting of poly(lactic acid) (PLA);
      wherein in the brain implant
      and poly(lactic acid) (PLA) is in a range from 75 wt.% to 85 wt.%,
      based on a total weight of the brain implant;
         or
the brain implant consists of:
   a) the core consisting of:
      25 wt.% to 35 wt.% of 5-aminolevulinic acid, and 75 wt.% to 65 wt.% of poly(ethylene oxide) (PEO), based on a total weight of the core; and
   b) the shell consisting of poly(lactic acid) (PLA);
      wherein in the brain implant
      poly(lactic acid) (PLA) is in a range from 45 wt.% to 55 wt.%,
      based on a total weight of the brain implant;
         or
the brain implant consists of:
   a) the core consisting of:
      25 wt.% to 35 wt.% of 5-aminolevulinic acid, and 75 wt.% to 65 wt.% of poly(ethylene oxide) (PEO), based on a total weight of the core; and
   b) the shell consisting of poly(lactic acid) (PLA);
      wherein in the brain implant
      poly(lactic acid) (PLA) is in a range from 20 wt.% to 35 wt.%,
      based on a total weight of the brain implant;
         or
the brain implant consists of:
   a) the core consisting of:
      25 wt.% to 35 wt.% of 5-aminolevulinic acid, and 75 wt.% to 65 wt.% of poly (lactic-co-glycolic acid) (PLGA), based on a total weight of the core; and
   b) the shell consisting of:
      5 wt.% to 15 wt.% of 5-aminolevulinic acid, and 95 wt.% to 85 wt.% of poly(lactic acid) (PLA) based on a total weight of the shell;
      wherein in the brain implant
      poly(lactic acid) (PLA) is in a range from 40 wt.% to 50 wt.%,
      based on a total weight of the brain implant;
         or
the brain implant consists of:
   a) the core consisting of:
      25 wt.% to 30 wt.% of 5-aminolevulinic acid, 5 wt.% to 15 wt.% of citric acid or fumaric acid, and 55 wt.% to 70 wt.% of poly(lactic acid) (PLA) based on a total weight of the core; and
   b) the shell consisting of poly(lactic acid) (PLA);
      wherein in the brain implant
      poly(lactic acid) (PLA) is in a range from 75 wt.% to 85 wt.%,
      based on a total weight of the brain implant;
         or
the brain implant consists of:
   a) the core consisting of:
      25 wt.% to 35 wt.% of 5-aminolevulinic acid, and 75 wt.% to 65 wt.% of poly (lactic-co-glycolic acid) (PLGA) based on a total weight of the core; and
   b) the shell consisting of:
      35 wt.% to 45 wt.% of polyethylene glycol 400, and 65 wt.% to 55 wt.% of poly(lactic acid) (PLA) based on a total weight of the shell;
      wherein in the brain implant
      and poly(lactic acid) (PLA) is in a range from 25 wt.% to 35 wt.%,
      based on a total weight of the brain implant.

In one aspect a brain implant is disclosed herein, wherein
the core has a height (h₁) in a range from 1 mm to 3 mm;
the upper membrane has a height (h₂) in a range from 0.05 mm to 0.60 mm, preferably 0.10 mm to 0.50 mm; and
the lower membrane has a height (h₃) in a range from 0.05 mm to 0.60 mm, preferably 0.10 mm to 0.50 mm; and
the spacer has a thickness (t₄) of at least 0.5 mm, and is preferably in a range from 0.5 mm to 1.5 mm.

In further aspect, the present invention provides the brain implant as described herein preferably in form of a wafer;
the core has a diameter (d₁) in a range of 10 mm to 12 mm and a height (h₁) in a range from 1 mm to 3 mm;
the upper membrane has a diameter (d₂) in a range of 12 mm to 14 mm and a height (h₂) in a range from 0.05 mm to 0.60 mm, preferably 0.10 mm to 0.50 mm; and
the lower membrane has a diameter (d₃) in a range of 12 mm to 14 mm and a height (h₃) in a range from 0.05 mm to 0.60 mm, preferably 0.10 mm to 0.50 mm, and
the spacer has a diameter (d₄) in a range of 12 mm to 14 mm and a thickness (t₄) in a range from 0.5 mm to 1.5 mm; and a height (h₄) in a range from 1.0 mm to 3.0 mm.

The brain implant or the wafer has preferably a cylindrical form due to the manufacturing process. However, also any other form or shape like patches or pillows could be used.

In further aspect, the present invention relates to the brain implant as described herein for use in the prophylaxis and/or treatment of brain cancer.

In further aspect, the present invention relates to the brain implant as described herein for use, wherein the brain cancer is selected from subtypes proneural (PN), mesenchymal (MES), and classical (CL) glioblastoma, pilocytic astrocytoma, pleomorphic xanthoastrocytoma, subependymal giant cell astrocytoma, fibrillary astrocytoma, anaplastic astrocytoma, oligodendrogliomas, anaplastic oligodendroglioma, ependymoma, subependymoma, choroid plexus tumor, choroid plexus papilloma, and choroid plexus carcinoma

In further aspect, the present invention relates to the brain implant as described herein for use in a combination with one of the following artemisinin compounds (1a - 1e) or a pharmaceutically acceptable salt thereof 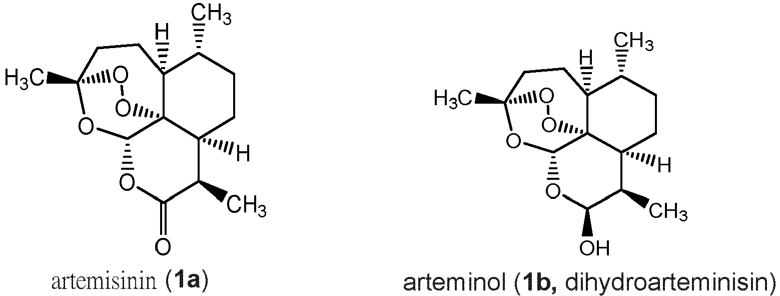 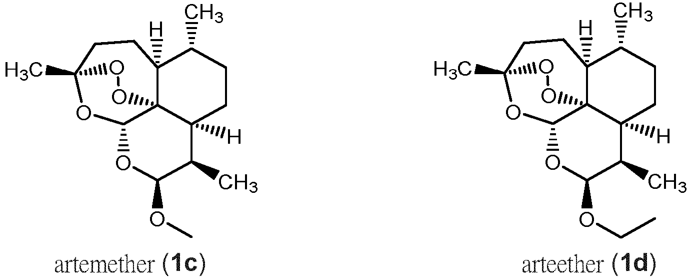 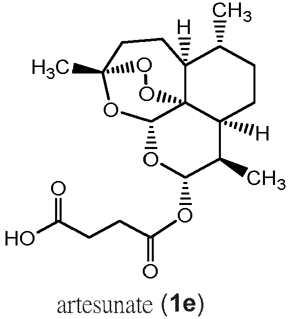 and preferably with artesunate (1e).

In further aspect, the present invention relates to the brain implant as described herein for use in combination with a radiotherapy, photodynamic therapy, immunotherapy, electromagnetic field therapy, hyperthermia therapy, chemotherapy, cancer immunotherapy, surgical therapy and/or cellular therapy such as Car-T and TIL. Preferred is a combination with chemotherapy, radiotherapy, and photodynamic therapy.

### Description of the invention

The present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based
   on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

The term "linear release" as used herein refers to the release of 5-ALA over a certain period of time, preferably over at least 3 days, more preferably over at least 4 days, and still more preferably over at least 5 days in a linear manner. The term "linear release" refers to a gradual release of 5-ALA into the resection cavity and/or the brain liquid, i.e. the cerebrospinal fluid, at a steady state over the time, i.e. the amount of 5-ALA released per unit of time is proportional to the amount of 5-ALA remaining in the brain implant in contrast to a bolus release which follows a Gaussian distribution. Thus, in order to obtain a consistent pharmacokinetic effect, a bolus release has to be avoided and a linear release is required in order to ensure a constant 5-ALA level around the brain implant in the cavities emerging from the brain surgery and/or intratumorally, releasing 5-ALA from the brain implant at a rate which does not change or at least not significantly change over a period of time of preferably at least 3 days, more preferably of at least 4 days, and still more preferably of at least 5 days. Thus, the linear release follows a zero-order or near zero-order release kinetic.

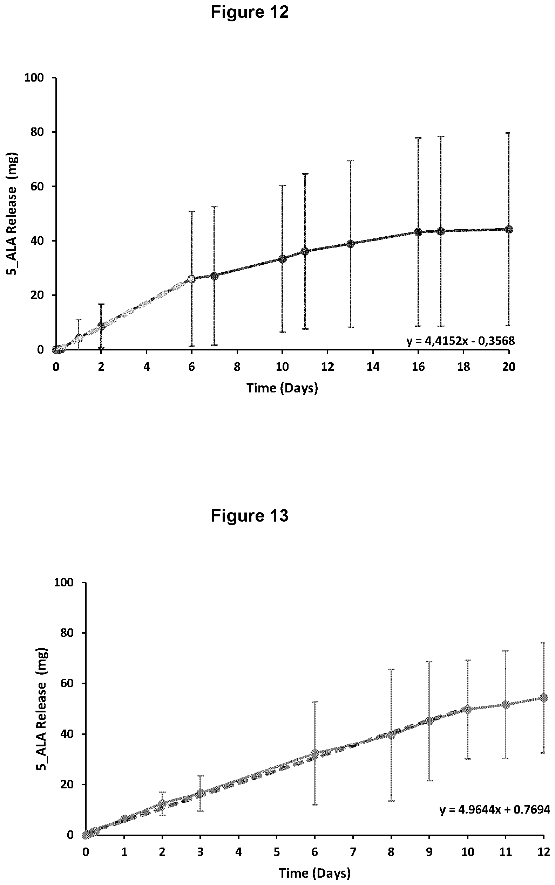

The term "continuous release" or "continuously released" refers to a release kinetic which starts with the linear release which goes over to a decreasing release of 5-ALA until all releasable 5-ALA is released from the brain implant. A typical continuous release kinetic is shown, for instance, in Figures 13, 17 and 19. The first 8 to 10 days represent the linear release which is followed by a decreasing release over the following days (about 10 days) until all 5-ALA is released. Consequently, the continuous release covers the first 8 to 10 days of linear release and the subsequent about 10 days of decreased release of 5-ALA.

The linear release of 5-ALA avoids the delivery of a bolus of 5-ALA. Severe side effects were observed in case of in vivo administration of a single, concentrated dose of at least 1 mg of 5-ALA over a short period of time of several minutes. The linear and continuous release of 5-ALA as used herein particularly does not refer to the administration of a single, concentrated dose of at least 1 mg of 5-ALA within seconds to 10 minutes, preferably within 1 to 10 minutes. This includes also a daily bolus application of 5-ALA. Thus, linear and continuous release as used herein does not refer to the daily administration of a single dose of at least 1 mg of 5-ALA within 1 to 5 minutes. The conclusion can be drawn from the mouse data that a bolus administration of 1.00 mg or more of 5-ALA administered within 5 minutes or less should strictly be avoided in order to avoid toxic side effects of the 5-ALA administration.

The brain implants of the present invention provide a linear release of 5-ALA over 3 or 4 or 5 or more days. During this linear release preferably 1 mg to 30 mg, more preferably 2 mg to 20 mg, more preferably 3 mg to 15 mg of 5-ALA are released into the cavities emerging from the brain surgery, i.e. the resection cavities and intratumoral, i.e. into the remaining tumor cells, in case the tumor was not removed completely. Subsequently, 5-ALA might still be released but not necessarily in a linear manner. However, 5-ALA is released in a continuous manner over the time and this continuous release includes the time of the linear release. This release kinetic is crucial and an important aspect of the brain implants of the present invention.

Consequently, the present invention is directed also to brain implants for linear release of 5-aminolevulinic acid wherein the brain implant releases 5-aminolevulinic acid linearly, i.e. with zero-order or near zero-order release kinetic, over at least 3 days in an amount of 1 mg to 30 mg, preferably in an amount of 2 mg to 20 mg, more preferably in an amount of 3 mg to 15 mg into cavities emerging from a brain surgery.

In the present invention, a spacer and a lower membrane may form together a vessel, or in other words, a vessel consists of a spacer and a lower membrane.

Thus, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a vessel consisting of a spacer, and a lower membrane, wherein the upper membrane, and the vessel each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

Preferably, the present invention refers to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt. %, preferably 26 wt.% to 55 wt. %, preferably 27 wt. % to 55 wt. %, more preferably 28 wt.% to 54 wt%, most preferably 30 wt.% to 50 wt% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 20 wt.% to 50 wt. %, preferably 26 wt.% to 43 wt. %, preferably 26 wt.% to 42 wt.%, most preferably 26 wt.% to 41 wt.% and the core polymer in a range from 80 wt.% to 50 wt.%, preferably 74 wt.% to 56 wt.%, preferably 73 wt.% to 57 wt.%, 72 wt.% to 58 wt.%, most preferably 71 wt.% to 59 wt.%, based on the total weight of the core; or
   when the core polymer is poly(lactic-co-glycolic acid) (PLGA), the core contains 5-aminolevulinic acid in a range from 60 wt.% to 80 wt.%, preferably 66 wt.% to 74 wt.% , preferably 67 wt.% to 73 wt. %, 68 wt.% to 72 wt. %, most preferably 69 wt.% to 71 wt.%, and the core polymer in a range from 40 wt.% to 20 wt.%, preferably 34 wt.% to 26 wt.% preferably 33 wt.% to 27 wt. %, 32 wt.% to 28 wt. %, most preferably 31 wt.% to 29 wt.%, based on the total weight of the core.

Preferably, the present invention refers to the brain implant for linear release of 5-aminolevulinic acid, wherein the shell further comprises 5-aminolevulinic acid in a range from 1 wt.% to 15 wt.% based on the total weight of the shell.

Thus, preferably the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA); wherein the shell further comprises 5-aminolevulinic acid in a range from 1 wt.% to 15 wt.% based on the total weight of the shell;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer and optinally 5-aminolevulinic acid, wherein the shell polymer is poly(lactic acid) (PLA);
   wherein 5-aminolevulinic acid is in a range from 1 wt.% to 15 wt.% based on the total weight of the shell;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

More preferably, the present invention refers to the the brain implant for linear release, wherein the shell further comprises 5-aminolevulinic acid in a range of 4 wt.% to 12 wt.% , preferably 5 wt.% to 12 wt. %, 5 wt.% to 11 wt. %, most preferably 6 wt.% to 10 wt. %, based on the total weight of the shell.

Thus, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA); wherein the shell further comprises 5-aminolevulinic acid in a range from 4 wt.% to 12 wt.% , preferably 5 wt.% to 12 wt. %, 5 wt.% to 11 wt. %, most preferably 6 wt.% to 10 wt.%, based on the total weight of the shell;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer and optinally 5-aminolevulinic acid, wherein the shell polymer is poly(lactic acid) (PLA);
   wherein 5-aminolevulinic acid is in a range from 4 wt.% to 12 wt.% , preferably 5 wt.% to 12 wt.%, 5 wt.% to 11 wt.%, most preferably 6 wt.% to 10 wt.%, based on the total weight of the shell;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

More preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
      and
b) a shell consisting of an upper membrane, a spacer, and a lower membrane, wherein the upper membrane, the spacer, and the lower membrane each comprise a shell polymer, wherein the shell polymer is poly(lactic acid) (PLA); wherein the shell further comprises 5-aminolevulinic acid in a range from 4 wt.% to 12 wt.% , preferably 5 wt.% to 12 wt. %, 5 wt.% to 11 wt. %, most preferably 6 wt.% to 10 wt.%, based on the total weight of the shell;
   characterized in that
   the shell polymer is in a range from 25 wt.% to 55 wt.%, 26 wt.% to 55 wt.%, preferably 27 wt.% to 55 wt.%, more preferably 28 wt.% to 54 wt%, most preferably 30 wt.% to 50 wt% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 26 wt.% to 43 wt.%, preferably 26 wt.% to 42 wt. %, most preferably 26 wt.% to 41 wt. % and the core polymer in a range from 74 wt.% to 56 wt.%, preferably 73 wt.% to 57 wt. %, 72 wt.% to 58 wt. %, most preferably 71 wt.% to 59 wt. %, based on the total weight of the core; or
   when the core polymer is poly(lactic-co-glycolic acid) (PLGA), the core contains 5-aminolevulinic acid in a range from 66 wt.% to 74 wt.% , preferably 67 wt.% to 73 wt. %, 68 wt.% to 72 wt. %, most preferably 69 wt.% to 71 wt. %, and the core polymer in a range from 34 wt.% to 26 wt.% preferably 33 wt.% to 27 wt. %, 32 wt.% to 28 wt.%, most preferably 31 wt.% to 29 wt.%, based on the total weight of the core.

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer and optinally 5-aminolevulinic acid, wherein the shell polymer is poly(lactic acid) (PLA);
   wherein 5-aminolevulinic acid is in a range from 4 wt.% to 12 wt.% , preferably 5 wt.% to 12 wt.%, 5 wt.% to 11 wt.%, most preferably 6 wt.% to 10 wt.%, based on the total weight of the shell;
   characterized in that
   the shell polymer is in a range from 25 wt.% to 55 wt.%, 26 wt.% to 55 wt.%, preferably 27 wt.% to 55 wt.%, more preferably 28 wt.% to 54 wt%, most preferably 30 wt.% to 50 wt% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 26 wt.% to 43 wt.%, preferably 26 wt.% to 42 wt. %, most preferably 26 wt.% to 41 wt. % and the core polymer in a range from 74 wt.% to 56 wt.%, preferably 73 wt.% to 57 wt. %, 72 wt.% to 58 wt. %, most preferably 71 wt.% to 59 wt. %, based on the total weight of the core; or
   when the core polymer is poly(lactic-co-glycolic acid) (PLGA), the core contains 5-aminolevulinic acid in a range from 66 wt.% to 74 wt.% , preferably 67 wt.% to 73 wt. %, 68 wt.% to 72 wt. %, most preferably 69 wt.% to 71 wt. %, and the core polymer in a range from 34 wt.% to 26 wt.% preferably 33 wt.% to 27 wt. %, 32 wt.% to 28 wt.%, most preferably 31 wt.% to 29 wt.%, based on the total weight of the core.

Still more preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA); wherein the shell further comprises 5-aminolevulinic acid in a range from 5 wt.% to 11 wt.%, most preferably 6 wt.% to 10 wt.%, based on the total weight of the shell;
   characterized in that
   the shell polymer is in a range from 28 wt.% to 54 wt%, most preferably 30 wt.% to 50 wt% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 26 wt.% to 42 wt.%, most preferably 26 wt.% to 41 wt.% and the core polymer in a range from 72 wt.% to 58 wt. %, most preferably 71 wt.% to 59 wt. %, based on the total weight of the core; or
   when the core polymer is poly(lactic-co-glycolic acid) (PLGA), the core contains 5-aminolevulinic acid in a range from 68 wt.% to 72 wt.%, most preferably 69 wt.% to 71 wt.%, and the core polymer in a range from 32 wt.% to 28 wt.%, most preferably 31 wt.% to 29 wt.%, based on the total weight of the core.

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer and optinally 5-aminolevulinic acid, wherein the shell polymer is poly(lactic acid) (PLA);
   wherein 5-aminolevulinic acid is in a range from 4 wt.% to 12 wt.% , preferably 5 wt.% to 12 wt.%, 5 wt.% to 11 wt.%, most preferably 6 wt.% to 10 wt.%, based on the total weight of the shell;
   characterized in that
   the shell polymer is in a range from 25 wt.% to 55 wt.%, 26 wt.% to 55 wt.%, preferably 27 wt.% to 55 wt.%, more preferably 28 wt.% to 54 wt%, most preferably 30 wt.% to 50 wt% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 26 wt.% to 43 wt.%, preferably 26 wt.% to 42 wt. %, most preferably 26 wt.% to 41 wt. % and the core polymer in a range from 74 wt.% to 56 wt.%, preferably 73 wt.% to 57 wt. %, 72 wt.% to 58 wt. %, most preferably 71 wt.% to 59 wt. %, based on the total weight of the core; or
   when the core polymer is poly(lactic-co-glycolic acid) (PLGA), the core contains 5-aminolevulinic acid in a range from 66 wt.% to 74 wt.% , preferably 67 wt.% to 73 wt. %, 68 wt.% to 72 wt. %, most preferably 69 wt.% to 71 wt. %, and the core polymer in a range from 34 wt.% to 26 wt.% preferably 33 wt.% to 27 wt. %, 32 wt.% to 28 wt.%, most preferably 31 wt.% to 29 wt.%, based on the total weight of the core.

Preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer and optinally 5-aminolevulinic acid, wherein the shell polymer is poly(lactic acid) (PLA);
   wherein 5-aminolevulinic acid is in a range from 5 wt.% to 11 wt.%, most preferably 6 wt.% to 10 wt.%, based on the total weight of the shell;
   characterized in that
   the shell polymer is in a range from 28 wt.% to 54 wt%, most preferably 30 wt.% to 50 wt% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 26 wt.% to 42 wt.%, most preferably 26 wt.% to 41 wt.% and the core polymer in a range from 72 wt.% to 58 wt. %, most preferably 71 wt.% to 59 wt. %, based on the total weight of the core; or
   when the core polymer is poly(lactic-co-glycolic acid) (PLGA), the core contains 5-aminolevulinic acid in a range from 68 wt.% to 72 wt.%, most preferably 69 wt.% to 71 wt.%, and the core polymer in a range from 32 wt.% to 28 wt.%, most preferably 31 wt.% to 29 wt.%, based on the total weight of the core.

The core polymer may be selected from biodegradable or bioresorbable polymers comprising one or more residues of lactic acid, glycolic acid, lactide, glycolide or a combination thereof.

The core polymer can comprise any lactide residue, Poly(lactic acid) has the following chemical forumular and is also known as poly(lactic acid) or poly(D,L-lactide), or polylactide, including all racemic and stereospecific forms of lactide, including, but not limited to, L-lactide, D-lactide, and D,L-lactide, or a mixture thereof.

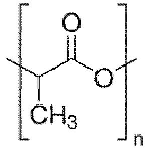

Poly(lactic acid) include, but are not limited to poly(L-lactide), poly(D-lactide), and poly(D,L-lactide), preferably poly(D,L-lactide). Preferably, the poly(lactic acid) as used in the present invention, has M_{w} from 5 to 35 kDa, preferably, M_{w} from 7 to 32 kDa, preferably, M_{w} from 8 to 30 kDa, more preferably, M_{w} from 9 to 29 kDa, most preferably, M_{w} from 10 to 28 kDa.

For example, Resomer ^{®} R 202 H has M_{w} from 10 to 18 kDa. Resomer ^{®} R 202 S has M_{w} from 10 to 18 kDa; Resomer ^{®} R 203 H has M_{w} from 18 to 24 kDa. Resomer ^{®} R 203 S has M_{w} from 18 to 28 kDa.

The PLA has an inherent viscosity of 0.1 to 1.2 dL/g, when measured at 25°C in chloroform at a concentration of 0.5 g/dL. Preferably, the PLGA has an inherent viscosity of 0.1 to 1.0 dL/g, 0.1 to 0.8 dL/g, 0.1 to 0.6 dL/g, more preferably 0.1 to 0.4 dL/g, most prefearbly 0.16 to 0.35 dL/g when measured at 25 °C in chloroform at a concentration of 0.5 g/dL.

RESOMER^{®} R as used herein is a platform of PLA / poly (D,L-lactide)-based biodegradable polymers for controlled release. Designed, in particular, for microparticles and drug-loaded implants, these amorphous polymers are available with either acid or ester end groups. Degradation times can vary from as little as a few weeks or less to nine months or more. Inherent Viscosities (IV) can range from 0.15 to 0.75 dL / g, with molecular weights between 10,000 and 28,000 Daltons. Polymer properties including crystallinity can be easily tuned to match specific formulation and release profile requirements.

| Polymer name | Inherent Viscosity (dl/g) | Compostion | End Group |
|---|---|---|---|
| RESOMER^{®}R 202H | 0.16 - 0.24 | Poly(*D,L*-lactide) | Acid |
| RESOMER^{®}R 202S | 0.16 - 0.24 | Poly(*D,L*-lactide) | Ester |
| RESOMER^{®}R 203H | 0.25 - 0.35 | Poly(*D,L*-lactide) | Acid |
| RESOMER^{®}R 203S | 0.25 - 0.35 | Poly(*D,L*-lactide) | Ester |

Poly(lactic-co-glycolic acid) (PLGA) is a copolymer of poly(lactic acid) (PLA) and poly glycolic acid (PGA), or poly(lactide-co-glycolide) is also uased as a synonym of PLGA. It is the best defined biomaterial available for drug delivery with respect to design and performance. Poly(lactic acid) contains an asymmetric α-carbon which is typically described as the D or L form in classical stereochemical terms and sometimes as R and S form, respectively. The enantiomeric forms of the polymer PLA are poly D-lactic acid (PDLA) and poly L-lactic acid (PLLA). PLGA is generally an acronym for poly (D,L-lactic-co-glycolic acid), or poly(D,L-lactide-co-glycolide), where D- and L- lactic acid forms are in equal ratio.

Preferably, the PLGA has a lactic acid weight content of 10 to 90% with the balance being glycolic acid. Preferably, the PLGA has a lactic acid weight content of 20 to 80%, 30 to 70%, preferably 40 to 60%, more preferably 45 to 55%; with the balance being glycolic acid.

Preferably, the PLGA has a lactic acid:glycolic acid weight ratio of 5:95, 15:85, 25:75, 40:60, 45:55, 50:50, 55:45, 60:40, 75:25, 85:15 or 95:5. In a preferred embodiment, the PLGA has a lactic acid:glycolic acid weight ratio from 40:60 to 60:40, preferably to 45:55 to 55:45, still more preferably to 47:53 to 53:47, most preferably 50:50. More preferrably, the PLGA has acid as end group and has a lactic acid:glycolic acid weight ratio of about 50:50. Preferably, the PLGA has a number average molecular weight of about 2 to 15 kDa, such as about 5 to 15 kDa, or about 5 to 12 kDa.

More preferably, the PLGA has a lactic acid:glycolic acid weight ratio ratio from 40:60 to 60:40, 45:55 to 55:45, preferably to 47:53 to 53:47, most preferaubly 50:50 and a number average molecular weight (M_{w}) from 7 to 240 kDa, 7 to 100 kDa, 7 to 50 kDa, 7 to 40 kDa, 7 to 30 kDa, more preferably 7 to 20 kDa, or most preferably 7 to 17 kDa. For example, Resomer ^{®} RG 502H has M_{w} from 7 to 17 kDa.

Preferably, the PLGA has an inherent viscosity of 0.15 to 1.2 dL/g, when measured at 25 °C in chloroform at a concentration of 0.5 g/dL. In a preferred embodiment, the PLGA has an inherent viscosity of 0.2 to 1.2 dL/g, such as 0.25 to 1.2 dL/g, 0.3 to 1.0 dL/g, 0.3 to 0.8 dL/g, or 0.3 to 0.6 dL/g when measured at 25 °C in chloroform at a concentration of 0.5 g/dL. In a most preferred embodiment, the PLGA has an inherent viscosity of 0.3 to 0.5 dL/g when measured at 25 °C in chloroform at a concentration of 0.5 g/dL.

Preferably, the PLGA has an inherent viscosity of 0.1 to 1.2 dL/g, when measured at 25 °C in chloroform at a concentration of 0.5 g/dL. Preferably, the PLGA has an inherent viscosity of 0.1 to 1.0 dL/g, 0.1 to 0.8 dL/g, 0.1 to 0.6 dL/g, 0.1 to 0.4 dL/g, more preferably 0.1 to 0.3 dL/g, most prefearbly 0.16 to 0.24 dL/g when measured at 25 °C in chloroform at a concentration of 0.5 g/dL.

Preferably, the PLGA has a lactic acid:glycolic acid weight ratio of 40:60 to 60:40, a number average molecular weight of 7 to 30 kDa and an inherent viscosity of 0.1 to 0.3 dl/g, when measured at 25 °C in chloroform at a concentration of 0.5 g/dL.

More preferably, the PLGA has a lactic acid:glycolic acid weight ratio of 45:55 to 55:45, a number average molecular weight of 7 to 20 kDa and an inherent viscosity of 0.1 to 0.3 dl/g, when measured at 25 °C in chloroform at a concentration of 0.5 g/dL.

More preferably, the PLGA has a lactic acid:glycolic acid weight ratio of 47:53 to 53:47, a number average molecular weight of 7 to 20 kDa and an inherent viscosity of 0.1 to 0.3 dl/g, when measured at 25 °C in chloroform at a concentration of 0.5 g/dL.

Most preferably, the PLGA has a lactic acid:glycolic acid weight ratio 50:50, a number average molecular weight of 7 to 17 kDa and an inherent viscosity of 0.16 to 0.24 d_/g, when measured at 25 °C in chloroform at a concentration of 0.5 g/dL.

RESOMER^{®} RG is a platform of PLGA / poly (D,L-lactide-co-glycolide)-based bioresorbable excipients for controlled release. Designed for use with a range of complex parenteral drug products, these amorphous polymers are also available with either acid or ester end groups. Standard mole ratios include 50:50, 65:35, 75:25 and 85:15. Degradation times can extend for up to 18 months or more. Inherent Viscosities (IV) can range from 0.09 to 1.7 dL / g, with molecular weights between 7,000 and 240,000 Daltons. Polymer properties including crystallinity can be easily tuned to match specific formulation and release profile requirements.

| Polymer name | Inherent Viscosity (dl/g) | Compostion | End Group |
|---|---|---|---|
| RESOMER^{®} RG 502 | 0.16 - 0.24 | Poly(D, L-lactide-co-glycolide) 50:50 | Ester |
| RESOMER^{®} RG 502H | 0.16 - 0.24 | Poly(D, L-lactide-co-glycolide) 50:50 | Acid |

### PEO: poly(ethylene oxide)

The pharmaceutical formulation of the present application may comprises poly(ethylene oxide), abbreviated as PEO.

Polyethylene glycol has the following chemical structure 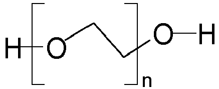 wherein n indicates the number of repeating units.

Preferably, the PEO has the average molecular weight (M_{w}) from 10 to 300 kDa, 50 to 300 kDa, 100 to 300 kDa, 150 to 250 kDa, 160 to 240 kDa, 170 to 230 kDa, 180 to 220 kDa, more preferably 190 to 210 kDa, or most preferably 200 kDa. For example, PEO as used herein has M_{w} = 200 kDa.

### PEG: polyethylene glycol

The pharmaceutical formulation of the present application may comprises polyethylene glycol, abbreviated as PEG.

Polyethylene glycol has the following chemical structure 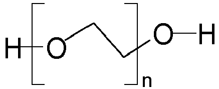 wherein n indicates the number of repeating units.

The chemical formula is C₂ₙH₄ₙ₊₂Oₙ₊₁ and the density is 1.125 g/mL.

Other IUPAC names of PEG are poly(oxyethylene) or poly(ethylene oxide). PEG is also known under the trademarks Carbowax^{™}, Kollisolv^{®}, Kolliphor^{®}, Polyglycol^{™} and the Ph. Eur. (Pharmacopoea Europaea) name Macrogol.

Several different polyethylene glycols such as PEG 200, PEG 300, PEG 400, PEG 600, PEG 1000, PEG 1500, PEG 2000, PEG 3000, PEG 4000, PEG 6000, PEG 8000 and so on are known.

The numbers in the names of the PEGs indicate their average molecular weight, e.g. a PEG with n = 9 has an average molecular weight of approximately 400 daltons, and is usually designated as PEG 400. Most PEGs include molecules with a distribution of molecular weights, i.e. they are polydisperse.

The size distribution can be characterized statistically by its weight average molecular weight (Mw) and its number average molecular weight (Mn), the ratio of which is called the polydispersity index (Mw/Mn). Mw and Mn can be measured by mass spectrometry.

It was found that polyethylene glycols having an average molecular weight in the range of from 380 g/mol to 420 g/mol are suitable for the present brain implant. The use of PEG 400 is particularly preferred for the present brain implant.

However, it is also possible to use mixtures of PEGs for the brain implant for linear release of 5-aminolevulinic acid. PEGs having an average molecular weight in the range of from 380 g/mol to 420 g/mol are at room temperature non-volatile liquids.

The terms "in the range of from *'value A'* to *'value B'"* and "an amount of from *'value A'* to *'value B'"* as used throughout the present application refers to a continuous group of possible values, wherein *'value A'* represents the lower end of said group and *'value B'* represents the upper end of said group. Values representing said lower and said upper ends are included in said group of possible values. For example, PEGs having an average molecular weight in the range of from 380 g/mol to 420 g/mol include PEGs having an average molecular weight of 380 g/mol and PEGs having an average molecular weight of 420 g/mol as well as PEGs having average molecular weights which are in between.

The term "room temperature" as used herein, is synonymous to the term "standard room temperature" and refers to a temperature in the range of from 19°C to 26°C. For example, PEGs which are "at room temperature non-volatile liquids" means that said PEGs are non-volatile liquids "at a temperature in the range of from 19°C to 26°C".

Therefore, it is also in accordance with the present invention to use mixtures of two, three or more PEGs in the brain implant. For instance, mixtures of PEG 300 and PEG 400, or PEG 200 and PEG 400, or PEG 200 and PEG 300 and PEG 400, and so on.

The average molecular weight of all mixtures of PEGs used for the present can be in the range of from 250 g/mol to 450 g/mol, preferably in the range of from 300 g/mol to 450 g/mol, and more preferably in the range of from 350 g/mol to 450 g/mol, and most preferably in the range of from 380 g/mol to 420 g/mol.

Polyethylene glycol (PEG) is a polyether compound. PEG is also known as poly(ethylene oxide) (PEO) or polyoxyethylene (POE), depending on its molecular weight. The structure of PEG is commonly expressed as H-(O-CH₂-CH₂)ₙ-OH. **PEG 400** (polyethylene glycol 400) is a low-molecular-weight grade of polyethylene glycol and has a molecular weight (Mₙ) 380-420 g/mol and average molecular weight(Mₙ) 400 g/mol.

The term "**weight percent**" (abbreviation: wt.%), as used herein, refers to the proportion of a substance in a mixture measured in grams per 100 g of mixture. The term weight percent, as used herein, is a designation for the mass fraction of a mixture.

The disclosure of a component, such as the core, consist of 25 wt.% to 75 wt.% of compound A and 75 wt.% to 25 wt.% of compound B has to be understood in the way that both compounds together have to add up to 100 wt.%.

Consequently, the disclosure
a) a core consisting of:
   5-aminolevulinic acid in a range from 25 wt.% to 75 wt.%,
   the core polymer in a range from 75 wt.% to 25 wt.%, and
   optionally the excipient in the core in a range of 5 wt.% to 15 wt.%, based on the total weight of the core,
   has to be understood in the way that in case the excipient is present, e.g. in an amount of 15 wt.%, the remaining 5-ALA and core polymer have to add up to 85 wt.% so that all ingredients together add up to 100 wt.%. Consequently, the core polymer cannot be present in the amount of 75 wt.% anymore, because 5-ALA must be present at least in the amount of 25 wt.% so that the maximum amount of the core polymer is 60 wt.%.

The term "**mass ratio**", as used herein, refers to a physicochemical quantity for the quantitative description of the composition of mixtures of substances. The mass ratio indicates the ratio of the masses of two considered mixture components to each other.

The mass ratio (m/m; [g]/[g]; m=mass) of the core to the shell is preferably 5:1 - 1:5, more preferably 3:1 - 1:3, still more preferably 2:1 - 1:2, still more preferably 2:1 - 1:1, and more preferably 1.5:1 - 1:1, and most preferably 1:1. The mass of the core here refers to the total mass of 5-aminolevulinic acid, a core polymer, and optionally the excipient in the core. The mass of the shell here refers to the total mass of the upper membrane, the spacer and the lower membrane (i.e. the upper membrane and the vessel consisting of the spacer and the lower membrane) comprising the shell polymer and optionally 5-aminolevulinic acid or the excipient in the shell.

The excipient in the core as described herein is a pH-regulator. The pH-regulator includes, but not limited to organic mono-carboxy acids such as glycolic acid, lactic acid, organic dicarboxylic acid such as oxalic acid, malonic acid, succinic acid, glutaric acid, tartaric acid, fumaric acid, maleic acid, malic acid, adipic acid (hexanedioic acid), or glutamic acid, and organic tri-carboxylic acid such as citric acid, sodium hydrogen citrate, or a mixture thereof.

Preferably, the excipient in the core is selected from acids comprising or consisting of glycolic acid, lactic acid, oxalic acid, malonic acid, succinic acid, glutaric acid, tartaric acid, fumaric acid, maleic acid, malic acid, adipic acid (hexanedioic acid), glutamic acid; citric acid, sodium hydrogen citrate, and a mixture thereof.

More preferably, the pH-regulator is selected from the group comprising or consisting of glycolic acid, lactic acid, malonic acid, succinic acid, glutaric acid, tartaric acid, fumaric acid, maleic acid, malic acid, citric acid, sodium hydrogen citrate, and a mixture thereof. Still more preferably, the acid is selected from the group consisting of malonic acid, succinic acid, fumaric acid, maleic acid, malic acid, citric acid, sodium hydrogen citrate, glycolic acid, lactic acid, and a mixture thereof.

More preferably, the excipient in the core as the pH-regulator is selected from the group consisting of citric acid, fumaric acid, glycolic acid, lactic acid, malic acid, tartaric acid, maleic acid, succinic acid and a mixture thereof; most preferably citric acid, fumaric acid and a mixture thereof.

Thus, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and
   wherein the brain implant further comprises an excipient in the core,
   wherein the excipient in the core is selected from the group consisting of citric acid, fumaric acid, glycolic acid, lactic acid, malic acid, tartaric acid, maleic acid, succinic acid and a mixture thereof; preferably, citiric acid, fumaric acid, and a mixture thereof.

**In** other words, the present invention relates to a brain implant for linear release of 5-aminolevulinic acid comprising
a) a core comprising:
   5-aminolevulinic acid, a core polymer, and an excipient;
   wherein the core polymer is selected from the group consisting of poly(lactic acid) (PLA), poly(ethylene oxide) (PEO), poly(ethylene glycol) (PEG), and poly(lactic-co-glycolic acid) (PLGA),
   the excipient in the core is selected from the group consisting of citric acid, fumaric acid, glycolic acid, lactic acid, malic acid, tartaric acid, maleic acid, succinic acid and a mixture thereof; preferably, citiric acid, fumaric acid, and a mixture thereof;
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

Preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA); wherein the shell further comprises 5-aminolevulinic acid in a range from 1 wt.% to 15 wt.% based on the total weight of the shell;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core, and wherein the brain implant further comprises an excipient in the core, the excipient in the core is selected from the group consisting of citric acid, fumaric acid, glycolic acid, lactic acid, malic acid, tartaric acid, maleic acid, succinic acid and a mixture thereof; preferably, citiric acid, fumaric acid, and a mixture thereof.

**In** other words, the present invention relates to a brain implant for linear release of 5-aminolevulinic acid comprising
a) a core comprising:
   5-aminolevulinic acid, a core polymer, and an excipient;
   wherein the core polymer is selected from the group consisting of poly(lactic acid) (PLA), poly(ethylene oxide) (PEO), poly(ethylene glycol) (PEG), and poly(lactic-co-glycolic acid) (PLGA),
   the excipient in the core is selected from the group consisting of citric acid, fumaric acid, glycolic acid, lactic acid, malic acid, tartaric acid, maleic acid, succinic acid and a mixture thereof; preferably, citiric acid, fumaric acid, and a mixture thereof;
   and
b) a shell consisting of an upper membrane, a spacer, and a lower membrane, wherein the upper membrane, the spacer, and the lower membrane each comprise a shell polymer and optinally 5-aminolevulinic acid,
   wherein the shell polymer is poly(lactic acid) (PLA);
   wherein 5-aminolevulinic acid is in a range from 1 wt.% to 15 wt.% based on the total weight of the shell;
      characterized in that
      the shell polymer is in a range from 20 wt.% to 60 wt.%, preferably 25 wt.% to 55 wt.% based on the total weight of the brain implant; and
      when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
      when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

Alternatively, the brain implant of the invention further comprises an excipient in the shell. Preferably, the excipient in the shell is polyethylene glycol (PEG), preferably PEG 200, PEG 300, PEG 400, PEG 600, more preferably PEG 300, PEG 400, most preferably PEG 400.

The average molecular weight of all mixtures of PEGs used for the present pharmaceutical formulation can be in the range of from 250 g/mol to 450 g/mol, preferably in the range of from 300 g/mol to 450 g/mol, and more preferably in the range of from 350 g/mol to 450 g/mol, and most preferably in the range of from 380 g/mol to 420 g/mol.

Thus, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and
   wherein the brain implant further comprises an excipient in the shell,
   wherein the excipient in the shell is selected from polyethylene glycol (PEG).

In other words, the present invention relates to a brain implant for linear release of 5-aminolevulinic acid comprising
a) a core comprising:
   5-aminolevulinic acid, and a core polymer;
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer and an excipient,
   wherein the shell polymer is poly(lactic acid) (PLA),
   the excipient in the shell is selected from polyethylene glycol (PEG);
      characterized in that
      the shell polymer is in a range from 20 wt.% to 60 wt.%, preferably 25 wt.% to 55 wt.% based on the total weight of the brain implant; and
      when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
      when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

Preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and wherein the brain implant further comprises an excipient in the core, or in the shell, the excipient in the core is selected from the group consisting of citric acid, fumaric acid, glycolic acid, lactic acid, malic acid, tartaric acid, maleic acid, succinic acid and a mixture thereof; preferably citric acid, fumaric acid, and a mixture thereof and/or the excipient in the shell is selected from polyethylene glycol (PEG).

More preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and
   wherein the brain implant further comprises an excipient in the core, or in the shell, the excipient in the core is selected from the group consisting of citric acid, fumaric acid, and a mixture thereof and/or
   the excipient in the shell is selected from PEG 300, PEG 400, PEG 600, more preferably PEG 300, PEG 400, most preferably PEG 400.

Preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and
   wherein the shell further comprises 5-aminolevulinic acid in a range from 1 wt.% to 15 wt.% based on the total weight of the shell;
   wherein the brain implant further comprises an excipient in the core, or in the shell; the excipient in the core is selected from the group consisting of citric acid, fumaric acid, glycolic acid, lactic acid, malic acid, tartaric acid, maleic acid, succinic acid and a mixture thereof; preferably citric acid, fumaric acid, and a mixture thereof and/or the excipient in the shell is selected from polyethylene glycol (PEG).

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer and optionally 5-aminolevulinic acid, wherein the shell polymer is poly(lactic acid) (PLA);
   wherein 5-aminolevulinic acid is in a range from 1 wt.% to 15 wt.% based on the total weight of the shell;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and wherein the brain implant further comprises an excipient in the core, or in the shell;
   the excipient in the core is selected from the group consisting of citric acid, fumaric acid, glycolic acid, lactic acid, malic acid, tartaric acid, maleic acid, succinic acid and a mixture thereof; preferably citric acid, fumaric acid, and a mixture thereof and/or the excipient in the shell is selected from polyethylene glycol (PEG).

In some preferred embodiments, the brain implant further comprises the excipient in the core, or in the shell, wherein the excipient in the core is in a range of 5 wt.% to 15 wt.%, based on the total weight of the core; or
the excipient in the shell is in a range of 35 wt.% to 45 wt.%, based on the total weight of the shell,
preferably, wherein the excipient in the core is in a range of 6 wt.% to 14 wt.%, 7 wt.% to 13 wt. %, more preferably 8 wt.% to 12 wt. %, most preferably 9 wt.% to 11 wt. %, based on the total weight of the core; or
the excipient in the shell is in a range of 35 wt.% to 45 wt.%, 36 wt.% to 44 wt.%, 37 wt.% to 43 wt. %, more preferably 38 wt.% to 42 wt. %, most preferably 39 wt.% to 41 wt.%, based on the total weight of the shell;
most preferably, the excipient in the core is 9.5 wt.% to 10.5 wt.%, based on the total weight of the core; or
the excipient in the shell is in a range from 39.5 wt.% to 40.5 wt.%, based on the total weight of the shell.

Thus, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% , preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and
   wherein the brain implant further comprises an excipient in the core, or in the shell; the excipient in the core is selected from the group consisting of citric acid, fumaric acid, glycolic acid, lactic acid, malic acid, tartaric acid, maleic acid, succinic acid and a mixture thereof; preferably citric acid, fumaric acid, and a mixture thereof and/or the excipient in the shell is selected from polyethylene glycol (PEG);
   the excipient in the core is in a range from 5 wt.% to 15 wt.%, based on the total weight of the core; or
   the excipient in the shell is in a range from 35 wt.% to 45 wt.%, based on the total weight of the shell.

More preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% , preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and wherein the brain implant further comprises an excipient in the core;
   the excipient in the core is selected from the group consisting of citric acid, fumaric acid, glycolic acid, lactic acid, malic acid, tartaric acid, maleic acid, succinic acid and a mixture thereof, preferably citric acid, fumaric acid, and a mixture thereof; and the excipient in the core is in a range from 5 wt.% to 15 wt.%, based on the total weight of the core.

In other words, the present invention relates to a brain implant for linear release of 5-aminolevulinic acid comprising
a) a core comprising:
   5-aminolevulinic acid and a core polymer, and an excipient;
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   the excipient in the core is selected from the group consisting of citric acid, fumaric acid, glycolic acid, lactic acid, malic acid, tartaric acid, maleic acid, succinic acid and a mixture thereof, preferably citric acid, fumaric acid, and a mixture thereof; and
   the excipient in the core is in a range from 5 wt.% to 15 wt.%, based on the total weight of the core;
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

More preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and
   wherein the brain implant further comprises an excipient in the shell;
   the excipient in the shell is selected from polyethylene glycol (PEG) and in a range from 35 wt.% to 45 wt.%, based on the total weight of the shell.

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, and an excipient, wherein the shell polymer is poly(lactic acid) (PLA);
   the excipient in the shell is selected from polyethylene glycol (PEG) and in a range from 35 wt.% to 45 wt.%, based on the total weight of the shell;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

More preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and
   wherein the shell further comprises 5-aminolevulinic acid in a range from 1 wt.% to 15 wt.% based on the total weight of the shell;
   wherein the brain implant further comprises an excipient in the core, or in the shell; the excipient in the core is in a range from 5 wt.% to 15 wt.%, based on the total weight of the core; or
   the excipient in the shell is in a range from 35 wt.% to 45 wt.%, based on the total weight of the shell.

More preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and
   wherein the shell further comprises 5-aminolevulinic acid in a range from 1 wt.% to 15 wt.% based on the total weight of the shell;
   wherein the brain implant further comprises an excipient in the core, and
   the excipient in the core is in a range from 5 wt.% to 15 wt.%, based on the total weight of the core.

In other words, the present invention relates to a brain implant for linear release of 5-aminolevulinic acid comprising
a) a core comprising:
   5-aminolevulinic acid, a core polymer, and an excipient,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   the excipient in the core is in a range from 5 wt.% to 15 wt.%, based on the total weight of the core;
   and
b) a shell consisting of an upper membrane, a spacer, and a lower membrane, wherein the upper membrane, the spacer, and the lower membrane each comprise a shell polymer, and 5-aminolevulinic acid,
   wherein the shell polymer is poly(lactic acid) (PLA);
   wherein 5-aminolevulinic acid in a range from 1 wt.% to 15 wt.% based on the total weight of the shell;
      characterized in that
      the shell polymer is in a range from 20 wt.% to 60 wt.%, preferably 25 wt.% to 55 wt.% based on the total weight of the brain implant; and
      when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
      when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

More preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and wherein the shell further comprises 5-aminolevulinic acid in a range from 1 wt.% to 15 wt.% based on the total weight of the shell;
   wherein the brain implant further comprises an excipient in the shell, and
   the excipient in the shell is in a range from 35 wt.% to 45 wt.%, based on the total weight of the shell.

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, 5-aminolevulinic acid, and an excipient, wherein the shell polymer is poly(lactic acid) (PLA),
   wherein 5-aminolevulinic acid is in a range from 1 wt.% to 15 wt.% based on the total weight of the shell,
   the excipient in the shell is in a range from 35 wt.% to 45 wt.%, based on the total weight of the shell;
      characterized in that
      the shell polymer is in a range from 20 wt.% to 60 wt.%, preferably 25 wt.% to 55 wt.% based on the total weight of the brain implant; and
      when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
      when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

More preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and
   wherein the shell further comprises 5-aminolevulinic acid in a range from 1 wt.% to 15 wt.% based on the total weight of the shell;
   wherein the brain implant further comprises an excipient in the core, or in the shell; the excipient in the core is selected from the group consisting of citric acid, fumaric acid, glycolic acid, lactic acid, malic acid, tartaric acid, maleic acid, succinic acid and a mixture thereof; preferably citric acid, fumaric acid, and a mixture thereof and/or the excipient in the shell is selected from polyethylene glycol (PEG);
   the excipient in the core is in a range from 5 wt.% to 15 wt.%, based on the total weight of the core; or
   the excipient in the shell is in a range from 35 wt.% to 45 wt.%, based on the total weight of the shell.

More preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and wherein the shell further comprises 5-aminolevulinic acid in a range from 1 wt.% to 15 wt.% based on the total weight of the shell;
   wherein the brain implant further comprises an excipient in the core,
   the excipient in the core is selected from the group consisting of citric acid, fumaric acid, glycolic acid, lactic acid, malic acid, tartaric acid, maleic acid, succinic acid and a mixture thereof; preferably citric acid, fumaric acid, and a mixture thereof, and the excipient in the core is in a range from 5 wt.% to 15 wt.%, based on the total weight of the core.

More preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and wherein the shell further comprises 5-aminolevulinic acid in a range from 1 wt.% to 15 wt.% based on the total weight of the shell;
   wherein the brain implant further comprises an excipient in the shell,
   the excipient in the shell is selected from polyethylene glycol (PEG); and
   the excipient in the shell is in a range from 35 wt.% to 45 wt.%, based on the total weight of the shell.

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
      and
b) a shell consisting of an upper membrane, a spacer, and a lower membrane, wherein the upper membrane, the spacer, and the lower membrane each comprise a shell polymer, 5-aminolevulinic acid, and an excipient, wherein the shell polymer is poly(lactic acid) (PLA),
   wherein 5-aminolevulinic acid is in a range from 1 wt.% to 15 wt.% based on the total weight of the shell,
   the excipient in the shell is selected from polyethylene glycol (PEG), and in a range from 35 wt.% to 45 wt.%, based on the total weight of the shell;
      characterized in that
      the shell polymer is in a range from 20 wt.% to 60 wt.%, preferably 25 wt.% to 55 wt.% based on the total weight of the brain implant; and
      when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
      when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

Preferably, in the brain implant as described herein, the excipient in the shell, in particular, the excipient in the upper membrane of the shell is PEG, and the PEG is selected from the group comprising or consisting of PEG 200, PEG 300, PEG 400, PEG 600, PEG 1000, PEG 1500, PEG 2000, PEG 3000, PEG 4000, PEG 6000, and PEG 8000; preferably, PEG 300, PEG 400, PEG 600, PEG 1000; more preferably PEG 300, PEG 400, PEG 600, still more preferably PEG 300, PEG 400; most preferably PEG 400.

Thus, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and
   wherein the brain implant further comprises an excipient in the shell,
   the excipient in the shell is polyethylene glycol 400 (PEG 400); and
   the excipient in the shell is in a range from 35 wt.% to 45 wt.%, based on the total weight of the shell.

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
      and
b) a shell consisting of an upper membrane, a spacer, and a lower membrane, wherein the upper membrane, the spacer, and the lower membrane each comprise a shell polymer, and polyethylene glycol 400 (PEG 400), wherein the shell polymer is poly(lactic acid) (PLA),
   the PEG 400 is in a range from 35 wt.% to 45 wt.%, based on the total weight of the shell;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

Preferably, in the brain implant as described herein, the excipient in the shell is only contained in the upper membrane and/or the lower membrane of the shell; more preferably the excipient in the shell is only contained in the upper membrane; still more preferably the excipient in the shell is only contained in the lower membrane; most preferably, the excipient in the shell is only contained in the upper and lower membranes of the shell.

Preferably, in the brain implant as described herein, the core is in a range from 45 wt.% to 70 wt.% based on the total weight of the brain implant. Preferably, the core is in a range from 46 wt.% to 70 wt.%, 48 wt.% to 70 wt.%, 49 wt.% to 70 wt.%, 50 wt.% to 70 wt.%, most preferably 50 wt.% to 69 wt.%, based on the total weight of the brain implant.

Thus, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt. %, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
   wherein the core is in a range from 45 wt.% to 70 wt.% based on the total weight of the brain implant.

Preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA); and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt. %, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
   wherein the shell further comprises 5-aminolevulinic acid in a range from 1 wt.% to 15 wt.% based on the total weight of the shell;
   wherein the core is in a range from 45 wt.% to 70 wt.% based on the total weight of the brain implant.

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
      and
b) a shell consisting of an upper membrane, a spacer, and a lower membrane, wherein the upper membrane, the spacer, and the lower membrane each
   comprise a shell polymer and 5-aminolevulinic acid,
   wherein the shell polymer is poly(lactic acid) (PLA);
   wherein 5-aminolevulinic acid in a range from 1 wt.% to 15 wt.% based on the total weight of the shell;
      characterized in that
      the shell polymer is in a range from 20 wt.% to 60 wt.%, preferably 25 wt.% to 55 wt.% based on the total weight of the brain implant; and
      when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
      when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
      wherein the core is in a range from 45 wt.% to 70 wt.% based on the total weight of the brain implant.

Preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% , preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
   wherein the brain implant further comprises an excipient in the core, or in the shell, wherein
   the excipient in the core is selected from the group consisting of citric acid, fumaric acid, glycolic acid, lactic acid, malic acid, tartaric acid, maleic acid, succinic acid and a mixture thereof; and/or
   the excipient in the shell is selected from polyethylene glycol (PEG);
   wherein the core is in a range from 45 wt.% to 70 wt.% based on the total weight of the brain implant.

Preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
   wherein the brain implant further comprises an excipient in the core, wherein
   the excipient in the core is selected from the group consisting of citric acid, fumaric acid, glycolic acid, lactic acid, malic acid, tartaric acid, maleic acid, succinic acid and a mixture thereof; and
   wherein the core is in a range from 45 wt.% to 70 wt.% based on the total weight of the brain implant.

In other words, the present invention relates to a brain implant for linear release of 5-aminolevulinic acid comprising
a) a core comprising:
   5-aminolevulinic acid, a core polymer, and an excipient,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   wherein the excipient in the core is selected from the group consisting of citric acid, fumaric acid, glycolic acid, lactic acid, malic acid, tartaric acid, maleic acid, succinic acid and a mixture thereof;
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
   wherein the core is in a range from 45 wt.% to 70 wt.% based on the total weight of the brain implant.

Preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
   wherein the brain implant further comprises an excipient in the shell, wherein
   the excipient in the shell is selected from polyethylene glycol (PEG);
   wherein the core is in a range from 45 wt.% to 70 wt.% based on the total weight of the brain implant.

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer and an excipient, wherein the shell polymer is poly(lactic acid) (PLA);
   wherein the excipient in the shell is selected from polyethylene glycol (PEG);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
   wherein the core is in a range from 45 wt.% to 70 wt.% based on the total weight of the brain implant.

Preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
   wherein the brain implant further comprises an excipient in the shell, wherein
   the excipient in the shell is selected from polyethylene glycol (PEG);
   wherein the excipient in the shell is only contained in the upper membrane and/or the lower membrane of the shell;
   wherein the core is in a range from 45 wt.% to 70 wt.% based on the total weight of the brain implant.

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer and an excipient, wherein the shell polymer is poly(lactic acid) (PLA); wherein the excipient in the shell is selected from polyethylene glycol (PEG); characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
   wherein the excipient in the shell is only contained in the upper membrane and/or the lower membrane of the shell;
   wherein the core is in a range from 45 wt.% to 70 wt.% based on the total weight of the brain implant.

Preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
      and
   b) a shell consisting of an upper membrane, a spacer, and a lower membrane, wherein the upper membrane, the spacer, and the lower membrane each comprise a shell polymer, wherein the shell polymer is poly(lactic acid) (PLA);
      characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
   wherein the shell further comprises 5-aminolevulinic acid in a range from 1 wt.% to 15 wt.% based on the total weight of the shell,
   wherein the brain implant further comprises an excipient in the core, or in the shell, wherein
   the excipient in the core is selected from the group consisting of citric acid, fumaric acid, glycolic acid, lactic acid, malic acid, tartaric acid, maleic acid, succinic acid and a mixture thereof; and/or
   the excipient in the shell is selected from polyethylene glycol (PEG);
   wherein the core is in a range from 45 wt.% to 70 wt.% based on the total weight of the brain implant.

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer and 5-aminolevulinic acid, wherein the shell polymer is poly(lactic acid) (PLA);
   wherein 5-aminolevulinic acid is in a range from 1 wt.% to 15 wt.% based on the total weight of the shell,
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
   wherein the brain implant further comprises an excipient in the core, or in the shell, wherein
   the excipient in the core is selected from the group consisting of citric acid, fumaric acid, glycolic acid, lactic acid, malic acid, tartaric acid, maleic acid, succinic acid and a mixture thereof; and/or
   the excipient in the shell is selected from polyethylene glycol (PEG);
   wherein the core is in a range from 45 wt.% to 70 wt.% based on the total weight of the brain implant.

In other words, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   wherein the core is in a range from 45 wt.% to 70 wt.% based on the total weight of the brain implant;
   and
b) a shell consisting of an upper membrane, a spacer, and a lower membrane, wherein the upper membrane, the spacer, and the lower membrane each comprise a shell polymer and 5-aminolevulinic acid,
   wherein the shell polymer is poly(lactic acid) (PLA);
   wherein 5-aminolevulinic acid in a range from 1 wt.% to 15 wt.% based on the total weight of the shell;
      characterized in that
      the shell polymer is in a range from 20 wt.% to 60 wt.%, preferably 25 wt.% to 55 wt.% based on the total weight of the brain implant; and
      when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
      when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

More preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and wherein the shell further comprises 5-aminolevulinic acid in a range from 1 wt.% to 15 wt.% based on the total weight of the shell;
   wherein the brain implant further comprises an excipient in the shell, and the excipient in the shell is in a range from 35 wt.% to 45 wt.%, based on the total weight of the shell.

More preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and wherein the shell further comprises 5-aminolevulinic acid in a range from 1 wt.% to 15 wt.% based on the total weight of the shell;
   wherein the brain implant further comprises an excipient in the shell, and the excipient in the shell is in a range from 35 wt.% to 45 wt.%, based on the total weight of the shell.

In some embodiments, the present invention refers to the brain implant for linear release of 5-aminolevulinic acid comprising the excipient in the shell, as described herein, wherein the excipient in the shell is PEG 400.

In some preferred embodiments, the present invention refers to the brain implant for linear release of 5-aminolevulinic acid comprising the excipient in the upper and lower membranes of the shell as described herein, wherein the excipient in the upper and lower membranes of the shell, is PEG 400.

Preferably, the brain implant for linear release of 5-aminolevulinic acid as described herein, consisting of:
a) a core consisting of:
   5-aminolevulinic acid in a range from 25 wt.% to 70 wt.%,
   a core polymer in a range from 70 wt.% to 25 wt.%, and
   optionally an excipient in the core in a range of 5 wt.% to 15 wt.%, based on the total weight of the core,
   wherein the core polymer is selected from the group consisting of poly(lactic acid) (PLA), poly(ethylene oxide) (PEO), poly(ethylene glycol) (PEG), and poly(lactic-co-glycolic acid) (PLGA); and
   the excipient in the core is the excipient in the core is selected from the group consisting of citric acid, fumaric acid, glycolic acid, lactic acid, malic acid, tartaric acid, maleic acid, succinic acid and a mixture thereof; preferably citric acid, fumaric acid or a mixture thereof;
   and
b) a shell consisting of:
   an upper membrane, a spacer, and a lower membrane each comprising
   a shell polymer in a range from 55 wt.% to 100 wt.%, and
   optionally, 5-aminolevulinic acid in a range from 1 wt.% to 15 wt.%, or
   optionally,an excipient in the shell in a range from 35 wt.% to 45 wt.% based on the total weight of the shell,
   wherein the shell polymer is poly(lactic acid) (PLA);
   wherein the excipient in the shell is PEG.

More preferably, the brain implant for linear release of 5-aminolevulinic acid as described herein, consisting of:
a) the core consisting of:
   5-aminolevulinic acid in a range from 25 wt.% to 75 wt.%,
   the core polymer in a range from 75 wt.% to 25 wt.%, and
   optionally the excipient in the core in a range of 5 wt.% to 15 wt.%, based on the total weight of the core,
   wherein the core polymer is selected from the group consisting of poly(lactic acid) (PLA), poly(ethylene oxide) (PEO), poly(ethylene glycol) (PEG), and poly(lactic-co-glycolic acid) (PLGA); and
   wherein the excipient in the core is citric acid, fumaric acid or a mixture thereof; and
b) the shell consisting of:
   the upper membrane, the spacer, and the lower membrane each comprising a shell polymer in a range from 55 wt.% to 100 wt.%, and
   optionally, 5-aminolevulinic acid in a range from 1 wt.% to 15 wt.%, or
   optionally,the excipient in the shell in a range from 35 wt.% to 45 wt.% based on the total weight of the shell,
   wherein the shell polymer is poly(lactic acid) (PLA);
   wherein the excipient in the shell is PEG 400.

More preferred, the present invention relates to the brain implant for linear release of 5-aminolevulinic acid, wherein
the brain implant consists of:
   a) the core consisting of:
      65 wt.% to 75 wt.% of 5-aminolevulinic acid, and 35 wt.% to 25 wt.% of poly (lactic-co-glycolic acid) (PLGA), based on a total weight of the core; and
   b) the shell consisting of poly(lactic acid) (PLA);
      wherein in the brain implant
      poly(lactic acid) (PLA) is in a range from 40 wt.% to 50 wt.%,
      based on a total weight of the brain implant;
         or
the brain implant consists of:
   a) the core consisting of:
      25 wt.% to 45 wt.% of 5-aminolevulinic acid and 75 wt.% to 55 wt.% of poly(lactic acid) (PLA), based on a total weight of the core; and
   b) the shell consisting of poly(lactic acid) (PLA);
      wherein in the brain implant
      poly(lactic acid) (PLA) is in a range from 75 wt.% to 85 wt.%,
      based on a total weight of the brain implant;
         or
the brain implant consists of:
   a) the core consisting of:
      25 wt.% to 35 wt.% of 5-aminolevulinic acid, and 75 wt.% to 65 wt.% of poly(ethylene oxide) (PEO), based on a total weight of the core; and
   b) the shell consisting of poly(lactic acid) (PLA);
      wherein in the brain implant
      poly(lactic acid) (PLA) is in a range from 45 wt.% to 55 wt.%,
      based on a total weight of the brain implant;
         or
the brain implant consists of:
   a) the core consisting of:
      65 wt.% to 75 wt.% of 5-aminolevulinic acid, and 35 wt.% to 25 wt.% of poly (lactic-co-glycolic acid) (PLGA), based on a total weight of the core; and
   b) the shell consisting of:
      5 wt.% to 15 wt.% of 5-aminolevulinic acid, and 95 wt.% to 85 wt.% of poly(lactic acid) (PLA) based on a total weight of the shell;
      wherein in the brain implant
      poly(lactic acid) (PLA) is in a range from 35 wt.% to 45 wt.%,
      based on a total weight of the brain implant;
         or
the brain implant consists of:
   a) the core consisting of:
      25 wt.% to 30 wt.% of 5-aminolevulinic acid, 5 wt.% to 15 wt.% of citric acid or fumaric acid, and 55 wt.% to 70 wt.% of poly(lactic acid) (PLA) based on a total weight of the core; and
   b) the shell consisting of poly(lactic acid) (PLA);
      wherein in the brain implant
      poly(lactic acid) (PLA) is in a range from 75 wt.% to 85 wt.%,
      based on a total weight of the brain implant;
         or
the brain implant consists of:
   a) the core consisting of:
      65 wt.% to 75 wt.% of 5-aminolevulinic acid, and 35 wt.% to 25 wt.% of poly (lactic-co-glycolic acid) (PLGA) based on a total weight of the core; and
   b) the shell consisting of:
      35 wt.% to 45 wt.% of polyethylene glycol 400, and 65 wt.% to 55 wt.% of poly(lactic acid) (PLA) based on a total weight of the shell;
      wherein in the brain implant
      poly(lactic acid) (PLA) is in a range from 25 wt.% to 35 wt.%,
      based on a total weight of the brain implant.
         or
the brain implant consists of:
   a) the core consisting of:
      25 wt.% to 45 wt.% of 5-aminolevulinic acid, and 75 wt.% to 55 wt.% of poly(lactic acid) or poly(ethylene glycol) or poly(ethylene oxide) based on the total weight of the core; or
      65 wt.% to 75 wt.% of 5-aminolevulinic acid, and 25 wt.% to 35 wt.% poly(lactic-co-glycolic acid) based on the total weight of the core;
         and
   b) the shell consisting of poly(lactic acid);
      wherein in the brain implant poly(lactic acid) is in a range from 20 wt.% to 60 wt.%, based on a total weight of the brain implant.

More preferably, the present invention refers to the brain implant for linear release of 5-aminolevulinic acid, selected from:
a brain implant comprising or consisting of
   15 wt.% to 16 wt.% of poly(lactic-co-glycolic acid) (PLGA),
   47 wt.% to 48 wt.% of poly(lactic acid) (PLA),
   36 wt.% to 37 wt.% of 5-aminolevulinic acid,
   based on the total weight of said brain implant;
   preferably the total weight of said brain implant is 230 mg;
a brain implant comprising or consisting of
   15 wt.% to 16 wt.% of poly(lactic-co-glycolic acid) (PLGA),
   28 wt.% to 29 wt.% of poly(lactic acid) (PLA),
   36 wt.% to 37 wt.% of 5-aminolevulinic acid,
   19 wt.% to 20 wt.% of PEG 400,
   based on the total weight of said brain implant;
   preferably the total weight of said brain implant is 230 mg;
a brain implant comprising or consisting of
   15 wt.% to 16 wt.% of poly(lactic-co-glycolic acid) (PLGA),
   42.5 wt.% to 43.5 wt.% of poly(lactic acid) (PLA),
   41 wt.% to 42 wt.% of 5-aminolevulinic acid,
   based on the total weight of said brain implant;
   preferably the total weight of said brain implant is 230 mg;
a brain implant comprising or consisting of
   15 wt.% to 16 wt.% of poly(lactic-co-glycolic acid) (PLGA),
   44.5 wt.% to 45.5 wt.% of poly(lactic acid) (PLA),
   39 wt.% to 40 wt.% of 5-aminolevulinic acid,
   based on the total weight of said brain implant;
   preferably the total weight of said brain implant is 230 mg;
a brain implant comprising or consisting of
   15 wt.% to 16 wt.% of poly(lactic-co-glycolic acid) (PLGA),
   43.5 wt.% to 44.5 wt.% of poly(lactic acid) (PLA),
   40 wt.% to 41 wt.% of 5-aminolevulinic acid,
   based on the total weight of said brain implant;
   preferably the total weight of said brain implant is 230 mg;
a brain implant comprising or consisting of
   76.5 wt.% to 77.5 wt.% of poly(lactic acid) (PLA),
   22.5 wt.% to 23.5 wt.% of 5-aminolevulinic acid,
   based on the total weight of said brain implant;
   preferably the total weight of said brain implant is 260 mg;
a brain implant comprising or consisting of
   82 wt.% to 83 wt.% of poly(lactic acid) (PLA),
   17 wt.% to 18 wt.% of 5-aminolevulinic acid,
   based on the total weight of said brain implant;
   preferably the total weight of said brain implant is 260 mg;
a brain implant comprising or consisting of
   79 wt.% to 80 wt.% of poly(lactic acid) (PLA),
   20 wt.% to 21 wt.% of 5-aminolevulinic acid,
   based on the total weight of said brain implant;
   preferably the total weight of said brain implant is 440 mg;
a brain implant comprising or consisting of
   80 wt.% to 81 wt.% of poly(lactic acid) (PLA),
   19 wt.% to 20 wt.% of 5-aminolevulinic acid,
   based on the total weight of said brain implant;
   preferably the total weight of said brain implant is 460 mg;
a brain implant comprising or consisting of
   77 wt.% to 78 wt.% of poly(lactic acid) (PLA),
   16 wt.% to 17 wt.% of 5-aminolevulinic acid,
   5.5 wt.% to 6.5 wt.% of citric acid,
   based on the total weight of said brain implant;
   preferably the total weight of said brain implant is 276.6 mg;
a brain implant comprising or consisting of
   77 wt.% to 78 wt.% of poly(lactic acid) (PLA),
   16 wt.% to 17 wt.% of 5-aminolevulinic acid,
   5.5 wt.% to 6.5 wt.% of fumaric acid,
   based on the total weight of said brain implant;
   preferably the total weight of said brain implant is 276.6 mg;
a brain implant consisting of
   35 wt.% to 36 wt.% of poly(ethylene oxide) (PEO),
   49 wt.% to 50 wt.% of poly(lactic acid) (PLA),
   15 wt.% to 16 wt.% of 5-aminolevulinic acid,
   based on the total weight of said brain implant;
   preferably the total weight of said brain implant is 295 mg;
      and
a brain implant comprising or consisting of
   32 wt.% to 33 wt.% of poly(ethylene oxide) (PEO),
   53 wt.% to 54 wt.% of poly(lactic acid) (PLA),
   14 wt.% to 15 wt.% of 5-aminolevulinic acid,
   based on the total weight of said brain implant;
   preferably the total weight of said brain implant is 320 mg;
   preferably, the poly(lactic acid) (PLA) is Resomer^{®} R 202S, 202H, 203S, or 203H;
   the poly(lactic-co-glycolic acid) (PLGA) is Resomer^{®} RG 502H.

Still more preferably, the present invention refers to the brain implant for linear release of 5-aminolevulinic acid, selected from:
a brain implant consisting of
   15.65 wt.% of poly(lactic-co-glycolic acid) (PLGA),
   47.83 wt.% of poly(lactic acid) (PLA),
   36.52 wt.% of 5-aminolevulinic acid,
   based on the total weight of said brain implant;
   preferably the total weight of said brain implant is 230 mg;
a brain implant consisting of
   15.65 wt.% of poly(lactic-co-glycolic acid) (PLGA),
   28.70 wt.% of poly(lactic acid) (PLA),
   36.52 wt.% of 5-aminolevulinic acid,
   19.13 wt.% of PEG 400,
   based on the total weight of said brain implant;
   preferably the total weight of said brain implant is 230 mg;
a brain implant consisting of
   15.65 wt.% of poly(lactic-co-glycolic acid) (PLGA),
   43.04 wt.% of poly(lactic acid) (PLA),
   41.30 wt.% of 5-aminolevulinic acid,
   based on the total weight of said brain implant;
   preferably the total weight of said brain implant is 230 mg;
a brain implant consisting of
   15.65 wt.% of poly(lactic-co-glycolic acid) (PLGA),
   44.96 wt.% of poly(lactic acid) (PLA),
   39.39 wt.% of 5-aminolevulinic acid,
   based on the total weight of said brain implant;
   preferably the total weight of said brain implant is 230 mg;
a brain implant consisting of
   15.65 wt.% of poly(lactic-co-glycolic acid) (PLGA),
   44.00 wt.% of poly(lactic acid) (PLA),
   40.35 wt.% of 5-aminolevulinic acid,
   based on the total weight of said brain implant;
   preferably the total weight of said brain implant is 230 mg;
a brain implant consisting of
   76.92 wt.% of poly(lactic acid) (PLA),
   23.08 wt.% of 5-aminolevulinic acid,
   based on the total weight of said brain implant;
   preferably the total weight of said brain implant is 260 mg;
a brain implant consisting of
   82.69 wt.% of poly(lactic acid) (PLA),
   17.31 wt.% of 5-aminolevulinic acid,
   based on the total weight of said brain implant;
   preferably the total weight of said brain implant is 260 mg;
a brain implant consisting of
   79.55 wt.% of poly(lactic acid) (PLA),
   20.45 wt.% of 5-aminolevulinic acid,
   based on the total weight of said brain implant;
   preferably the total weight of said brain implant is 440 mg;
a brain implant consisting of
   80.43 wt.% of poly(lactic acid) (PLA),
   19.57 wt.% of 5-aminolevulinic acid,
   based on the total weight of said brain implant;
   preferably the total weight of said brain implant is 460 mg;
a brain implant consisting of
   77.73 wt.% of poly(lactic acid) (PLA),
   16.27 wt.% of 5-aminolevulinic acid,
   6.0 wt.% of citric acid,
   based on the total weight of said brain implant;
   preferably the total weight of said brain implant is 276.6 mg;
a brain implant consisting of
   85.19 wt.% of poly(lactic acid) (PLA),
   16.27 wt.% of 5-aminolevulinic acid,
   6.0 wt.% of fumaric acid,
   based on the total weight of said brain implant;
   preferably the total weight of said brain implant is 276.6 mg;
a brain implant consisting of
   35.59 wt.% of poly(ethylene oxide) (PEO),
   49.15 wt.% of poly(lactic acid) (PLA),
   15.25 wt.% of 5-aminolevulinic acid,
   based on the total weight of said brain implant;
   preferably the total weight of said brain implant is 295 mg;
   and
a brain implant consisting of
   32.81 wt.% of poly(ethylene oxide) (PEO),
   53.13 wt.% of poly(lactic acid) (PLA),
   14.06 wt.% of 5-aminolevulinic acid,
   based on the total weight of said brain implant;
   preferably the total weight of said brain implant is 320 mg;
   preferably poly(lactic acid) (PLA) is Resomer ^{®} R 203H;
   the poly(lactic-co-glycolic acid) (PLGA) is Resomer^{®} RG 502H.

More preferred, the present invention relates to the brain implant for linear release of 5-aminolevulinic acid, comprising or constiting of:
a) the core consisting of:
   14 wt.% to 16 wt.% of poly(lactic-co-glycolic acid) (PLGA),
   35 wt.% to 37 wt.% of 5-aminolevulinic acid,
      and
b) the shell consisting of:
   the upper membrane consisting of
   4 wt.% to 5 wt.% of poly(lactic acid) (PLA);
      and
   the spacer and the lower membrane, i.e. the vessel consisting of 43 wt.% to 44 wt.% of poly(lactic acid) (PLA),
      based on the total weight of said brain implant;
      preferably, poly(lactic acid) (PLA) is Resomer^{®} R 202S, or 203S;
      poly(lactic-co-glycolic acid) (PLGA) is Resomer^{®} RG 502H; and/or
      the total weight of said brain implant is in a range from 200 mg to 300 mg, preferably from 210 mg to 280 mg, from 210 mg to 260 mg, more preferably from 220 mg to 250 mg, most preferably, from 220 mg to 240 mg;
         or
         a) the core consisting of:
            14 wt.% to 16 wt.% of poly(lactic-co-glycolic acid) (PLGA),
            35 wt.% to 37 wt.% of 5-aminolevulinic acid,
               and
         b) the shell consisting of:
            the upper membrane consisting of
               2 wt.% to 3 wt.% of poly(lactic acid) (PLA),
            1 wt.% to 2 wt.% of polyethylene glycol 400 (PEG 400);
               and
            the spacer and the lower membrane, i.e. the vessel consisting of
               25 wt.% to 27 wt.% of poly(lactic acid) (PLA),
            16 wt.% to 18 wt.% of polyethylene glycol 400 (PEG 400);
         based on the total weight of said brain implant;
      preferably, poly(lactic acid) (PLA) is Resomer^{®} R203 S; poly(lactic-co-glycolic acid) (PLGA) is Resomer^{®} RG 502H; and/or the total weight of said brain implant is in a range from 200 mg to 300 mg, preferably from 210 mg to 280 mg, from 210 mg to 260 mg, more preferably from 220 mg to 250 mg, most preferably, from 220 mg to 240 mg;
         or
         a) the core consisting of:
            14 wt.% to 16 wt.% of poly(lactic-co-glycolic acid) (PLGA),
            35 wt.% to 37 wt.% of 5-aminolevulinic acid,
               and
         b) the shell consisting of:
            the upper membrane consisting of
               3.5 to 4.5 wt.% of poly(lactic acid) (PLA),
            0.1 to 1 wt.% of 5-aminolevulinic acid;
               and
            the spacer and the lower membrane, i.e. the vessel consisting of 38 to 42 wt.% of poly(lactic acid) (PLA),
            2 to 5 wt.% of 5-aminolevulinic acid;
         based on the total weight of said brain implant;
      preferably, poly(lactic acid) (PLA) is Resomer^{®} R 203S;
      poly(lactic-co-glycolic acid) (PLGA) is Resomer^{®} RG 502H; and/or
      the total weight of said brain implant is in a range from 200 mg to 300 mg, preferably from 210 mg to 280 mg, from 210 mg to 260 mg, more preferably from 220 mg to 250 mg, most preferably, from 220 mg to 240 mg;
         or
         a) the core consisting of:
            34 wt.% to 48 wt.% of poly(lactic acid) (PLA),
            17 wt.% to 21 wt.% of 5-aminolevulinic acid,
               and
         b) the shell consisting of:
            the upper membrane consisting of
               2 wt.% to 7 wt.% of poly(lactic acid) (PLA),
               and
            the spacer and the lower membrane, i.e. the vessel consisting of
               27 wt.% to 39 wt.% of poly(lactic acid) (PLA),
         based on the total weight of said brain implant;
      preferably, poly(lactic acid) (PLA) is Resomer^{®} R 203H; and/or the total weight of said brain implant is in a range from 200 mg to 500 mg, preferably from 220 mg to 500 mg, from 240 mg to 500 mg, from 240 mg to 480 mg, more preferably from 250 mg to 480 mg, most preferably, from 260 mg to 460 mg;
         or
         a) the core consisting of:
            37 wt.% to 39 wt.% of poly(lactic acid) (PLA),
            16 wt.% to 17 wt.% of 5-aminolevulinic acid,
            5 wt.% to 7 wt.% of citric acid, or fumaric acid,
               and
         b) the shell consisting of:
            the upper membrane consisting of
               3 wt.% to 4 wt.% of poly(lactic acid) (PLA),
               and
            the spacer and the lower membrane, i.e. the vessel consisting of
               35 wt.% to 37 wt.% of poly(lactic acid) (PLA),
         based on the total weight of said brain implant;
      preferably, poly(lactic acid) (PLA) is Resomer^{®} R 203H; and/or
      the total weight of said brain implant is in a range from 200 mg to 300 mg, preferably from 220 mg to 300 mg, from 240 mg to 300 mg, more preferably from 260 mg to 300 mg, most preferably, from 260 mg to 280 mg;
         or
         a) the core consisting of:
            32 wt.% to 37 wt.% of polylethylene oxide (PEO),
            13 wt.% to 16 wt.% of 5-aminolevulinic acid,
               and
         b) the shell consisting of:
            the upper membrane consisting of
               13 wt.% to 16 wt.% of poly(lactic acid) (PLA),
               and
            the spacer and the lower membrane, i.e. the vessel consisting of 35 wt.% to 39 wt.% of poly(lactic acid) (PLA),
         based on the total weight of said brain implant;
      preferably, poly(lactic acid) (PLA) is Resomer^{®} R 202S; and/or
      the total weight of said brain implant is in a range from 250 mg to 350 mg, preferably from 260 mg to 350 mg, from 270 mg to 340 mg, more preferably from 280 mg to 340 mg, most preferably, from 280 mg to 330 mg.

Still more preferred, the present invention relates to the brain implant for linear release of 5-aminolevulinic acid, constiting of:
a) the core consisting of:
   15.65 wt.% of poly(lactic-co-glycolic acid) (PLGA),
   36.52 wt.% of 5-aminolevulinic acid,
      and
b) the shell consisting of:
   the upper membrane consisting of
      4.35 wt.% of poly(lactic acid) (PLA);
      and
   the spacer and the lower membrane, i.e. the vessel consisting of
      43.48 wt.% of poly(lactic acid) (PLA),
   based on the total weight of said pharmaceutical composition;
   preferably, poly(lactic acid) (PLA) is Resomer^{®} R 202S, 203S, or 203H; preferably R 202S, or 203S;
   poly(lactic-co-glycolic acid) (PLGA) is Resomer^{®} RG 502H; and/or the total weight of said brain implant is in a range from 200 mg to 300 mg, preferably from 210 mg to 280 mg, from 210 mg to 260 mg, from 220 mg to 250 mg, more preferably from 220 mg to 240 mg, most preferably 230 mg;
      or
      a) the core consisting of:
         15.65 wt.% of poly(lactic-co-glycolic acid) (PLGA),
         36.52 wt.% of 5-aminolevulinic acid,
            and
      b) the shell consisting of:
         the upper membrane consisting of
            2.61 wt.% of poly(lactic acid) (PLA),
         1.74 wt.% of polyethylene glycol 400 (PEG 400);
            and
         the spacer and the lower membrane, i.e. the vessel consisting of
            26.09 wt.% of poly(lactic acid) (PLA),
         17.39 wt.% of polyethylene glycol 400 (PEG 400);
         based on the total weight of said pharmaceutical composition;
   preferably, poly(lactic acid) (PLA) is Resomer^{®} R203 S;
   poly(lactic-co-glycolic acid) (PLGA) is Resomer^{®} RG 502H; and/or
   the total weight of said brain implant is in a range from 200 mg to 300 mg, preferably from 210 mg to 280 mg, from 210 mg to 260 mg, from 220 mg to 250 mg, more preferably from 220 mg to 240 mg, most preferably 230 mg;
      or
      a) the core consisting of:
         15.65 wt.% of poly(lactic-co-glycolic acid) (PLGA),
         36.52 wt.% of 5-aminolevulinic acid,
            and
      b) the shell consisting of:
         the upper membrane consisting of
            3.91 wt.% of poly(lactic acid) (PLA),
         0.43 wt.% of 5-aminolevulinic acid;
            and
         the spacer and the lower membrane, i.e. the vessel consisting of
            39.13 wt.% of poly(lactic acid) (PLA),
         4.35 wt.% of 5-aminolevulinic acid;
         based on the total weight of said pharmaceutical composition;
   preferably, poly(lactic acid) (PLA) is Resomer^{®} R 203S;
   poly(lactic-co-glycolic acid) (PLGA) is Resomer^{®} RG 502H; and/or
   the total weight of said brain implant is in a range from 200 mg to 300 mg, preferably from 210 mg to 280 mg, from 210 mg to 260 mg, from 220 mg to 250 mg, more preferably from 220 mg to 240 mg, most preferably 230 mg;
      or
      a) the core consisting of:
         15.65 wt.% of poly(lactic-co-glycolic acid) (PLGA),
         36.52 wt.% of 5-aminolevulinic acid,
            and
      b) the shell consisting of:
         the upper membrane consisting of
            4.09 wt.% of poly(lactic acid) (PLA),
         0.26 wt.% of 5-aminolevulinic acid;
            and
         the spacer and the lower membrane, i.e. the vessel consisting of
            40.87 wt.% of poly(lactic acid) (PLA),
         2.61 wt.% of 5-aminolevulinic acid;
         based on the total weight of said pharmaceutical composition;
   preferably, poly(lactic acid) (PLA) is Resomer^{®} R 203S;
   poly(lactic-co-glycolic acid) (PLGA) is Resomer^{®} RG 502H; and/or
   the total weight of said pharmaceutical composition is in a range from 200 mg to 280 mg, from 200 mg to 260 mg, from 210 mg to 250 mg, more preferably from 220 mg to 240 mg, most preferably 230 mg;
      or
      a) the core consisting of:
         15.65 wt.% of poly(lactic-co-glycolic acid) (PLGA),
         36.52 wt.% of 5-aminolevulinic acid,
            and
      b) the shell consisting of:
         the upper membrane consisting of
            4.00 wt.% of poly(lactic acid) (PLA),
         0.35 wt.% of 5-aminolevulinic acid;
            and
         the spacer and the lower membrane, i.e. the vessel consisting of
            40.00 wt.% of poly(lactic acid) (PLA),
         3.48 wt.% of 5-aminolevulinic acid;
         based on the total weight of said pharmaceutical composition;
   preferably, poly(lactic acid) (PLA) is Resomer^{®} R 203S;
   poly(lactic-co-glycolic acid) (PLGA) is Resomer^{®} RG 502H; and/or
   the total weight of said pharmaceutical composition is in a range from 200 mg to 280 mg, from 200 mg to 260 mg, from 210 mg to 250 mg, more preferably from 220 mg to 240 mg, most preferably 230 mg;
      or
      a) the core consisting of:
         34.62 wt.% of poly(lactic acid) (PLA),
         23.08 wt.% of 5-aminolevulinic acid,
            and
      b) the shell consisting of:
         the upper membrane consisting of
            3.85 wt.% of poly(lactic acid) (PLA),
            and
         the spacer and the lower membrane, i.e. the vessel consisting of 38.46 wt.% of poly(lactic acid) (PLA),
         based on the total weight of said pharmaceutical composition;
   preferably, poly(lactic acid) (PLA) is Resomer^{®} R 203H; and/or the total weight of said pharmaceutical composition is in a range from 200 mg to 300 mg, from 220 mg to 300 mg, from 240 mg to 280 mg, more preferably from 250 mg to 270 mg, most preferably 260 mg;
      or
      a) the core consisting of:
         40.38 wt.% of poly(lactic acid) (PLA),
         17.31 wt.% of 5-aminolevulinic acid,
            and
      b) the shell consisting of:
         the upper membrane consisting of
            3.85 wt.% of poly(lactic acid) (PLA),
            and
         the spacer and the lower membrane, i.e. the vessel consisting of
            38.46 wt.% of poly(lactic acid) (PLA),
         based on the total weight of said pharmaceutical composition;
   preferably, poly(lactic acid) (PLA) is Resomer^{®} R 203H; and/or the total weight of said pharmaceutical composition is in a range from 200 mg to 300 mg, from 220 mg to 300 mg, from 240 mg to 280 mg, more preferably from 250 mg to 270 mg, most preferably 260 mg;
      or
      a) the core consisting of:
         47.73 wt.% of poly(lactic acid) (PLA),
         20.45 wt.% of 5-aminolevulinic acid,
            and
      b) the shell consisting of:
         the upper membrane consisting of
            2.27 wt.% of poly(lactic acid) (PLA),
            and
         the spacer and the lower membrane, i.e. the vessel consisting of
            29.55 wt.% of poly(lactic acid) (PLA),
         based on the total weight of said pharmaceutical composition;
   preferably, poly(lactic acid) (PLA) is Resomer^{®} R 203H; and/or the total weight of said pharmaceutical composition is in a range from 400 mg to 500 mg, from 400 mg to 480 mg, from 420 mg to 460 mg, more preferably from 430 mg to 450 mg, most preferably from 440 mg;
      or
      a) the core consisting of:
         45.65 wt.% of poly(lactic acid) (PLA),
         19.57 wt.% of 5-aminolevulinic acid,
            and
      b) the shell consisting of:
         the upper membrane consisting of
            6.52 wt.% of poly(lactic acid) (PLA),
            and
         the spacer and the lower membrane, i.e. the vessel consisting of
            28.26 wt.% of poly(lactic acid) (PLA),
         based on the total weight of said pharmaceutical composition;
   preferably, poly(lactic acid) (PLA) is Resomer^{®} R 203H; and/or the total weight of said pharmaceutical composition is in a range from 400 mg to 500 mg, from 420 mg to 500 mg, from 440 mg to 480 mg, more preferably from 450 mg to 470 mg, most preferably from 460 mg;
      or
      a) the core consisting of:
         37.96 wt.% of poly(lactic acid) (PLA),
         16.27 wt.% of 5-aminolevulinic acid,
         6.00 wt.% of citric acid,
            and
      b) the shell consisting of:
         the upper membrane consisting of
         3.62 wt.% of poly(lactic acid) (PLA),
            and
         the spacer and the lower membrane, i.e. the vessel consisting of 36.15 wt.% of poly(lactic acid) (PLA),
         based on the total weight of said pharmaceutical composition;
   preferably, poly(lactic acid) (PLA) is Resomer^{®} R 203H; and/or
   the total weight of said pharmaceutical composition is in a range from 240 mg to 330 mg, from 240 mg to 300 mg, from 250 mg to 290 mg, more preferably from 260 mg to 280 mg, most preferably from 270 mg to 280 mg;
      or
      a) the core consisting of:
         37.96 wt.% of poly(lactic acid) (PLA),
         16.27 wt.% of 5-aminolevulinic acid,
         6.00 wt.% of fumaric acid,
            and
      b) the shell consisting of:
         the upper membrane consisting of
            3.62 wt.% of poly(lactic acid) (PLA),
            and
         the spacer and the lower membrane, i.e. the vessel consisting of
            36.15 wt.% of poly(lactic acid) (PLA),
         based on the total weight of said pharmaceutical composition;
   preferably, poly(lactic acid) (PLA) is Resomer^{®} R 203H; and/or the total weight of said pharmaceutical composition is in a range from 240 mg to 330 mg, from 240 mg to 300 mg, from 250 mg to 290 mg, more preferably from 260 mg to 280 mg, most preferably from 270 mg to 280 mg;
      or
      a) the core consisting of:
         35.59 wt.% of polylethylene oxide (PEO),
         15.25 wt.% of 5-aminolevulinic acid,
            and
      b) the shell consisting of:
         the upper membrane consisting of
            13.56 wt.% of poly(lactic acid) (PLA),
            and
         the spacer and the lower membrane, i.e. the vessel consisting of 35.59 wt.% of poly(lactic acid) (PLA),
         based on the total weight of said pharmaceutical composition;
   preferably, poly(lactic acid) (PLA) is Resomer^{®} R 202S; and/or
   the total weight of said pharmaceutical composition is in a range from 250 mg to 350 mg, from 260 mg to 340 mg, from 270 mg to 330 mg, from 280 mg to 320 mg, more preferably from 290 mg to 310 mg, most preferably 295 mg;
      or
      a) the core consisting of:
         32.81 wt.% of polylethylene oxide (PEO),
         14.06 wt.% of 5-aminolevulinic acid,
            and
      b) the shell consisting of:
         the upper membrane consisting of
         15.63 wt.% of poly(lactic acid) (PLA),
            and
         the spacer and the lower membrane, i.e. the vessel consisting of 37.50 wt.% of poly(lactic acid) (PLA),
         based on the total weight of said pharmaceutical composition;
   preferably, poly(lactic acid) (PLA) is Resomer^{®} R 202S; and/or
   the total weight of said pharmaceutical composition is in a range from 270 mg to 370 mg, from 280 mg to 360 mg, from 290 mg to 350 mg, from 300 mg to 340 mg, more preferably from 310 mg to 330 mg, most preferably 320 mg.

Preferably, in the brain implants as described herein,
the poly(lactic-co-glycolic acid) (PLGA) has a lactic acid:glycolic acid weight ratio o from 40:60 to 60:40, 45:55 to 55:45, preferably to 47:53 to 53:47, most preferably 50:50; and has M_{w} from 7 to 30 kDa, more preferably 7 to 20 kDa, most preferably 7 to 17 kDa;

For example, Resomer ^{®} RG 502H has M_{w} from 7 to 17 kDa;
the poly(lactic acid) (PLA) has the average molecular weight (M_{w}) from 8 to 30 kDa, more preferably, M_{w} from 9 to 29 kDa, most preferably, M_{w} from 10 to 28 kDa;
Resomer ^{®} R 202 H has M_{w} from 10 to 18 kDa. Resomer ^{®} R 202 S has M_{w} from 10 to 18 kDa; Resomer ^{®} R 203 H has M_{w} from 18 to 24 kDa. Resomer ^{®} R 203 S has M_{w} from 18 to 28 kDa;
the polylethylene oxide (PEO) has the average molecular weight (M_{w}) from 170 to 230 kDa, 180 to 220 kDa, more preferably 190 to 210 kDa, or most preferably 200 kDa;

For example, the PEO as used herein has M_{w} of 200 kDa.

Thus, the present invention relates to a brain implant for linear release of 5-aminolevulinic acid comprising:
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
      and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt. %, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.
   wherein
   the PLGA has a lactic acid:glycolic acid weight ratio o from 40:60 to 60:40, 45:55 to 55:45, preferably to 47:53 to 53:47, most preferably 50:50; and has M_{w} from 7 to 30 kDa, more preferably 7 to 20 kDa, most preferably 7 to 17 kDa;
   the poly(lactic acid) (PLA) has the average molecular weight (M_{w}) from 8 to 30 kDa, more preferably, M_{w} from 9 to 29 kDa, most preferably, M_{w} from 10 to 28 kDa;
   the polylethylene oxide (PEO) has the average molecular weight (M_{w}) from 170 to 230 kDa, 180 to 220 kDa, more preferably 190 to 210 kDa, most preferably 200 kDa.

Preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer and optinally 5-aminolevulinic acid, wherein the shell polymer is poly(lactic acid) (PLA);
   wherein 5-aminolevulinic acid is in a range from 1 wt.% to 15 wt.% based on the total weight of the shell;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
      wherein
   the PLGA has a lactic acid:glycolic acid weight ratio o from 40:60 to 60:40, 45:55 to 55:45, preferably to 47:53 to 53:47, most preferably 50:50; and has M_{w} from 7 to 30 kDa, more preferably 7 to 20 kDa, most preferably 7 to 17 kDa;
   the poly(lactic acid) (PLA) has the average molecular weight (M_{w}) from 8 to 30 kDa, more preferably, M_{w} from 9 to 29 kDa, most preferably, M_{w} from 10 to 28 kDa;
   the polylethylene oxide (PEO) has the average molecular weight (M_{w}) from 170 to 230 kDa, 180 to 220 kDa, more preferably 190 to 210 kDa, most preferably 200 kDa.

Preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; and wherein the brain implant further comprises an excipient in the core, or in the shell, the excipient in the core is selected from the group consisting of citric acid, fumaric acid, glycolic acid, lactic acid, malic acid, tartaric acid, maleic acid, succinic acid and a mixture thereof; preferably citric acid, fumaric acid, and a mixture thereof and/or the excipient in the shell is selected from polyethylene glycol (PEG);
      wherein
   the PLGA has a lactic acid:glycolic acid weight ratio o from 40:60 to 60:40, 45:55 to 55:45, preferably to 47:53 to 53:47, most preferably 50:50; and has M_{w} from 7 to 30 kDa, more preferably 7 to 20 kDa, most preferably 7 to 17 kDa;
   the poly(lactic acid) (PLA) has the average molecular weight (M_{w}) from 8 to 30 kDa, more preferably, M_{w} from 9 to 29 kDa, most preferably, M_{w} from 10 to 28 kDa;
   the polylethylene oxide (PEO) has the average molecular weight (M_{w}) from 170 to 230 kDa, 180 to 220 kDa, more preferably 190 to 210 kDa, most preferably 200 kDa.

More preferalby, the present invention relates to a brain implant for linear release of 5-aminolevulinic acid comprising
a) a core comprising:
   5-aminolevulinic acid and a core polymer, and an excipient;
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   the excipient in the core is selected from the group consisting of citric acid, fumaric acid, glycolic acid, lactic acid, malic acid, tartaric acid, maleic acid, succinic acid and a mixture thereof, preferably citric acid, fumaric acid, and a mixture thereof; and
   the excipient in the core is in a range from 5 wt.% to 15 wt.%, based on the total weight of the core;
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core;
      wherein
   the PLGA has a lactic acid:glycolic acid weight ratio o from 40:60 to 60:40, 45:55 to 55:45, preferably to 47:53 to 53:47, most preferably 50:50; and has M_{w} from 7 to 30 kDa, more preferably 7 to 20 kDa, most preferably 7 to 17 kDa;
   the poly(lactic acid) (PLA) has the average molecular weight (M_{w}) from 8 to 30 kDa, more preferably, M_{w} from 9 to 29 kDa, most preferably, M_{w} from 10 to 28 kDa;
   the polylethylene oxide (PEO) has the average molecular weight (M_{w}) from 170 to 230 kDa, 180 to 220 kDa, more preferably 190 to 210 kDa, most preferably 200 kDa.

More preferably, the present invention relates to a brain implant for linear release of 5-aminolevulinic acid comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, and an excipient, wherein the shell polymer is poly(lactic acid) (PLA);
   the excipient in the shell is selected from polyethylene glycol (PEG) and in a range from 35 wt.% to 45 wt.%, based on the total weight of the shell;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core; wherein
      the PLGA has a lactic acid:glycolic acid weight ratio o from 40:60 to 60:40, 45:55 to 55:45, preferably to 47:53 to 53:47, most preferably 50:50; and has M_{w} from 7 to 30 kDa, more preferably 7 to 20 kDa, most preferably 7 to 17 kDa;
      the poly(lactic acid) (PLA) has the average molecular weight (M_{w}) from 8 to 30 kDa, more preferably, M_{w} from 9 to 29 kDa, most preferably, M_{w} from 10 to 28 kDa;
      the polylethylene oxide (PEO) has the average molecular weight (M_{w}) from 170 to 230 kDa, 180 to 220 kDa, more preferably 190 to 210 kDa, most preferably 200 kDa.

Preferably, the brain implant for linear release of 5-aminolevulinic acid as described herein, consisting of:
a) a core consisting of:
   5-aminolevulinic acid in a range from 25 wt.% to 70 wt.%,
   a core polymer in a range from 70 wt.% to 25 wt.%, and
   optionally an excipient in the core in a range of 5 wt.% to 15 wt.%, based on the total weight of the core,
   wherein the core polymer is selected from the group consisting of poly(lactic acid) (PLA), poly(ethylene oxide) (PEO), poly(ethylene glycol) (PEG), and poly(lactic-co-glycolic acid) (PLGA); and
   the excipient in the core is the excipient in the core is selected from the group consisting of citric acid, fumaric acid, glycolic acid, lactic acid, malic acid, tartaric acid, maleic acid, succinic acid and a mixture thereof; preferably citric acid, fumaric acid or a mixture thereof;
      and
b) a shell consisting of:
   an upper membrane, a spacer, and a lower membrane each comprising
   a shell polymer in a range from 55 wt.% to 100 wt.%, and
   optionally, 5-aminolevulinic acid in a range from 1 wt.% to 15 wt.%, or
   optionally,an excipient in the shell in a range from 35 wt.% to 45 wt.% based on the total weight of the shell,
   wherein the shell polymer is poly(lactic acid) (PLA);
   wherein the excipient in the shell is PEG,
   wherein
   the PLGA has a lactic acid:glycolic acid weight ratio o from 40:60 to 60:40, 45:55 to 55:45, preferably to 47:53 to 53:47, most preferably 50:50; and has M_{w} from 7 to 30 kDa, more preferably 7 to 20 kDa, most preferably 7 to 17 kDa;
   the poly(lactic acid) (PLA) has the average molecular weight (M_{w}) from 8 to 30 kDa, more preferably, M_{w} from 9 to 29 kDa, most preferably, M_{w} from 10 to 28 kDa; the polylethylene oxide (PEO) has the average molecular weight (M_{w}) from 170 to 230 kDa, 180 to 220 kDa, more preferably 190 to 210 kDa, most preferably 200 kDa.

Still more preferably, the brain implant for linear release of 5-aminolevulinic acid as described herein, consisting of:
a) the core consisting of:
   5-aminolevulinic acid in a range from 25 wt.% to 75 wt.%,
   the core polymer in a range from 75 wt.% to 25 wt.%, and
   optionally the excipient in the core in a range of 5 wt.% to 15 wt.%, based on the total weight of the core,
   wherein the core polymer is selected from the group consisting of poly(lactic acid) (PLA), poly(ethylene oxide) (PEO), poly(ethylene glycol) (PEG), and poly(lactic-co-glycolic acid) (PLGA); and
   wherein the excipient in the core is citric acid, fumaric acid or a mixture thereof;
      and
b) the shell consisting of:
   the upper membrane, the spacer, and the lower membrane each comprising a shell polymer in a range from 55 wt.% to 100 wt.%, and
   optionally, 5-aminolevulinic acid in a range from 1 wt.% to 15 wt.%, or optionally,the excipient in the shell in a range from 35 wt.% to 45 wt.% based on the total weight of the shell,
   wherein the shell polymer is poly(lactic acid) (PLA);
   wherein the excipient in the shell is PEG 400,
   wherein
   the PLGA has a lactic acid:glycolic acid weight ratio o from 40:60 to 60:40, 45:55 to 55:45, preferably to 47:53 to 53:47, most preferably 50:50; and has M_{w} from 7 to 30 kDa, more preferably 7 to 20 kDa, most preferably 7 to 17 kDa;
   the poly(lactic acid) (PLA) has the average molecular weight (M_{w}) from 8 to 30 kDa, more preferably, M_{w} from 9 to 29 kDa, most preferably, M_{w} from 10 to 28 kDa;
   the polylethylene oxide (PEO) has the average molecular weight (M_{w}) from 170 to 230 kDa, 180 to 220 kDa, more preferably 190 to 210 kDa, most preferably 200 kDa.

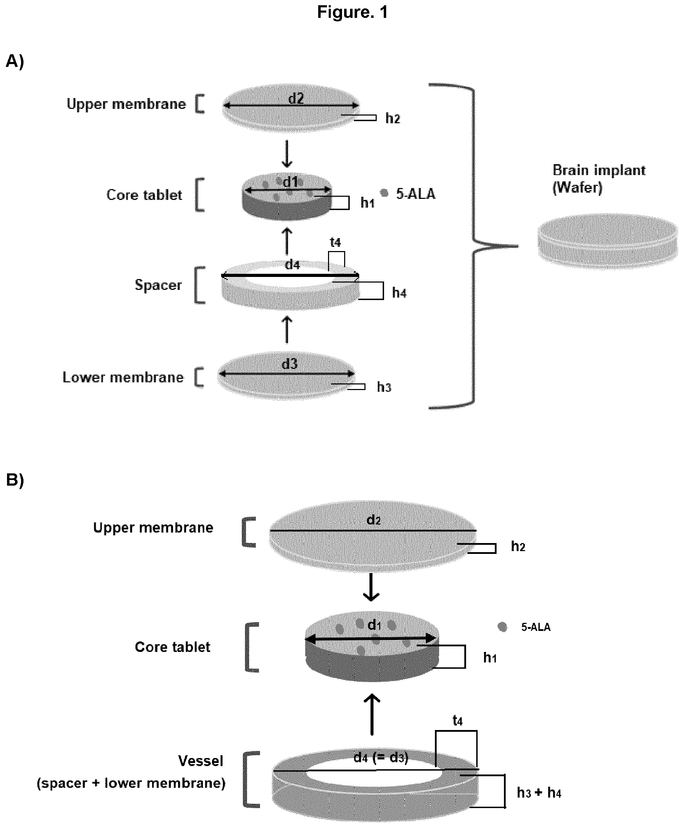

Preferably, the brain implant of the present invention is preferably in form of a wafer (see Figure 1 A);
the core has a diameter (d₁) in a range of 10 mm to 12 mm and a height (h₁) in a range from 0.5 mm to 3.0 mm, preferably from 1.0 mm to 1.5 mm;
the upper membrane has a diameter (d₂) in a range of 12 mm to 14 mm and a height (h₂) in a range from 0.05 mm to 0.60 mm, preferably 0.10 mm to 0.50 mm; and the lower membrane has a diameter (d₃) in a range of 12 mm to 14 mm and a height (h₃) in a range from 0.05 mm to 0.60 mm, preferably 0.10 mm to 0.50 mm, and the spacer has a diameter (d₄) in a range of 12 mm to 14 mm and a thickness (t₄) in a range from 1 mm to 2 mm; and a height (h₄) in a range from 0.5 mm to 3.0 mm, preferably from 1.0 mm to 1.5 mm.

Preferably, in all brain implants disclosed herein h₁ = h₄ and/or h₂ = h₃.

Alternatively, the brain implant of the present invention is preferably in form of a wafer (see Figure 1 B);
the core has a diameter (d₁) in a range of 10 mm to 12 mm and a height (h₁) in a range 0.5 mm to 3.0 mm, preferably from 1.0 mm to 1.5 mm;
the upper membrane has a diameter (d₂) in a range of 12 mm to 14 mm and a height (h₂) in a range from 0.05 mm to 0.60 mm, preferably 0.10 mm to 0.50 mm;
the vessel consists of and the spacer and the lower membrane; and the vessel has a diameter (d₄ = ds) in a range of 12 mm to 14 mm and a height (h₃ + h₄) in a range from 1.1 mm to 2.0 mm, and and a thickness (t₄) in a range from 1 mm to 2 mm. Preferably h₂ = h₃.

Alternatively, further brain implant are disclosed herein which are preferably in form of a wafer, wherein
the core has a height (h₁) in a range from 1.0 mm to 3.0 mm;
the upper membrane has a height (h₂) in a range from 0.05 mm to 0.60 mm, preferably 0.10 mm to 0.50 mm; and
the lower membrane has a height (h₃) in a range from 0.05 mm to 0.60 mm, preferably 0.10 mm to 0.50 mm.

Most preferably, all brain implants disclosed herein have the height (h₂) in the range from 0.05 mm to 0.60 mm, preferably 0.10 mm to 0.50 mm; and/or have the height (h₃) in the range from 0.05 mm to 0.60 mm, preferably 0.10 mm to 0.50 mm; and/or have h₂ = h₃.

The terms d₁, d₂, d₃, d₄, and t₁, t₂, t₃, t₄ and h₄ as used herein are depicted in Figure 1A and 1B).

Preferably, the brain implant in form of a wafer as described herein, the PLGA has a lactic acid:glycolic acid weight ratio o from 40:60 to 60:40, 45:55 to 55:45, preferably to 47:53 to 53:47, most preferably 50:50; and has M_{w} from 7 to 30 kDa, more preferably 7 to 20 kDa, most preferably 7 to 17 kDa;
the poly(lactic acid) (PLA) has the average molecular weight (M_{w}) from 8 to 30 kDa, more preferably, M_{w} from 9 to 29 kDa, most preferably, M_{w} from 10 to 28 kDa;
the polylethylene oxide (PEO) has the average molecular weight (M_{w}) from 170 to 230 kDa, 180 to 220 kDa, more preferably 190 to 210 kDa, most preferably 200 kDa.

The total amount of the acid (except 5-aminolevulinic acid), preferably citric acid or fumaric acid, in the brain implant of the present invention may be in a range from 1 wt% to 40 wt%, preferably from 1 wt% to 30 wt%, more preferably from 1 wt% to 25 wt%, more preferably from 1 wt% to 20 wt%, more preferably from 1 wt% to 15 wt%, more preferably from 1 wt% to 10 wt%, most preferably from 5 wt% to 10 wt%.

Moreover, the amount of 5-aminolevulinic acid in the brain implant of the present invention may be in a range from 10 mg to 200 mg, preferably from 10 mg 180 mg, preferably from 10 mg to 170 mg, preferably from 15 mg to 150 mg, preferably from 15 mg to 140 mg, preferably from 20 mg to 120 mg, preferably from 30 mg to 120 mg, more preferably from 35 mg to 110 mg, most preferably from 40 mg to 100 mg.

Also, the amount of the acid (of course except 5-aminolevulinic acid), preferably citric acid or fumaric acid, in the brain implant of the present invention may be in a range preferably from 10 mg 45 mg, more preferably from 10 mg to 40 mg, still more preferably from 10 mg to 35 mg, still more preferably from 12 mg to 35 mg, still more preferably from 12 mg to 30 mg, still more preferably from 12 mg to 25 mg, more preferably from 14 mg to 20 mg, preferably from 14 mg to 18 mg.

### Preparation Methods

In another aspect, the present invention refers to a method for producing the brain implant for linear releas of 5-aminolevulinic acid as descrebied herein.

Thus, the present invention refers to a method for producing the brain implant for linear release of 5-aminolevulinic acid as described above, comprising:
Step 1) Preparing a core comprising 5-aminolevulinic acid (ALA) and a core polymer by using vapour control module (VCM) D10 mm chamber,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
Step 2) preparing an upper membrane of a shell comprising a shell polymer by using a vapour control module (VCM) D20 mm chamber and heating the upper membrane at 120°C;
Step 3) preparing a vessel of the shell comprising the shell polymer by using a vapour control module (VCM) D20 mm chamber and heating the formed vessel at 120°C, wherein the vessel consisting of a spacer and a lower membrane; and
Step 4) inserting the core prepared in the step 1) into the vessel of the shell prepared in the step 3 and closing the core in the vessel with the upper membrane of the shell prepared in the step 2) and applying a predetermined pressure to seal the upper membrane and the vessel of the shell and heating a formed tablet at 130°C,
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core.

Preferably, the present invention refers to a method for producing the brain implant for linear release of 5-aminolevulinic acid as described above, comprising:
Step 1) Preparing a core comprising 5-aminolevulinic acid (ALA) and a core polymer by using vapour control module (VCM) D10 mm chamber,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
Step 2) preparing an upper membrane of a shell comprising a shell polymer by using a vapour control module (VCM) D20 mm chamber and heating the upper membrane at 120°C;
Step 3) preparing a vessel of the shell comprising the shell polymer by using a vapour control module (VCM) D20 mm chamber and heating the formed vessel at 120°C, wherein the vessel consisting of a spacer and a lower membrane; and
Step 4) inserting the core prepared in the step 1) into the vessel of the shell prepared in the step 3 and closing the core in the vessel with the upper membrane of the shell prepared in the step 2) and applying a predetermined pressure to seal the upper membrane and the vessel of the shell and heating a formed tablet at 130°C,
   characterized in that
   the shell polymer is in a range from 25 wt.% to 55 wt.%, 26 wt.% to 55 wt.%, preferably 27 wt.% to 55 wt.%, more preferably 28 wt.% to 54 wt%, most preferably 30 wt.% to 50 wt% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 26 wt.% to 43 wt.%, preferably 26 wt.% to 42 wt. %, most preferably 26 wt.% to 41 wt. % and the core polymer in a range from 74 wt.% to 56 wt.%, preferably 73 wt.% to 57 wt. %, 72 wt.% to 58 wt. %, most preferably 71 wt.% to 59 wt. %, based on the total weight of the core; or
   when the core polymer is poly(lactic-co-glycolic acid) (PLGA), the core contains 5-aminolevulinic acid in a range from 66 wt.% to 74 wt.% , preferably 67 wt.% to 73 wt. %, 68 wt.% to 72 wt. %, most preferably 69 wt.% to 71 wt. %, and the core polymer in a range from 34 wt.% to 26 wt.% preferably 33 wt.% to 27 wt. %, 32 wt.% to 28 wt.%, most preferably 31 wt.% to 29 wt.%, based on the total weight of the core.

In some preferred embodiments, the present invention refers to a method for producing brain implant for linear release of 5-aminolevulinic acid as descrebied herein,
wherein
in the step 1)
the core further comprises an excipient in a range from 5 wt.% to 15 wt.% based on the total weight of the core, and
the excipient in the core is selected from the group consisting of citric acid, fumaric acid, glycolic acid, lactic acid, malic acid, tartaric acid, maleic acid, succinic acid and a mixture thereof;
   or
in the steps 2) and 3)
the upper membrane and the vessel of the shell each further comprise 5-aminolevulinic acid in a range from 1 wt.% to 15 wt.% based on the total weight of the shell; or
the upper membrane and the vessel of the shell each further comprise an excipient in a range from 35 wt.% to 45 wt.%, based on the total weight of the shell, and the excipient in the shell is selected from polyethylene glycol (PEG).

Thus, the present invention refers to a method for producing the brain implant for linear release of 5-aminolevulinic acid as described above, comprising:
Step 1) Preparing a core comprising 5-aminolevulinic acid (ALA) and a core polymer by using vapour control module (VCM) D10 mm chamber,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
Step 2) preparing an upper membrane of a shell comprising a shell polymer by using a vapour control module (VCM) D20 mm chamber and heating the upper membrane at 120°C;
Step 3) preparing a vessel of the shell comprising the shell polymer by using a vapour control module (VCM) D20 mm chamber and heating the formed vessel at 120°C, wherein the vessel consisting of a spacer and a lower membrane; and
Step 4) inserting the core prepared in the step 1) into the vessel of the shell prepared in the step 3 and closing the core in the vessel with the upper membrane of the shell prepared in the step 2) and applying a predetermined pressure to seal the upper membrane and the vessel of the shell and heating a formed tablet at 130°C,
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core,
   wherein
   in the step 1)
   the core further comprises an excipient in a range from 5 wt.% to 15 wt.% based on the total weight of the core, and
   the excipient in the core is selected from the group consisting of citric acid, fumaric acid, glycolic acid, lactic acid, malic acid, tartaric acid, maleic acid, succinic acid and a mixture thereof;
      or
   in the steps 2) and 3)
   the upper membrane and the vessel of the shell each further comprise 5-aminolevulinic acid in a range from 1 wt.% to 15 wt.% based on the total weight of the shell; or
   the upper membrane and the vessel of the shell each further comprise an excipient in a range from 35 wt.% to 45 wt.%, based on the total weight of the shell, and the excipient in the shell is selected from polyethylene glycol (PEG).

In other words, the present invention refers to a method for producing the brain implant for linear release of 5-aminolevulinic acid as described above, comprising
Step 1) Preparing a core comprising 5-aminolevulinic acid (ALA), a core polymer, and an excipient by using vapour control module (VCM) D10 mm chamber,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   wherein the excipient in the core is in a range from 5 wt.% to 15 wt.% based on the total weight of the core, and
   the excipient in the core is selected from the group consisting of citric acid, fumaric acid, glycolic acid, lactic acid, malic acid, tartaric acid, maleic acid, succinic acid and a mixture thereof; preferably, citric acid, fumaric acid, and a mixture thereof;
Step 2) preparing an upper membrane of a shell comprising a shell polymer by using a vapour control module (VCM) D20 mm chamber and heating the upper membrane at 120°C;
Step 3) preparing a vessel of the shell comprising the shell polymer by using a vapour control module (VCM) D20 mm chamber and heating the formed vessel at 120°C, wherein the vessel consisting of a spacer and a lower membrane; and
Step 4) inserting the core prepared in the step 1) into the vessel of the shell prepared in the step 3 and closing the core in the vessel with the upper membrane of the shell prepared in the step 2) and applying a predetermined pressure to seal the upper membrane and the vessel of the shell and heating a formed tablet at 130°C,
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core;
      or
   a method for producing the brain implant for linear release of 5-aminolevulinic acid as described above, comprising
      Step 1) Preparing a core comprising 5-aminolevulinic acid (ALA) and a core polymer by using vapour control module (VCM) D10 mm chamber,
         wherein the core polymer is selected from the group consisting of poly(lactic acid) (PLA), poly(ethylene oxide) (PEO), poly(ethylene glycol) (PEG), and poly(lactic-co-glycolic acid) (PLGA);
      Step 2) preparing an upper membrane of a shell comprising a shell polymer and 5-aminolevulinic acid by using a vapour control module (VCM) D20 mm chamber and heating the upper membrane at 120°C;
      Step 3) preparing a vessel of the shell comprising the shell polymer and 5-aminolevulinic acid by using a vapour control module (VCM) D20 mm chamber and heating the formed vessel at 120°C, wherein the vessel consisting of a spacer and a lower membrane; and
      Step 4) inserting the core prepared in the step 1) into the vessel of the shell prepared in the step 3 and closing the core in the vessel with the upper membrane of the shell prepared in the step 2) and applying a predetermined pressure to seal the upper membrane and the vessel of the shell and heating a formed tablet at 130°C,
         characterized in that
         the shell polymer is in a range from 20 wt.% to 60 wt.%, preferably 25 wt.% to 55 wt.% based on the total weight of the brain implant; and
         when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
         when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core,
         wherein in the steps 2) and 3)
         in the upper membrane and the vessel of the shell, 5-aminolevulinic acid is in a range from 1 wt.% to 15 wt.% based on the total weight of the shell;
            or
         a method for producing the brain implant for linear release of 5-aminolevulinic acid as described above, comprising
            Step 1) Preparing a core comprising 5-aminolevulinic acid (ALA) and acore polymer by using vapour control module (VCM) D10 mm chamber,
               wherein the core polymer is selected from the group consisting of poly(lactic acid) (PLA), poly(ethylene oxide) (PEO), poly(ethylene glycol) (PEG), and poly(lactic-co-glycolic acid) (PLGA);
            Step 2) preparing an upper membrane of a shell comprising a shell polymer and an expient by using a vapour control module (VCM) D20 mm chamber and heating the upper membrane at 120°C;
            Step 3) preparing a vessel of the shell comprising the shell polymer and the expient by using a vapour control module (VCM) D20 mm chamber and heating the formed vessel at 120°C, wherein the vessel consisting of a spacer and a lower membrane; and
            Step 4) inserting the core prepared in the step 1) into the vessel of the shell prepared in the step 3 and closing the core in the vessel with the upper membrane of the shell prepared in the step 2) and applying a predetermined pressure to seal the upper membrane and the vessel of the shell and heating a formed tablet at 130°C,
               characterized in that
               the shell polymer is in a range from 20 wt.% to 60 wt.%, preferably 25 wt.% to 55 wt.% based on the total weight of the brain implant; and
               when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
               when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core.
               wherein in the upper membrane and the vessel of the shell, the excipient is in a range from 35 wt.% to 45 wt.%, based on the total weight of the shell, and the excipient in the shell is selected from polyethylene glycol (PEG).

Alternatively, the present invention refers to a method for producing the brain implant for linear release of 5-aminolevulinic acid as described above, comprising:
Step 1') preparing a coextruded strand consisting of a core material and a spacer material by using a hot melt co-extrusion device, wherein the core material comprises 5-aminolevulinic acid (ALA) and a core polymer; and the spacer material comprises a shell polymer;
   wherein the core polymer is selected from the group consisting of poly(lactic acid) (PLA), poly(ethylene oxide) (PEO), poly(ethylene glycol) (PEG), and poly(lactic-co-glycolic acid) (PLGA); and the shell polymer is poly(lactic acid) (PLA);
Step 2') cutting the coextruded strand of step 1') to obtain a core coated with a spacer;
Step 3') preparing an upper membrane and a lower membrane from a shell material comprising the shell polymer;
Step 4') sealing the core coated with the spacer of step 2') with the upper membrane and the lower membrane of step 3') by applying a predetermined pressure at a predetermined temperature;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core.

Preferably, the present invention refers to a method for producing the brain implant for linear release of 5-aminolevulinic acid as described above, comprising:
Step 1') preparing a coextruded strand consisting of a core material and a spacer material by using a hot melt co-extrusion device, wherein the core material comprises 5-aminolevulinic acid (ALA) and a core polymer; and the spacer material comprises a shell polymer;
   wherein the core polymer is selected from the group consisting of poly(lactic acid) (PLA), poly(ethylene oxide) (PEO), poly(ethylene glycol) (PEG), and poly(lactic-co-glycolic acid) (PLGA); and the shell polymer is poly(lactic acid) (PLA);
Step 2') cutting the coextruded strand of step 1') to obtain a core coated with a spacer;
Step 3') preparing an upper membrane and a lower membrane from a shell material comprising the shell polymer;
Step 4') sealing the core coated with the spacer of step 2') with the upper membrane and the lower membrane of step 3') by applying a predetermined pressure at a predetermined temperature;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core,
      characterized in that
   the shell polymer is in a range from 25 wt.% to 55 wt.%, 26 wt.% to 55 wt.%, preferably 27 wt.% to 55 wt.%, more preferably 28 wt.% to 54 wt%, most preferably 30 wt.% to 50 wt% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 26 wt.% to 43 wt.%, preferably 26 wt.% to 42 wt. %, most preferably 26 wt.% to 41 wt. % and the core polymer in a range from 74 wt.% to 56 wt.%, preferably 73 wt.% to 57 wt. %, 72 wt.% to 58 wt. %, most preferably 71 wt.% to 59 wt. %, based on the total weight of the core; or
   when the core polymer is poly(lactic-co-glycolic acid) (PLGA), the core contains 5-aminolevulinic acid in a range from 66 wt.% to 74 wt.% , preferably 67 wt.% to 73 wt. %, 68 wt.% to 72 wt. %, most preferably 69 wt.% to 71 wt. %, and the core polymer in a range from 34 wt.% to 26 wt.% preferably 33 wt.% to 27 wt. %, 32 wt.% to 28 wt.%, most preferably 31 wt.% to 29 wt.%, based on the total weight of the core.

**In** some preferred embodiments, the present invention refers to a method for producing brain implant for linear release of 5-aminolevulinic acid as descrebied herein,
wherein
in the step 1')
the core material further comprises an excipient in a range from 5 wt.% to 15 wt.% based on the total weight of the core, and
the excipient in the core is selected from the group consisting of citric acid, fumaric acid, glycolic acid, lactic acid, malic acid, tartaric acid, maleic acid, succinic acid and a mixture thereof;
   or
in the step 3')
the shell material further comprise 5-aminolevulinic acid in a range from 1 wt.% to 15 wt.% based on the total weight of the shell material; or
the shell material further comprise an excipient in a range from 35 wt.% to 45 wt.%, based on the total weight of the shell material, and
the excipient in the shell is selected from polyethylene glycol (PEG).

Thus, the present invention refers to a method for producing the brain implant for linear release of 5-aminolevulinic acid as described above, comprising:
Step 1') preparing a coextruded strand consisting of a core material and a spacer material by using a hot melt co-extrusion device, wherein the core material comprises 5-aminolevulinic acid (ALA), a core polymer and an excipient, and the spacer material comprises a shell polymer;
   wherein the core polymer is selected from the group consisting of poly(lactic acid) (PLA), poly(ethylene oxide) (PEO), poly(ethylene glycol) (PEG), and poly(lactic-co-glycolic acid) (PLGA); and the shell polymer is poly(lactic acid) (PLA);
Step 2') cutting the coextruded strand of step 1') to obtain a core coated with a spacer;
Step 3') preparing an upper membrane and a lower membrane from a shell material comprising the shell polymer;
Step 4') sealing the core coated with the spacer of step 2') with the upper membrane and the lower membrane of step 3') by applying a predetermined pressure at a predetermined temperature;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core;
      wherein
   the excipient in the core in a range from 5 wt.% to 15 wt.% based on the total weight of the core, and
   the excipient in the core is selected from the group consisting of citric acid, fumaric acid, glycolic acid, lactic acid, malic acid, tartaric acid, maleic acid, succinic acid and a mixture thereof; preferably citric acid, fumaric acid, and a mixture thereof,
      or
   in the step 3')
   the shell material further comprise 5-aminolevulinic acid in a range from 1 wt.% to 15 wt.% based on the total weight of the shell material; or
   the shell material further comprise an excipient in a range from 35 wt.% to 45 wt.%, based on the total weight of the shell material, and
   the excipient in the shell is selected from polyethylene glycol (PEG).

Thus, the present invention refers to a method for producing the brain implant for linear release of 5-aminolevulinic acid as described above, comprising
Step 1') preparing a coextruded strand consisting of a core material and a spacer material by using a hot melt co-extrusion device, wherein the core material comprises 5-aminolevulinic acid (ALA) and a core polymer; and the spacer material comprises a shell polymer;
   wherein the core polymer is selected from the group consisting of poly(lactic acid) (PLA), poly(ethylene oxide) (PEO), poly(ethylene glycol) (PEG), and poly(lactic-co-glycolic acid) (PLGA); and the shell polymer is poly(lactic acid) (PLA);
Step 2') cutting the coextruded strand of step 1') to obtain a core coated with a spacer;
Step 3') preparing an upper membrane and a lower membrane from a shell material comprising the shell polymer;
Step 4') sealing the core coated with the spacer of step 2') with the upper membrane and the lower membrane of step 3') by applying a predetermined pressure at a predetermined temperature;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core;
   wherein
   in the step 1')
   the core material further comprises an excipient in a range from 5 wt.% to 15 wt.% based on the total weight of the core, and
   the excipient in the core is selected from the group consisting of citric acid, fumaric acid, glycolic acid, lactic acid, malic acid, tartaric acid, maleic acid, succinic acid and a mixture thereof;
      or
   a method for producing the brain implant for linear release of 5-aminolevulinic acid as described above, comprising
      Step 1') preparing a coextruded strand consisting of a core material and a spacer material by using a hot melt co-extrusion device, wherein the core material comprises 5-aminolevulinic acid (ALA) and a core polymer; and the spacer material comprises a shell polymer;
         wherein the core polymer is selected from the group consisting of poly(lactic acid) (PLA), poly(ethylene oxide) (PEO), poly(ethylene glycol) (PEG), and poly(lactic-co-glycolic acid) (PLGA); and the shell polymer is poly(lactic acid) (PLA);
      Step 2') cutting the coextruded strand of step 1') to obtain a core coated with a spacer;
      Step 3') preparing an upper membrane and a lower membrane from a shell material comprising the shell polymer;
      Step 4') sealing the core coated with the spacer of step 2') with the upper membrane and the lower membrane of step 3') by applying a predetermined pressure at a predetermined temperature;
         characterized in that
         the shell polymer is in a range from 20 wt.% to 60 wt.%, preferably 25 wt.% to 55 wt.% based on the total weight of the brain implant; and
         when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
         when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core;
         wherein
         in the step 3')
         the shell material further comprise 5-aminolevulinic acid in a range from 1 wt.% to 15 wt.% based on the total weight of the shell material;
      or
   a method for producing the brain implant for linear release of 5-aminolevulinic acid as described above, comprising
      Step 1') preparing a coextruded strand consisting of a core material and a spacer material by using a hot melt co-extrusion device, wherein the core material comprises 5-aminolevulinic acid (ALA) and a core polymer; and the spacer material comprises a shell polymer;
         wherein the core polymer is selected from the group consisting of poly(lactic acid) (PLA), poly(ethylene oxide) (PEO), poly(ethylene glycol) (PEG), and poly(lactic-co-glycolic acid) (PLGA); and the shell polymer is poly(lactic acid) (PLA);
      Step 2') cutting the coextruded strand of step 1') to obtain a core coated with a spacer;
      Step 3') preparing an upper membrane and a lower membrane from a shell material comprising the shell polymer;
      Step 4') sealing the core coated with the spacer of step 2') with the upper membrane and the lower membrane of step 3') by applying a predetermined pressure at a predetermined temperature;
         characterized in that
         the shell polymer is in a range from 20 wt.% to 60 wt.%, preferably 25 wt.% to 55 wt.% based on the total weight of the brain implant; and
         when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
         when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core;
         wherein
         in the step 3')
         the shell material further comprise an excipient in a range from 35 wt.% to 45 wt.%, based on the total weight of the shell material, and
         the excipient in the shell is selected from polyethylene glycol (PEG).

In other words, the present invention refers to a method for producing the brain implant for linear release of 5-aminolevulinic acid as described above, comprising
Step 1') preparing a coextruded strand consisting of a core material and a spacer material by using a hot melt co-extrusion device, wherein the core material comprises 5-aminolevulinic acid (ALA), a core polymer and an excipient; and the spacer material comprises a shell polymer;
   wherein the core polymer is selected from the group consisting of poly(lactic acid) (PLA), poly(ethylene oxide) (PEO), poly(ethylene glycol) (PEG), and poly(lactic-co-glycolic acid) (PLGA); and the shell polymer is poly(lactic acid) (PLA);
Step 2') cutting the coextruded strand of step 1') to obtain a core coated with a spacer;
Step 3') preparing an upper membrane and a lower membrane from a shell material comprising the shell polymer;
Step 4') sealing the core coated with the spacer of step 2') with the upper membrane and the lower membrane of step 3') by applying a predetermined pressure at a predetermined temperature;
   characterized in that
   the shell polymer is in a range from 20 wt.% to 60 wt.%, preferably 25 wt.% to 55 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core;
   wherein
   the excipient in the core is in a range from 5 wt.% to 15 wt.% based on the total weight of the core, and
   the excipient in the core is selected from the group consisting of citric acid, fumaric acid, glycolic acid, lactic acid, malic acid, tartaric acid, maleic acid, succinic acid and a mixture thereof; preferably citric acid, fumaric acid, and a mixture thereof;
   or
   a method for producing the brain implant for linear release of 5-aminolevulinic acid as described above, comprising
      Step 1') preparing a coextruded strand consisting of a core material and a spacer material by using a hot melt co-extrusion device, wherein the core material comprises 5-aminolevulinic acid (ALA) and a core polymer; and the spacer material comprises a shell polymer;
         wherein the core polymer is selected from the group consisting of poly(lactic acid) (PLA), poly(ethylene oxide) (PEO), poly(ethylene glycol) (PEG), and poly(lactic-co-glycolic acid) (PLGA); and the shell polymer is poly(lactic acid) (PLA);
      Step 2') cutting the coextruded strand of step 1') to obtain a core coated with a spacer;
      Step 3') preparing an upper membrane and a lower membrane from a shell material comprising the shell polymer and 5-aminolevulinic acid,
         wherein 5-aminolevulinic acid in a range from 1 wt.% to 15 wt.% based on the total weight of the shell material;
      Step 4') sealing the core coated with the spacer of step 2') with the upper membrane and the lower membrane of step 3') by applying a predetermined pressure at a predetermined temperature;
         characterized in that
         the shell polymer is in a range from 20 wt.% to 60 wt.%, preferably 25 wt.% to 55 wt.% based on the total weight of the brain implant; and
         when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
         when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core;
      or
   a method for producing the brain implant for linear release of 5-aminolevulinic acid as described above, comprising
      Step 1') preparing a coextruded strand consisting of a core material and a spacer material by using a hot melt co-extrusion device, wherein the core material comprises 5-aminolevulinic acid (ALA) and a core polymer; and the spacer material comprises a shell polymer;
         wherein the core polymer is selected from the group consisting of poly(lactic acid) (PLA), poly(ethylene oxide) (PEO), poly(ethylene glycol) (PEG), and poly(lactic-co-glycolic acid) (PLGA); and the shell polymer is poly(lactic acid) (PLA);
      Step 2') cutting the coextruded strand of step 1') to obtain a core coated with a spacer;
      Step 3') preparing an upper membrane and a lower membrane from a shell material comprising the shell polymer and an excipient;
         wherein the excipient is in a range from 35 wt.% to 45 wt.%, based on the total weight of the shell material, and
         the excipient in the shell is selected from polyethylene glycol (PEG);
      Step 4') sealing the core coated with the spacer of step 2') with the upper membrane and the lower membrane of step 3') by applying a predetermined pressure at a predetermined temperature;
         characterized in that
         the shell polymer is in a range from 20 wt.% to 60 wt.%, preferably 25 wt.% to 55 wt.% based on the total weight of the brain implant; and
         when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
         when the core polymer is poly(lactic-co-glycolic acid) (PLGA), the core contains 5-aminolevulinic acid in a range from 65 wt.% to 75 wt.% and the core polymer in a range from 35 wt.% to 25 wt.%, based on the total weight of the core.

### Medical Use

Suprisingly, the brain implants of the present invention comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
      and
   b) a shell consisting of an upper membrane, a spacer, and a lower membrane, wherein the upper membrane, the spacer, and the lower membrane each comprise a shell polymer, wherein the shell polymer is poly(lactic acid) (PLA);
      characterized in that
      the shell polymer is in a range from 20 wt.% to 60 wt.% based on the total weight of the brain implant; and
      when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
      when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core
      demonstrated the continuous release of a therapeutically effective amount of 5-aminolevulinic acid (5-ALA), covering the linear release of 5-ALA for at least 3 days, preferably at least 4 days, more preferably at least 5 days when being implanted into a resection cavity after at least partial surgical resection of a brain tumor.

Surprisingly, the inventors have found that low dose and continuous intracranial or intratumoral release of 5-aminolevulinic acid (5-ALA) by the brain implants disclosed herein for several days and up to weeks post-surgery is highly tolerable and shows superior efficacy over other known application routes in the treatment of brain tumor.

While intermittent intracranial bolus injections of 5-ALA in mice (initially 100 µg/day, reduced to 50 µg/day due to toxicity) showed a positive trend toward tumor growth restriction (see Example 5A), this regimen was associated with systemic adverse effects, including weight loss and reduced survival.

Switching to a continuous, low-dose intracranial release of 5-ALA (delivering 50 µg/day) by a brain implantant according to the invention yielded robust antitumor activity without compromising body weight (see Example 5B). Notably, this approach was more effective than a systemic high-dose (120 mg/kg) regimen, underscoring the benefits of localized, continuous drug delivery by the brain implant according to the invention.

The inventors were able to further demonstrate the favorable safety profile of the inventive brain implants in mouse studies. In healthy, non-tumor-bearing mice, the continuous intracranial delivery of 5-ALA over one month did not affect body weight, blood cell parameters, or serum toxicity markers (see Example 5C).

In addition, testing various 5-ALA doses, either alone or in combination with ARS (artesunate 1e), revealed that even an extremely low 5-ALA dose (10 µg/day) significantly reduced tumor growth in mice, while maintaining excellent tolerability (see Example 5D).

Although continuous intracranial 5-ALA delivery alone significantly reduced tumor growth (see Example 5E), its combination with orally administered ARS produced a synergistic antitumor effect. ARS administered as a monotherapy did not limit tumor growth, highlighting the enhanced efficacy achieved through their combination (see Example 5F).

Consequently, the experimental data provided herein substantiate that continuous, low-dose intracranial release of 5-ALA by the brain implant according to the invention, either alone or in combination with oral administration of ARS, provides a synergistic, potent antineoplastic effect in brain tumor models while minimizing systemic toxicity. This innovative brain implant offers a promising, safe, and effective therapeutic approach for the treatment of brain tumors.

Thus, another aspect of the present invention is directed to a brain implant described herein for use in the prophylaxis and/or treatment of brain cancer. Thus, the present invention is also directed to a brain implant for linear release of 5-aminolevulinic acid for use in the prophylaxis and/or treatment of brain cancer, the brain implant comprising
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

In another embodiment the brain implant described herein is used in the treatment of brain cancer.

The term "at least partial surgical resection" as used herein refers to the surgical procedure in which a portion or entirety of a brain tumor mass is removed from the brain. This includes partial resection, i.e. removal of some, but not all, of the tumor tissue, which may be necessitated by factors such as the tumor's proximity to critical brain structures, patient health considerations, or surgical feasibility, and total resection, i.e. complete removal of the visible tumor mass as determined by intraoperative imaging or postoperative assessment. Thus, the term "at least partial surgical resection" encompasses any surgical intervention aimed at debulking the tumor, reducing its volume, or excising as much of the pathological tissue as is safely achievable, with the intent of minimizing symptoms, improving prognosis, or facilitating adjunct therapies such as radiation or chemotherapy.

A "therapeutically effective amount" or "therapeutically effective dosage" of the composition, 5-ALA or a therapeutic agent, is any amount of said composition, 5-ALA or the therapeutic agent that, when used alone or in combination with another therapeutic agent after at least partial surgical resection of the brain tumor, prevents or slows tumor regrowth/recurrence, protects a subject against the onset of the brain tumor or promotes tumor regression evidenced by a decrease in severity of cancer symptoms, particularly tumor size, an increase in frequency and duration of disease symptom-free periods, or a prevention of impairment or disability due to the disease affliction. The ability of the therapeutic agent to promote tumor regression can be evaluated using a variety of methods known to the skilled practitioner, such as in human subjects during clinical trials, in animal model systems predictive of efficacy in humans, or by assaying the activity of the agent in *in vitro* assays. By way of example, an anti-tumor agent promotes tumor regression in a subject. In preferred embodiments, a therapeutically effective amount of 5-ALA promotes tumor regression to the point of eliminating the brain tumor. "Promoting cancer regression" means that administering an effective amount of 5-ALA, alone or in combination with an anti-cancer agent, results in a reduction in tumor growth or size, necrosis of the tumor, a decrease in severity of at least one disease symptom, an increase in frequency and duration of disease symptom-free periods, or a prevention of impairment or disability due to the disease affliction. In addition, the terms "effective" and "effectiveness" with regard to a treatment includes both pharmacological effectiveness and physiological safety. Pharmacological effectiveness refers to the ability of the drug to promote cancer regression in the patient. Physiological safety refers to the level of toxicity, or other adverse physiological effects at the cellular, organ and/or organism level (adverse effects) resulting from administration of the agent.

As used herein, the terms "5-aminolevulinic acid", "delta-aminolevulinic acid", "ALA" and "5-ALA" are used interchangeably and encompass 5-amino-4-oxopentanoic acid.

The term "5-aminolevulinic acid" as well as the synonyms thereof encompasses also salts such as the hydrochloride, hydrobromide, and hydroiodide salt and especially the hydrochloride salt thereof.

Suitable salts of "5-aminolevulinic acid" include, but are not limited to, salts with acids such as maleic, fumaric, benzoic, ascorbic, succinic, acetic, formic, oxalic, propionic, tartaric, salicylic, citric, gluconic, lactic, mandelic, cinnamic, oleic, tannic, aspartic, stearic, palmitic, glycolic, glutamic, gluconic, glucaronic, saccharic, isonicotinic, methanesulfonic, ethanesulfonic, p-toluenesulfonic, benzenesulfonic acids, or pamoic (e.g, 1,1'-methylene-bis-(2-hydroxy-3-naphthoate)) acid. Suitable inorganic acids include, but are not limited to, hydrochloric, hydrobromic, hydroiodic, sulfuric, phosphoric, or nitric acid. Preferred is the HCl salt of 5-ALA.

According to the present invention, doses of the composition, of 5-aminolevulinic acid or any other therapeutic agent are expressed as a free base. In other words, even if the considered agent is a pharmaceutically acceptable salt of a small molecule, e.g. the hydrochloride salt of 5-aminolevulinic acid, the doses and daily doses are expressed herein on the bases of the corresponding small molecule free base, e.g. 5-aminolevulinic acid. In the *in vivo* experiments disclosed herein the HCl salt of 5-ALA was used.

The term "shell" refers to the part of an intracranial drug delivery system that completely covers the core. The shell consists of an upper membrane which covers the top of the core, a spacer which horizontally surrounds the core, and a lower membrane which covers the bottom of the core.

The term "wt.%" refers to >>weight %<< and >>percent per weight<<.

The disclosure a component, such as the core, consist of 25 wt.% to 75 wt.% of compound A and 75 wt.% to 25 wt.% of compound B has to be understood in the way that both compounds together have to add up to 100 wt.%.

Consequently, the disclosure
a) a core consisting of:
   5-aminolevulinic acid in a range from 25 wt.% to 75 wt.%,
   the core polymer in a range from 75 wt.% to 25 wt.%, and
   optionally the excipient in the core in a range of 5 wt.% to 15 wt.%, based on the total weight of the core,
   has to be understood in the way that in case the excipient is present, e.g. in an amount of 15 wt.%, the remaining 5-ALA and core polymer have to add up to 85 wt.% so that all ingredients together add up to 100 wt.%. Consequently, the core polymer cannot be present in the amount of 75 wt.% anymore, because 5-ALA must be present at least in the amount of 25 wt.% so that the maximum amount of the core polymer is 60 wt.%.

As used herein, the term "treating" or "treatment" encompasses to reversing, alleviating or inhibiting the progress of a disease, disorder or condition, or improvement of one or more symptoms of such disease, disorder or condition, to which such term applies. As used herein, "treating" or "treatment" may also refer to decreasing the probability or incidence of the occurrence of a disease, disorder or condition in a mammal as compared to an untreated control population, or as compared to the same mammal prior to treatment. For example, as used herein, "treating" may refer to preventing a disease, disorder or condition, and may include delaying or preventing the onset of a disease, disorder or condition, or delaying or preventing the symptoms associated with a disease, disorder or condition. As used herein, "treating" may also refer to reducing the severity of a disease, disorder or condition or symptoms associated with such disease, disorder or condition prior to a mammal's affliction with the disease, disorder or condition. Such prevention or reduction of the severity of a disease, disorder or condition prior to affliction relates to the administration of the composition of the present invention, as described herein, to a subject that is not at the time of administration afflicted with the disease, disorder or condition. As used herein the term "treating" may also refer to preventing the recurrence of a disease, disorder or condition or of one or more symptoms associated with such disease, disorder or condition. The terms "treatment" and "therapeutically," as used herein, refer to the act of treating, as "treating" is defined above.

Preferably, a disease, disorder is a cancer selected from brain cancer, in particular glioblastoma.

The term "patient" or "subject," as used herein, refers to a mammalian subject (primates (e.g. humans, cows, sheep, goats, pigs, horses, dogs, cats, rabbits, rats, mice and the like), preferably a human subject (e.g. a man, a woman or a child), that has, is suspected of having, or is or may be susceptible to a condition associated with brain cancer

The term "resection cavity" refers to an anatomical space created in the brain after the surgical removal of a tumor or parts of a tumor. It comprises the tumor bed, i.e. the area that was occupied by the tumor, and may include surrounding peritumoral tissue that potentially harbors residual microscopic tumor cells. This cavity is clinically significant because it serves as a treatment target (such as direct drug delivery systems) that aim to locally release therapeutic agents to eliminate remaining infiltrative tumor cells and reduce the risk of recurrence.

The term "released into resection cavity" refers to the direct release of the composition comprising 5-ALA into the resection cavity and its immediately adjacent (peritumoral) brain tissue after at least partial surgical resection of the brain tumor. Said release mode is designed to target residual infiltrative tumor cells that remain at the margins of the resection cavity and are a primary cause of recurrence, thereby helping to prevent recurrence while minimizing systemic toxicity.

The term "in a controlled-release manner" refers to the delivery or release of a composition or therapeutic agent such as ARS at a predetermined and regulated rate over a specified period. This mode of release is designed to maintain consistent therapeutic levels of the composition in the target site, minimizing fluctuations in drug concentration that could lead to subtherapeutic effects or toxicity. Controlled release encompasses, but is not necessarily limited to, substantially continuous delivery, and patterned delivery (e.g., intermittent delivery over a period of time that is interrupted by regular or irregular time intervals).

Preferably, the brain implant as described herein is useful for the prophylaxis and/or treatment of the brain cancer, wherein the brain cancer is selected from subtypes proneural (PN), mesenchymal (MES), and classical (CL) glioblastoma, pilocytic astrocytoma, pleomorphic xanthoastrocytoma, subependymal giant cell astrocytoma, fibrillary astrocytoma, anaplastic astrocytoma, oligodendrogliomas, anaplastic oligodendroglioma, ependymoma, subependymoma, choroid plexus tumor, choroid plexus papilloma, and choroid plexus carcinoma.

More preferably, the brain implant as described herein is useful for the prophylaxis and/or treatment of the brain cancer, wherein the brain cancer is selected from subtypes proneural (PN), mesenchymal (MES), and classical (CL) glioblastoma.

Most preferably, the brain implants as described herein is useful for the treatment of glioblastoma including recurrent glioblastoma .

Thus, in a preferred embodiment the brain implant for use in the prophylaxis and/or treatment of glioblastoma comprises
a) a core comprising:
   5-aminolevulinic acid and a core polymer,
   wherein the core polymer is selected from the group consisting of poly(lactic acid) (PLA), poly(ethylene oxide) (PEG), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEG), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core.

In a preferred embodiment, the brain implant described herein releases a therapeutically effective amount of 5-ALA continuously over 1 to 30 days, preferably 7 to 29 days, more preferably 14 to 28 days and linearly over 3 to 5 days. Thus, in a preferred embodiment the brain implant for use in the prophylaxis and/or treatment of brain cancer comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, continuously releases a therapeutically effective amount of 5-ALA continuously over 1 to 30 days, preferably 7 to 29 days, more preferably 14 to 28 days and linearly over 3 to 5 days to the resection cavity in a continuous release manner after at least partial surgical resection of the brain cancer.

In a preferred embodiment, the therapeutically effective amount of 5-ALA is linearly released into the resection cavity over at least 4 days. Thus, in one embodiment, the brain implant for use in the prophylaxis and/or treatment of brain cancer comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
      and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA over at least 4 days to the resection cavity after at least partial surgical resection of the brain cancer.

Preferably, the therapeutically effective amount of 5-ALA is continuously released into the resection cavity over at least 4 days, more preferably at least 5 days, more preferably at least 6 days, more preferably at least 7 days, more preferably at least 8 days, more preferably at least 9 days, more preferably at least 10 days, more preferably at least 11 days, more preferably at least 12 days, more preferably at least 13 days, more preferably at least 14 days, more preferably at least 15 days, more preferably at least 16 days, more preferably at least 17 days, more preferably at least 18 days, more preferably at least 19 days, more preferably at least 20 days, more preferably at least 21 days, more preferably at least 22 days, more preferably at least 23 days, more preferably at least 24 days, more preferably at least 25 days, more preferably at least 26 days, more preferably at least 27 days, more preferably at least 28 days, more preferably at least 29 days, and more preferably at least 30 days after at least partial surgical resection of the brain cancer.

In an alternative embodiment, the therapeutically effective amount of 5-ALA is continuously released into the resection cavity between 2 to 40 days or 4 days to 30 days, more preferably between 5 days and 30 days, more preferably between 10 days and 29 days, more preferably between 14 days and 28 days, and most preferably between 16 days and 27 days after at least partial surgical resection of the brain cancer.

In an alternative embodiment, the therapeutically effective amount of 5-ALA is continuously released into the resection cavity between 3 days to 15 days, more preferably between 4 days and 14 days, more preferably between 5 days and 13 days, more preferably between 6 days and 12 days, and most preferably between 7 days and 11 days after at least partial surgical resection of the brain cancer.

**In** an alternative embodiment, the therapeutically effective amount of 5-ALA is continuously released into the resection cavity between 8 days to 20 days, more preferably between 9 days and 19 days, more preferably between 10 days and 18 days, more preferably between 11 days and 17 days, and most preferably between 12 days and 16 days after at least partial surgical resection of the brain cancer.

**In** an alternative embodiment, the therapeutically effective amount of 5-ALA is continuously released into the resection cavity between 13 days to 25 days, more preferably between 14 days and 24 days, more preferably between 15 days and 23 days, more preferably between 16 days and 22 days, and most preferably between 17 days and 21 days after at least partial surgical resection of the brain cancer.

In an alternative embodiment, the therapeutically effective amount of 5-ALA is continuously released into the resection cavity between 18 days to 30 days, more preferably between 19 days and 29 days, more preferably between 20 days and 28 days, more preferably between 21 days and 27 days, and most preferably between 22 days and 26 days after at least partial surgical resection of the brain cancer.

In an alternative embodiment, the therapeutically effective amount of 5-ALA is continuously released into the resection cavity between 23 days to 35 days, more preferably between 24 days and 34 days, more preferably between 25 days and 33 days, more preferably between 26 days and 32 days, and most preferably between 27 days and 30 days after at least partial surgical resection of the brain cancer.

In a preferred embodiment, the brain implant for use in the prophylaxis and/or treatment of brain cancer comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
      and
   b) a shell consisting of an upper membrane, a spacer, and a lower membrane, wherein the upper membrane, the spacer, and the lower membrane each comprise a shell polymer, wherein the shell polymer is poly(lactic acid) (PLA);
      characterized in that
      the shell polymer is in a range from 20 wt.% to 60 wt.% based on the total weight of the brain implant; and
      when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
      when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
      wherein the brain implant, when in use, continuously releases a therapeutically effective amount of 5-ALA at a constant rate of 0.1 mg - 100 mg per day into the resection cavity after at least partial surgical resection of the brain cancer

Preferably, the therapeutically effective amount of 5-ALA is continuously released at a constant rate of 0.1 mg - 100 mg per day, more preferably at a constant rate of 0.15 mg - 90 mg per day, more preferably at a constant rate of 0.2 mg - 80 mg per day, more preferably at a constant rate of 0.25 mg - 70 mg per day, more preferably at a constant rate of 0.3 mg - 60 mg per day, more preferably at a constant rate of 0.35 mg - 50 mg per day, more preferably at a constant rate of 0.4 mg - 40 mg per day, more preferably at a constant rate of 0.42 mg - 30 mg per day, more preferably at a constant rate of 0.45 mg - 20 mg per day, and most preferably at a constant rate of 0.5 mg - 10 mg per day. Preferably the linear release in these preferred constant rates is linear over 3 to 10 days or at least 3 days, preferably at least 4 days, and more preferably at least 5 days.

In a preferred embodiment, the brain implant for use in the prophylaxis and/or treatment of brain cancer comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA at a rate of 0.5 mg - 50 mg per day, preferably 1.0 mg - 40 mg per day, more preferably 2.0 mg - 30 mg per day, and most preferably 3.0 mg - 20 mg per day into the resection cavity after at least partial surgical resection of the brain cancer

**In** a preferred embodiment, the brain implant for use in the prophylaxis and/or treatment of brain cancer comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA at a rate of 0.5 mg - 20 mg per day into the resection cavity after at least partial surgical resection of the brain cancer

In an alternative embodiment, the therapeutically effective amount of 5-ALA is linearly over 3 to 10 days or continuously released at a rate of about 0.1 mg per day; more preferably at a rate of about 0.2 mg per day; more preferably at a rate of about 0.3 mg per day; more preferably at a rate of about 0.4 mg per day; more preferably at a rate of about 0.5 mg per day; more preferably at a rate of about 0.6 mg per day; more preferably at a rate of about 0.7 mg per day; more preferably at a rate of about 0.8 mg per day; more preferably at a rate of about 0.9 mg per day; more preferably at a rate of about 1.0 mg per day; more preferably at a rate of about 1.1 mg per day; more preferably at a rate of about 1.2 mg per day; more preferably at a rate of about 1.3 mg per day; more preferably at a rate of about 1.4 mg per day; more preferably at a rate of about 1.5 mg per day; more preferably at a rate of about 1.6 mg per day; more preferably at a rate of about 1.7 mg per day; more preferably at a rate of about 1.8 mg per day; more preferably at a rate of about 1.9 mg per day; more preferably at a rate of about 2.0 mg per day; more preferably at a rate of about 2.1 mg per day; more preferably at a rate of about 2.2 mg per day; more preferably at a rate of about 2.3 mg per day; more preferably at a rate of about 2.4 mg per day; more preferably at a rate of about 2.5 mg per day; more preferably at a rate of about 2.6 mg per day; more preferably at a rate of about 2.7 mg per day; more preferably at a rate of about 2.8 mg per day; more preferably at a rate of about 2.9 mg per day; more preferably at a rate of about 3.0 mg per day; more preferably at a rate of about 3.1 mg per day; more preferably at a rate of about 3.2 mg per day; more preferably at a rate of about 3.3 mg per day; more preferably at a rate of about 3.4 mg per day; more preferably at a rate of about 3.5 mg per day; more preferably at a rate of about 3.6 mg per day; more preferably at a rate of about 3.7 mg per day; more preferably at a rate of about 3.8 mg per day; more preferably at a rate of about 3.9 mg per day; more preferably at a rate of about 4.0 mg per day; more preferably at a rate of about 4.1 mg per day; more preferably at a rate of about 4.2 mg per day; more preferably at a rate of about 4.3 mg per day; more preferably at a rate of about 4.4 mg per day; more preferably at a rate of about 4.5 mg per day; more preferably at a rate of about 4.6 mg per day; more preferably at a rate of about 4.7 mg per day; more preferably at a rate of about 4.8 mg per day; more preferably at a rate of about 4.9 mg per day; more preferably at a rate of about 5.0 mg per day; more preferably at a rate of about 5.1 mg per day; more preferably at a rate of about 5.2 mg per day; more preferably at a rate of about 5.3 mg per day; more preferably at a rate of about 5.4 mg per day; more preferably at a rate of about 5.5 mg per day; more preferably at a rate of about 5.6 mg per day; more preferably at a rate of about 5.7 mg per day; more preferably at a rate of about 5.8 mg per day; more preferably at a rate of about 5.9 mg per day; more preferably at a rate of about 6.0 mg per day; more preferably at a rate of about 6.1 mg per day; more preferably at a rate of about 6.2 mg per day; more preferably at a rate of about 6.3 mg per day; more preferably at a rate of about 6.4 mg per day; more preferably at a rate of about 6.5 mg per day; more preferably at a rate of about 6.6 mg per day; more preferably at a rate of about 6.7 mg per day; more preferably at a rate of about 6.8 mg per day; more preferably at a rate of about 6.9 mg per day; more preferably at a rate of about 7.0 mg per day; more preferably at a rate of about 7.1 mg per day; more preferably at a rate of about 7.2 mg per day; more preferably at a rate of about 7.3 mg per day; more preferably at a rate of about 7.4 mg per day; more preferably at a rate of about 7.5 mg per day; more preferably at a rate of about 7.6 mg per day; more preferably at a rate of about 7.7 mg per day; more preferably at a rate of about 7.8 mg per day; more preferably at a rate of about 7.9 mg per day; more preferably at a rate of about 8.0 mg per day; more preferably at a rate of about 8.1 mg per day; more preferably at a rate of about 8.2 mg per day; more preferably at a rate of about 8.3 mg per day; more preferably at a rate of about 8.4 mg per day; more preferably at a rate of about 8.5 mg per day; more preferably at a rate of about 8.6 mg per day; more preferably at a rate of about 8.7 mg per day; more preferably at a rate of about 8.8 mg per day; more preferably at a rate of about 8.9 mg per day; more preferably at a rate of about 9.0 mg per day; more preferably at a rate of about 9.1 mg per day; more preferably at a rate of about 9.2 mg per day; more preferably at a rate of about 9.3 mg per day; more preferably at a rate of about 9.4 mg per day; more preferably at a rate of about 9.5 mg per day; more preferably at a rate of about 9.6 mg per day; more preferably at a rate of about 9.7 mg per day; more preferably at a rate of about 9.8 mg per day; more preferably at a rate of about 9.9 mg per day; and most preferably at a rate of about 10.0 mg per day.

In an alternative embodiment, the therapeutically effective amount of 5-ALA is linearly over at least 3 days or continuously released at a rate of about 100 mg per day; more preferably at a rate of about 98 mg per day; more preferably at a rate of about 96 mg per day; more preferably at a rate of about 94 mg per day; more preferably at a rate of about 92 mg per day; more preferably at a rate of about 90 mg per day; more preferably at a rate of about 88 mg per day; more preferably at a rate of about 86 mg per day; more preferably at a rate of about 84 mg per day; more preferably at a rate of about 82 mg per day; more preferably at a rate of about 80 mg per day; more preferably at a rate of about 78 mg per day; more preferably at a rate of about 76 mg per day; more preferably at a rate of about 74 mg per day; more preferably at a rate of about 72 mg per day; more preferably at a rate of about 70 mg per day; more preferably at a rate of about 68 mg per day; more preferably at a rate of about 66 mg per day; more preferably at a rate of about 64 mg per day; more preferably at a rate of about 62 mg per day; more preferably at a rate of about 60 mg per day; more preferably at a rate of about 58 mg per day; more preferably at a rate of about 56 mg per day; more preferably at a rate of about 54 mg per day; more preferably at a rate of about 52 mg per day; more preferably at a rate of about 50 mg per day; more preferably at a rate of about 48 mg per day; more preferably at a rate of about 46 mg per day; more preferably at a rate of about 44 mg per day; more preferably at a rate of about 42 mg per day; more preferably at a rate of about 40 mg per day; more preferably at a rate of about 38 mg per day; more preferably at a rate of about 36 mg per day; more preferably at a rate of about 34 mg per day; more preferably at a rate of about 32 mg per day; more preferably at a rate of about 30 mg per day; more preferably at a rate of about 28 mg per day; more preferably at a rate of about 26 mg per day; more preferably at a rate of about 24 mg per day; more preferably at a rate of about 22 mg per day; more preferably at a rate of about 20 mg per day; more preferably at a rate of about 19 mg per day; more preferably at a rate of about 18 mg per day; more preferably at a rate of about 17 mg per day; more preferably at a rate of about 16 mg per day; more preferably at a rate of about 15 mg per day; more preferably at a rate of about 14 mg per day; more preferably at a rate of about 13 mg per day; more preferably at a rate of about 12 mg per day; more preferably at a rate of about 11 mg per day; more preferably at a rate of about 10 mg per day; more preferably at a rate of about 9 mg per day; more preferably at a rate of about 8 mg per day; more preferably at a rate of about 7 mg per day; more preferably at a rate of about 6 mg per day; more preferably at a rate of about 5 mg per day; more preferably at a rate of about 4.5 mg per day; more preferably at a rate of about 4 mg per day; more preferably at a rate of about 3.5 mg per day; more preferably at a rate of about 3.0 mg per day; more preferably at a rate of about 2.5 mg per day; more preferably at a rate of about 2.0 mg per day; more preferably at a rate of about 1.5 mg per day; more preferably at a rate of about 1.0 mg per day; more preferably at a rate of about 0.9 mg per day; more preferably at a rate of about 0.8 mg per day; more preferably at a rate of about 0.7 mg per day; more preferably at a rate of about 0.6 mg per day; and most preferably at a rate of about 0.5 mg per day.

In a preferred embodiment, the brain implant for use in the prophylaxis and/or treatment of brain cancer comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, continuously releases a therapeutically effective amount of 5-ALA into the resection cavity over 1 to 50 days or 1 to 40 days or 1 to 30 days after at least partial surgical resection of the brain tumor, and wherein the 5-aminolevulinic acid is linearly over 3 to 10 days or continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day.

In a preferred embodiment, the brain implant for use in the prophylaxis and/or treatment of brain cancer comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, continuously releases a therapeutically effective amount of 5-ALA into the resection cavity for at least 3 days after at least partial surgical resection of the brain tumor, wherein the 5-aminolevulinic acid is linearly over 3 to 10 days or continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day.

In a preferred embodiment, the brain implant for use in the prophylaxis and/or treatment of brain cancer comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly over 3 to 10 days or continuously releases a therapeutically effective amount of 5-ALA into the resection cavity for at least 10 days after at least partial surgical resection of the brain tumor, wherein the 5-aminolevulinic acid is continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day.

In a preferred embodiment, the brain implant for use in the prophylaxis and/or treatment of brain cancer comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, continuously releases a therapeutically effective amount of 5-ALA into the resection cavity for at least 10 days after at least partial surgical resection of the brain tumor, wherein the 5-aminolevulinic acid is linearly over 3 to 10 days or continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day, wherein the brain cancer is glioblastoma.

In a preferred embodiment, the brain implant for use in the prophylaxis and/or treatment of brain cancer comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, continuously releases a therapeutically effective amount of 5-ALA into the resection cavity for at least 20 days after at least partial surgical resection of the brain tumor, wherein the 5-aminolevulinic acid is linearly over 3 to 10 days or continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day.

In a preferred embodiment, the brain implant for use in the prophylaxis and/or treatment of brain cancer comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly over 3 to 10 days or continuously releases a therapeutically effective amount of 5-ALA into the resection cavity for at least 25 days after at least partial surgical resection of the brain tumor, wherein the 5-aminolevulinic acid is continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day.

In a preferred embodiment, the brain implant for use in the prophylaxis and/or treatment of brain cancer comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly over 3 to 10 days or continuously releases a therapeutically effective amount of 5-ALA into the resection cavity for at least 30 days after at least partial surgical resection of the brain tumor, wherein the 5-aminolevulinic acid is continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day.

Introducing 5-ALA or PEG also in the shell, alters the release kinetics likely due to pore formation in the outer shell. Introducing excipients like citric acid or fumaric acid into the core polymer lowers the pH and stabilizes 5-ALA over the extended time period since ALA is unstable under basic conditions.

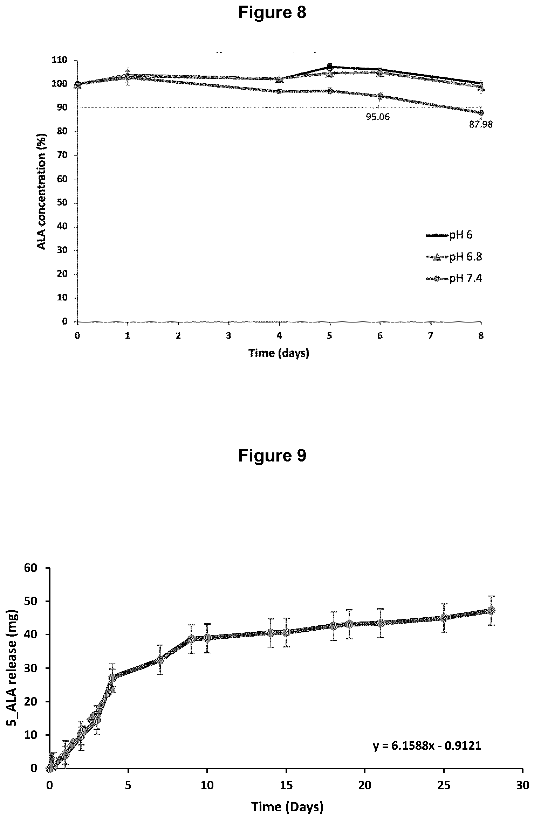

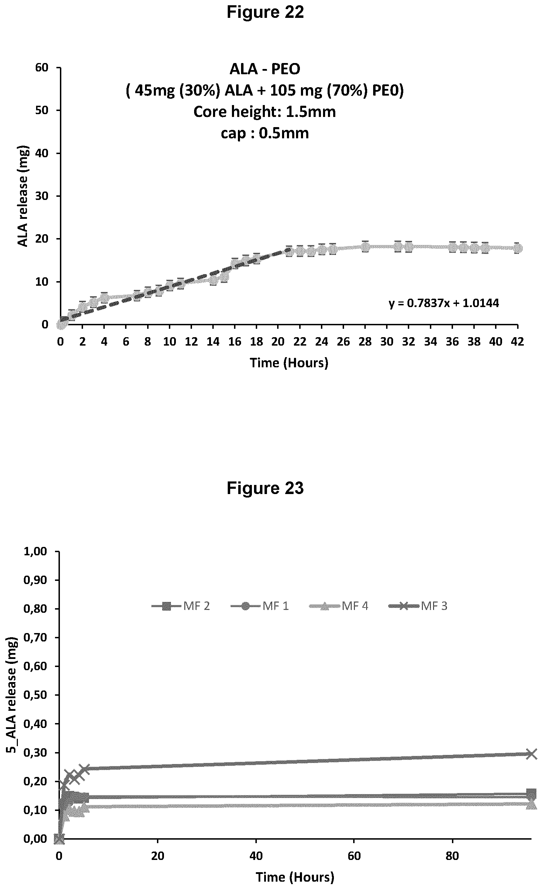

The controlling and core can be generated from any bio-compatible polymer with the brain. Particular examples include poly (lactic-co-glycolic acid), poly(lactic acid), poly(ethylene glycol), poly(ethylene oxide). Any suitable approach can be employed to control the drug release. For example, using polymer-drug mixtures at specific ratios, using polymer-polymer blends at different ratios, modifying the pH at the middle layer, using additives, e.g., surfactants/platiciser, and changing the drug's solid state. A unique aspect of the invention is that it can give a zero-order or near zero-order release without any "burst" effect for 7 days, preferably 10 days or more **(****Figures 9-22****).** This type of drug release is problematic to achieve with water-soluble small drugs as when combined directly with rate-releasing polymers, they often show either rapid release or are entrapment into the rate-controlling system **(****Figures 23-25****).**

The brain implants described herein can be placed into the brain cavity following glioblastoma resection, and when combined with systemically artesunate treatment, will bridge the 3-4-week gap from surgery to standard of care chemotherapy and radiotherapy.

Thus, a further aspect of the present invention is directed to the brain implant as described herein for use in the prophylaxis and/or treatment of brain cancer, in particular CL glioblastoma in combination with a therapeutically effective amount of an artemisinin compound selected from **1a, 1b, 1c, 1d,** and **1e** or a pharmaceutically acceptable salt thereof:

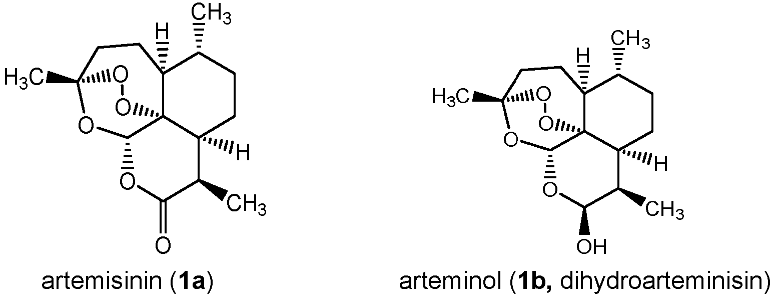

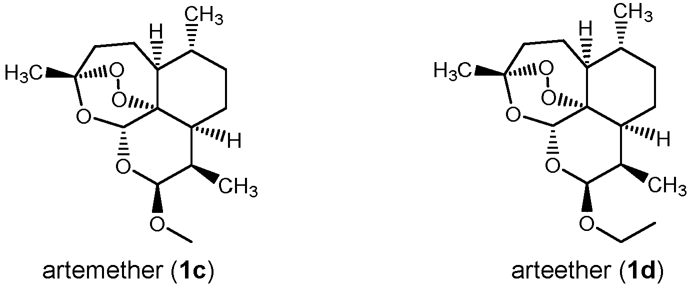

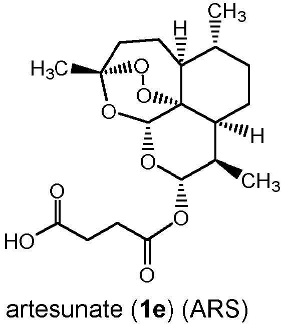

Preferably, the artemisinin compound is administered orally, intracranially, or intravenously and preferably daily during the release of the therapeutically effective amount of the 5-ALA by the inventive brain implant.

The term "intracranial" or "intracranially" refers to the injection or administration of the artemisinin compound into the brain tissue. This covers intracerebral delivery, injection or administration into the cerebrospinal fluid (e.g. via intracerebroventricular (ICV) or intrathecal injection), as well as delivery into cavities emerging from brain surgeries.

The artemisinin compound will typically be administered together with a suitable acceptable carrier selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices and could also be administered together with a carrier promoting crossing the blood brain barrier such as virus like particles, neurotropic viruses, exosomes, and nanoparticles. For example, for oral administration in the form of tablets or capsules, the artemisinin compound may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the artemisinin compound or the respective pharmaceutically active salt as active ingredient.

As used herein, and unless otherwise specified, the term "pharmaceutically acceptable salt(s) of artemisinin", includes, but is not limited to, salts of acidic or basic moieties of compounds described herein. Basic moieties are capable of forming a wide variety of salts with various inorganic and organic acids. The acids that can be used to prepare pharmaceutically acceptable acid addition salts of such basic compounds are those that form non-toxic acid addition salts, e.g. salts containing pharmacologically acceptable anions. Suitable organic acids include, but are not limited to, maleic, fumaric, benzoic, ascorbic, succinic, acetic, formic, oxalic, propionic, tartaric, salicylic, citric, gluconic, lactic, mandelic, cinnamic, oleic, tannic, aspartic, stearic, palmitic, glycolic, glutamic, gluconic, glucaronic, saccharic, isonicotinic, methanesulfonic, ethanesulfonic, p-toluenesulfonic, benzenesulfonic acids, or pamoic (e.g, 1,1'-methylene-bis-(2-hydroxy-3-naphthoate)) acids. Suitable inorganic acids include, but are not limited to, hydrochloric, hydrobromic, hydroiodic, sulfuric, phosphoric, or nitric acids. Compounds that include an amine moiety can form pharmaceutically acceptable salts with various amino acids, in addition to the acids mentioned above. Chemical moieties that are acidic in nature are capable of forming base salts with various pharmacologically acceptable cations. Examples of such salts are alkali metal or alkaline earth metal salts and, particularly, calcium, magnesium, sodium, lithium, zinc, potassium, or iron salts.

In a preferred embodiment, the brain implant may be used in the prophylaxis and/or treatment of brain cancer, in particular CL glioblastoma in combination with artesunate (1e) or a pharmaceutically acceptable salt thereof:

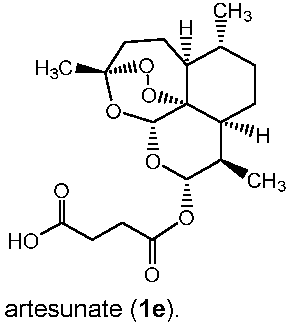

In a preferred embodiment, the brain implant for use in the prophylaxis and/or treatment of brain cancer in combination with an artemisinin compound selected from **1a, 1b, 1c, 1d,** and **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, and wherein the daily dose of the artemisinin compound is in a range of 0.5 mg artemisinin compound per kg body weight to 10 mg artemisinin compound per kg body weight.

Preferably, the daily dose of the artemisinin compound is between about 0.1 mg and 100 mg artemisinin compound per kg body weight, more preferably about 0.2 mg and 80 mg artemisinin compound per kg body weight, more preferably about 0.3 mg and 50 mg artemisinin compound per kg body weight, more preferably about 0.4 mg and 25 mg artemisinin compound per kg body weight, more preferably about 0.5 mg and 10 mg artemisinin compound per kg body weight, more preferably about 1 mg and 8 mg artemisinin compound per kg body weight, more preferably about 2 mg and 6 mg artemisinin compound per kg body weight, and most preferably about 3 mg artemisinin compound per kg body weight.

Preferably, the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the brain implant for use in the prophylaxis and/or treatment of brain cancer in combination with an artemisinin compound selected from 1a, 1b, 1c, 1d, and 1e or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, and wherein the artemisinin compound is administered daily orally, intracranially, or intravenously to the subject during the release of the therapeutically effective amount of the composition into the resection cavity by the brain implant, and wherein the total daily dose of the artemisinin compound is in a range of 100 mg to 300 mg, preferably 150 mg to 250 mg, more preferably 180 mg to 220 mg, and most preferably about 200 mg.

Preferably, the total daily dose of the artemisinin compound is 100 mg, more preferably 105 mg, more preferably 110 mg, more preferably 115 mg, more preferably 120 mg, more preferably 125 mg, more preferably 130 mg, more preferably 135 mg, more preferably 140 mg, more preferably 145 mg, more preferably 150 mg, more preferably 155 mg, more preferably 160 mg, more preferably 165 mg, more preferably 170 mg, more preferably 175 mg, more preferably 180 mg, more preferably 185 mg, more preferably 190 mg, more preferably 195 mg, and most preferably 200 mg.

Preferably, the total daily dose of the artemisinin compound is 300 mg, more preferably 295 mg, more preferably 290 mg, more preferably 285 mg, more preferably 280 mg, more preferably 275 mg, more preferably 270 mg, more preferably 265 mg, more preferably 260 mg, more preferably 255 mg, more preferably 250 mg, more preferably 245 mg, more preferably 240 mg, more preferably 235 mg, more preferably 230 mg, more preferably 225 mg, more preferably 220 mg, more preferably 215 mg, more preferably 210 mg, more preferably 205 mg, and most preferably 200 mg.

In a preferred embodiment, the brain implant for use in the prophylaxis and/or treatment of brain cancer in combination with an artemisinin compound selected from 1a, 1b, 1c, 1d, and 1e or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, and wherein the artemisinin compound is administered daily orally, intracranially or intravenously to the subject during the release of the therapeutically effective amount of the composition into the resection cavity by the brain implant, and wherein a dose of the artemisinin compound of 100 mg is administered twice daily to the subject (e.g. in the morning 100 mg artemisinin compound and in the evening 100 mg artemisinin compound).

**In** one embodiment, the therapeutically effective amount of 5-ALA is continuously released into the resection cavity over 1 to 50 days or 1 to 40 days or 1 to 30 days, more preferably over 2 to 30 days, more preferably over 3 to 30 days, more preferably over 4 to 30 days, and most preferably over 8 to 30 days together with a daily oral administration of the artemisinin compound.

**In** one embodiment, the therapeutically effective amount of 5-ALA is continuously released into the resection cavity over at least 5 days, more preferably at least 6 days, more preferably at least 7 days, more preferably at least 8 days, more preferably at least 9 days, more preferably at least 10 days, more preferably at least 11 days, more preferably at least 12 days, more preferably at least 13 days, more preferably at least 14 days, more preferably at least 15 days, more preferably at least 16 days, more preferably at least 17 days, more preferably at least 18 days, more preferably at least 19 days, more preferably at least 20 days, more preferably at least 21 days, more preferably at least 22 days, more preferably at least 23 days, more preferably at least 24 days, more preferably at least 25 days, more preferably at least 26 days, more preferably at least 27 days, more preferably at least 28 days, more preferably at least 29 days, and more preferably at least 30 days after at least partial surgical resection of the brain tumor together with a daily oral administration of the artemisinin compound.

**In** one embodiment, the therapeutically effective amount of 5-ALA is continuously released into the resection cavity between 4 days to 30 days, more preferably between 5 days and 30 days, more preferably between 10 days and 29 days, more preferably between 14 days and 28 days, and most preferably between 16 days and 27 days after at least partial surgical resection of the brain tumor together with a daily oral administration of the artemisinin compound.

**In** another embodiment, the therapeutically effective amount of 5-ALA is continuously released into the resection cavity together with a daily oral administration of the artemisinin compound between 3 days to 15 days, more preferably between 4 days and 14 days, more preferably between 5 days and 13 days, more preferably between 6 days and 12 days, and most preferably between 7 days and 11 days after at least partial surgical resection of the brain tumor.

In another embodiment, the therapeutically effective amount of 5-ALA is continuously released into the resection cavity together with a daily oral administration of the artemisinin compound between 8 days to 20 days, more preferably between 9 days and 19 days, more preferably between 10 days and 18 days, more preferably between 11 days and 17 days, and most preferably between 12 days and 16 days after at least partial surgical resection of the brain tumor.

In another embodiment, the therapeutically effective amount of 5-ALA is continuously released into the resection cavity together with a daily oral administration of the artemisinin compound between 13 days to 25 days, more preferably between 14 days and 24 days, more preferably between 15 days and 23 days, more preferably between 16 days and 22 days, and most preferably between 17 days and 21 days after at least partial surgical resection of the brain tumor.

In another embodiment, the therapeutically effective amount of 5-ALA is continuously released into the resection cavity together with a daily oral administration of the artemisinin compound between 18 days to 30 days, more preferably between 19 days and 29 days, more preferably between 20 days and 28 days, more preferably between 21 days and 27 days, and most preferably between 22 days and 26 days after at least partial surgical resection of the brain tumor.

In another embodiment, the therapeutically effective amount of 5-ALA is continuously released into the resection cavity together with a daily oral administration of the artemisinin compound between 23 days to 35 days, more preferably between 24 days and 34 days, more preferably between 25 days and 33 days, more preferably between 26 days and 32 days, and most preferably between 27 days and 30 days after at least partial surgical resection of the brain tumor.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously for at least 3 days to the subject during the release of the therapeutically effective amount of 5-ALA into the resection cavity by the brain implant, and wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously for at least 3 days to the subject during the release of the therapeutically effective amount of the composition into the resection cavity by the brain implant, wherein the artemisinin compound is daily administered orally, intracranially, or intravenously for at least 10 days to the subject during the release of the therapeutically effective amount of 5-ALA into the resection cavity by the direct intracranial drug delivery system, and wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously for at least 3 days to the subject during the release of the therapeutically effective amount of the composition into the resection cavity by the brain implant, wherein the artemisinin compound is daily administered orally, intracranially, or intravenously for at least 15 days to the subject during the release of the therapeutically effective amount of 5-ALA into the resection cavity by the brain implant, and wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously for at least 3 days to the subject during the release of the therapeutically effective amount of the composition into the resection cavity by the brain implant, wherein the artemisinin compound is daily administered orally, intracranially, or intravenously for at least 20 days to the subject during the release of the therapeutically effective amount of 5-ALA into the resection cavity by the brain implant, and wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously for at least 3 days to the subject during the release of the therapeutically effective amount of the composition into the resection cavity by the brain implant, wherein the artemisinin compound is daily administered orally, intracranially, or intravenously for at least 25 days to the subject during the release of the therapeutically effective amount of 5-ALA into the resection cavity by the brain implant, and wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously for at least 3 days to the subject during the release of the therapeutically effective amount of the composition into the resection cavity by the brain implant, wherein the artemisinin compound is daily administered orally, intracranially, or intravenously for at least 30 days to the subject during the release of the therapeutically effective amount of 5-ALA into the resection cavity by the brain implant, and wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously for at least 3 days to the subject during the release of the therapeutically effective amount of 5-ALA into the resection cavity by the brain implant, wherein the artemisinin compound is daily administered orally, intracranially, or intravenously and simultaneously with the linear and continuous release of 5-ALA into the resection cavity for at least 3 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid is continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously for at least 10 days to the subject during the release of the therapeutically effective amount of 5-ALA into the resection cavity by the brain implant, wherein the artemisinin compound is daily administered orally, intracranially, or intravenously and simultaneously with the continuous release of 5-ALA into the resection cavity for at least 3 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid is continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously for at least 3 days to the subject during the release of the therapeutically effective amount of 5-ALA into the resection cavity by the brain implant, wherein the artemisinin compound is daily administered orally, intracranially, or intravenously and simultaneously with the continuous release of 5-ALA into the resection cavity for at least 15 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid is continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously for at least 3 days to the subject during the release of the therapeutically effective amount of 5-ALA into the resection cavity by the brain implant, wherein the artemisinin compound is daily administered orally, intracranially, or intravenously and simultaneously with the continuous release of 5-ALA into the resection cavity for at least 20 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid is continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously for at least 3 days to the subject during the release of the therapeutically effective amount of 5-ALA into the resection cavity by the brain implant, wherein the artemisinin compound is daily administered orally, intracranially, or intravenously and simultaneously with the continuous release of 5-ALA into the resection cavity for at least 25 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid is continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously for at least 3 days to the subject during the release of the therapeutically effective amount of 5-ALA into the resection cavity by the brain implant, wherein the artemisinin compound is daily administered orally, intracranially, or intravenously and simultaneously with the continuous release of 5-ALA into the resection cavity for at least 30 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid is continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously and simultaneously with the continuous release of 5-ALA into the resection cavity for at least 3 days, and wherein the mass ratio of administered 5-ALA to the artemisinin compound **1e** is between 1:1 and 1:3000, preferably between 1:2 and 1:2000, more preferably between 1:3 and 1:1000, more preferably between 1:5 and 1:500, more preferably between 1:6 and 1:400, more preferably between 1:7 and 1:300, more preferably between 1:8 and 1:200, more .preferably between 1:9 and 1:150, and most preferably between 1:10 and 1:100.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously and simultaneously with the continuous release of 5-ALA into the resection cavity for at least 10 days, and wherein the mass ratio of administered 5-ALA to the artemisinin compound **1e** is between 1:1 and 1:3000, preferably between 1:2 and 1:2000, more preferably between 1:3 and 1:1000, more preferably between 1:5 and 1:500, more preferably between 1:6 and 1:400, more preferably between 1:7 and 1:300, more preferably between 1:8 and 1:200, more .preferably between 1:9 and 1:150, and most preferably between 1:10 and 1:100.

In a preferred embodiment, the composition for use in treating brain tumor in a subject, the composition comprises 5-aminolevulinic acid, wherein, in use, the composition is provided by a direct intracranial drug delivery system to a subject after at least partial surgical resection of the brain tumor in combination with an artemisinin compound **1e** or a pharmaceutically acceptable salt thereof, wherein the direct intracranial drug delivery system releases a therapeutically effective amount of the composition into a resection cavity in a continuous release manner, wherein the artemisinin compound is daily administered orally, intracranially, or intravenously and simultaneously with the continuous release of the composition into the resection cavity for at least 15 days, and wherein the mass ratio of administered 5-ALA to the artemisinin compound **1e** is between 1:1 and 1:3000, preferably between 1:2 and 1:2000, more preferably between 1:3 and 1:1000, more preferably between 1:5 and 1:500, more preferably between 1:6 and 1:400, more preferably between 1:7 and 1:300, more preferably between 1:8 and 1:200, more .preferably between 1:9 and 1:150, and most preferably between 1:10 and 1:100.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously and simultaneously with the continuous release of 5-ALA into the resection cavity for at least 20 days, and wherein the mass ratio of administered 5-ALA to the artemisinin compound **1e** is between 1:1 and 1:3000, preferably between 1:2 and 1:2000, more preferably between 1:3 and 1:1000, more preferably between 1:5 and 1:500, more preferably between 1:6 and 1:400, more preferably between 1:7 and 1:300, more preferably between 1:8 and 1:200, more .preferably between 1:9 and 1:150, and most preferably between 1:10 and 1:100.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously and simultaneously with the continuous release of 5-ALA into the resection cavity for at least 25 days, and wherein the mass ratio of administered 5-ALA to the artemisinin compound **1e** is between 1:1 and 1:3000, preferably between 1:2 and 1:2000, more preferably between 1:3 and 1:1000, more preferably between 1:5 and 1:500, more preferably between 1:6 and 1:400, more preferably between 1:7 and 1:300, more preferably between 1:8 and 1:200, more .preferably between 1:9 and 1:150, and most preferably between 1:10 and 1:100.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously and simultaneously with the continuous release of 5-ALA into the resection cavity for at least 30 days, and wherein the mass ratio of administered 5-ALA to the artemisinin compound **1e** is between 1:1 and 1:3000, preferably between 1:2 and 1:2000, more preferably between 1:3 and 1:1000, more preferably between 1:5 and 1:500, more preferably between 1:6 and 1:400, more preferably between 1:7 and 1:300, more preferably between 1:8 and 1:200, more .preferably between 1:9 and 1:150, and most preferably between 1:10 and 1:100.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously and simultaneously with the continuous release of 5-ALA into the resection cavity for at least 7 days, wherein the daily dose of the artemisinin compound is about 0.5 mg artemisinin compound per kg body weight, 0.6 mg artemisinin compound per kg body weight, 0.7 mg artemisinin compound per kg body weight, 0.8 mg artemisinin compound per kg body weight, 0.9 mg artemisinin compound per kg body weight, 1 mg artemisinin compound per kg body weight, 1.5 mg artemisinin compound per kg body weight, 2.0 mg artemisinin compound per kg body weight, 2.5 mg artemisinin compound per kg body weight, 3.0 mg artemisinin compound per kg body weight, 3.5 mg artemisinin compound per kg body weight, 4.0 mg artemisinin compound per kg body weight, 4.5 mg artemisinin compound per kg body weight, 5 mg artemisinin compound per kg body weight, 6 mg artemisinin compound per kg body weight, 7 mg artemisinin compound per kg body weight, 8 mg artemisinin compound per kg body weight, and most preferably about 9 mg, and about 10 mg artemisinin compound per kg body weight, and wherein the 5-aminolevulinic acid is continuously released at a rate of about 5 mg per day.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously and simultaneously with the continuous release of 5-ALA into the resection cavity for at least 7 days, wherein the daily dose of the artemisinin compound is about 3 mg artemisinin compound per kg body weight, and wherein the 5-aminolevulinic acid is continuously released at a rate of about 0.1 mg - 100 mg per day, more preferably at a rate of 0.15 mg - 90 mg per day, more preferably at a rate of 0.2 mg - 80 mg per day, more preferably at a rate of 0.25 mg - 70 mg per day, more preferably at a rate of 0.3 mg - 60 mg per day, more preferably at a rate of 0.35 mg - 50 mg per day, more preferably at a rate of 0.4 mg - 40 mg per day, more preferably at a rate of 0.42 mg - 30 mg per day, more preferably at a rate of 0.45 mg - 20 mg per day, more preferably at a rate of 0.5 mg - 10 mg per day, and most preferably at a rate of 2 mg - 20 mg per day.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously and simultaneously with the continuous release of 5-ALA into the resection cavity for at least 7 days, wherein the daily dose of the artemisinin compound is about 3 mg artemisinin compound per kg body weight, and wherein the 5-aminolevulinic acid is continuously released at a rate of about 1 mg per day.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously and simultaneously with the linear release of 5-ALA into the resection cavity for at least 7 days, wherein the daily dose of the artemisinin compound is about 3 mg artemisinin compound per kg body weight, and wherein the 5-aminolevulinic acid is continuously released at a rate of between 3.0 mg per day to 5.0 mg per day.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously and simultaneously with the continuous release of 5-ALA into the resection cavity for at least 7 days, wherein the daily dose of the artemisinin compound is about 3 mg artemisinin compound per kg body weight, and wherein the 5-aminolevulinic acid is continuously released at a rate of about 10 mg per day.

In a preferred embodiment, the brain implant for use in the prophylaxis and/or treatment of brain cancer in combination with artemisinin compound **1e** or a pharmaceutically acceptable salt thereof, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously and simultaneously with the continuous release of 5-ALA into the resection cavity for at least 7 days, wherein the daily dose of the artemisinin compound is about 3 mg artemisinin compound per kg body weight, and wherein the 5-aminolevulinic acid is continuously released at a rate of about 15 mg per day.

The artemisinin compound will typically be administered together with suitable acceptable carrier selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the artemisinin compound may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the artemisinin compound or the respective pharmaceutically active salt as active ingredient.

Suitable binders include starch, gelatin, natural sugars, corn sweeteners, natural and synthetic gums such as acacia, sodium alginate, carboxymethylcellulose, poly(ethylene glycol) and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the artemisinin compound may be formulated in sustained release formulation to provide the rate controlled release of the artemisinin compound to optimise the therapeutic effect(s), e.g. anti-cancer activity or activity against cancer metastases and the like. Suitable dosage forms for sustained release include tablets having layers of varying disintegration rates or controlled release, polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn, rice, and potato, and celluloses such as microcrystalline cellulose. The amount of diluent in the composition can range from about 5 to about 95 % by weight of the total composition, preferably from about 25 to about 75 weight%, and more preferably from about 30 to about 60 weight%.

The term disintegrants refers to materials added to the composition to support break apart (disintegrate) and release the artemisinin compound. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight% of the composition, more preferably from about 5 to about 10 weight%.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat, corn, rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight% of the composition, preferably from about 3 to about 10 weight %, and more preferably from about 3 to about 6 weight%.

Lubricants refer to a class of substances which are added to the dosage form to enable the tablet granules etc. after being compressed to release from the mould by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, poly(ethylene glycol)s and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight% of the final composition, preferably from about 0.5 to about 2 weight%.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight% of the composition, preferably from about 0.1 to 1 weight %.

More preferably, the brain implant as described herein can be used in the prophylaxis and/or treatment of brain cancer, in particular CL glioblastoma further in combination with at least one anti-glioblastoma drug selected from the group comprising or consisting of temozolomide, dexamethasone, lomustine, methotrexate, everolimus, carmustine, cyclophosphamide, cisplatin, carboplatin, 5-fluorouracil, triptolide, homoharringtonin, dactinomycin, doxorubicin, epirubicin, idarubicin, ribavirin, topotecan, flubendazole, itraconazole, vindesine sulfate, cerivastatin, vincristine, vinorebine, nisoldipine, deoxyadenosine, chloro-2'-deoxyadenosine, 5-nonyloxytryptamine, 2(1H)-pyrimidinone, pitavastatin, sertraline, irinotecan, clofazimine, and docetaxel; preferably temozolomide, lomustine, cisplatin, 5-fluorouracil, carmustine, and irinotecan.

Preferred, the dose of the anti-glioblastoma drug is administered orally daily is at least 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, 1.00, 1.05, 1.10, 1.15, 1.20, 1.25, 1.30, 1.35, 1.40, 1.45, 1.50, 2.00, 2.50, 3.00, 3.50, 4.00, 4.50, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5 or 15.0 mg/kg anti-glioblastoma drug per body weight per day.

Still more preferably, the brain implant as described herein can be used in the treatment of brain cancer, in particular CL glioblastoma further in combination with at least one anti-glioblastoma drug selected from the group consisting of temozolomide, lomustine, cisplatin, 5-fluorouracil.

In a preferred embodiment, the brain implant for use in the prophylaxis and/or treatment of brain cancer in combination with a therapeutically effective amount of an artemisinin compound selected from **1a, 1b, 1c, 1d,** and **1e** or a pharmaceutically acceptable salt thereof, and at least one anti-glioblastoma drug, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously to the subject during the release of the therapeutically effective amount of 5-ALA into the resection cavity by the brain implant. and wherein the therapeutically effective amount of the at least one anti-glioblastoma drug is administered systemically to the subject during the release of the therapeutically effective amount of 5-ALA into the resection cavity by the brain implant, and wherein the at least one anti-glioblastoma drug selected from the group consisting of temozolomide, lomustine, cisplatin, and 5 fluorouracil,.

In a preferred embodiment, the brain implant for use in the prophylaxis and/or treatment of brain cancer in combination with a therapeutically effective amount of an artemisinin compound selected from **1a, 1b, 1c, 1d,** and **1e** or a pharmaceutically acceptable salt thereof, and at least one anti-glioblastoma drug, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEO), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously to the subject during the release of the therapeutically effective amount of 5-ALA into the resection cavity by the brain implant. and wherein the therapeutically effective amount of the at least one anti-glioblastoma drug is administered systemically to the subject during the release of the therapeutically effective amount of 5-ALA into the resection cavity by the brain implant in a range of 0.01 to 100 mg/kg per body weight per day.

Still more preferably, the brain implant as described herein can be used in the treatment of brain cancer, in particular CL glioblastoma further in combination with a radiotherapy.

Another aspect of the present invention is directed to the brain implant as described herein for use in the prophylaxis and/or treatment of brain cancer in combination with a radiotherapy, photodynamic therapy, immunotherapy, electromagnetic field therapy, hyperthermia therapy, chemotherapy, cancer immunotherapy, surgical therapy and/or cellular therapy such as Car-T and TIL. Preferred is a combination with chemotherapy, radiotherapy, and photodynamic therapy..

In a preferred embodiment, the brain implant for use in the prophylaxis and/or treatment of brain cancer in combination with a radiotherapy, photodynamic therapy, immunotherapy, electromagnetic field therapy, hyperthermia therapy, chemotherapy, cancer immunotherapy, surgical therapy, or cellular therapy, the brain implant comprises
a) a core comprising:
   5-aminolevulinic acid and a core polymer,
   wherein the core polymer is selected from the group consisting of poly(lactic acid) (PLA), poly(ethylene oxide) (PEO), poly(ethylene glycol) (PEG), and poly(lactic-co-glycolic acid) (PLGA);
   and
b) a shell consisting of an upper membrane, a spacer, and a lower membrane, wherein the upper membrane, the spacer, and the lower membrane each comprise a shell polymer, wherein the shell polymer is poly(lactic acid) (PLA);
   characterized in that
   the shell polymer is in a range from 20 wt.% to 60 wt.% based on the total weight of the brain implant; and
   when the core polymer is poly(lactic acid) (PLA), poly(ethylene oxide) (PEG), poly(ethylene glycol) (PEG), the core contains 5-aminolevulinic acid in a range from 25 wt.% to 45 wt.%, and the core polymer in a range from 75 wt.% to 55 wt.% based on the total weight of the core; or
   when the core polymer is poly(lactic-co-glycolic acid) (PLGA), the core contains 5-aminolevulinic acid in a range from 65 wt.% to 75 wt.% and the core polymer in a range from 35 wt.% to 25 wt.% based on the total weight of the core,
   wherein the brain implant, when in use, linearly releases a therapeutically effective amount of 5-ALA into the resection cavity after at least partial surgical resection of the brain cancer, wherein the artemisinin compound is daily administered orally, intracranially, or intravenously for at least 3 days to the subject during the release of the therapeutically effective amount of the composition into the resection cavity by the brain implant,

Preferably, the brain implant for use in the prophylaxis and/or treatment of brain cancer as described herein is combined with a radiotherapy or photodynamic therapy.

### Description of Figures

**Figure 1****:**
   **A)** an image representing a brain implant: upper membrane + core + spacer + lower membrane;
   **B)** an image representing a brain implant: upper membrane + core + vessel (spacer + lower membrane)
**Figure 2****:**
   **A)** an image representing manufactured core
   **B)** an image representing manufactured upper membrane (cap) of brain implant
   **C)** an image representing manufactured vessel (spacer + lower membrane) for the device
   **D)** an image representing the assembled brain implant by sealing all the components
**Figure 3****:** Brain implant manufactuing by hot melt preparation;
**Figure 4****:** Brain implant manufactuing by coextrusion
**Figure 5: A****)** 5-ALA calibration cure in PBS; **B)** Chromatogram showing a peak around 6 minutes indicating 5-ALA
**Figure 6****:** Calibration curve for extraction method
**Figure 7****:** Active release of 5-ALA in Franz cell over 96 h
**Figure 8****:** Degradation of 0.02% 5-aminolevolinic acid in PBS (pH 7.4, 6.8, 6) at 37°C. This Figure shows that the organic acids in the core stabilize 5-ALA.
**Figure 9****:** Active release of F3 (70/G/PLGA502H/A- 203S/0.15/ND). Formulation composition described in **Table 1.** The plotted points represent the mean ± SD (n = 3)
**Figure 10****:** Active release of F4 (70/G/PLGA502H/A- 202S/0.2/ND). Formulation composition described in **Table 1.** The plotted points represent the mean ± SD (n = 3).
**Figure 11****:** Active release of F10 (70/G/PLGA502H/A- 203S/40/E-PEG400/0.2/ND). Formulation composition described in **Table 1.** The plotted points represent the mean ± SD (n = 3).
**Figure 12** Active release F11 (70/G/PLGA502H/A - 203S/0.2/10/D) Formulation composition described in **Table 1.** The plotted points represent the mean ± SD (n = 3).
**Figure 13** Active release of F14 (70/G/PLGA502H/A - 203S/0.2/6/D). Formulation composition described in **Table 1.** The plotted points represent the mean ± SD (n = 3)
**Figure 14** Active release of F15 (70/G/PLGA502H/A - 203S/0.2/8/D). Formulation composition described in **Table 1.** The plotted points represent the mean ± SD (n = 3)
**Figure 15** Active release of F19 (40/L/203H/A - 203H/0.2/ND). Formulation composition described in **Table 2.** The plotted points represent the mean ± SD (n = 3)
**Figure 16** Active release of F20 (30/L/203H/A - 203H/0.2/ND). Formulation composition described in **Table 2.** The plotted points represent the mean ± SD (n = 3)
**Figure 17** Active release of F21 (2x-30/L/203H/A - 203H/0.2/ND). Formulation composition described in **Table 3.** The plotted points represent the mean ± SD (n = 3)
**Figure 18** Active release of F22 (2x-30/L/203H/A - 203H/0.3/ND). Formulation composition described in **Table 3.** The plotted points represent the mean ± SD (n = 3)
**Figure 19** Active release of F24 (27/203H/A/10/C - 203H/0.2/ND). Formulation composition described in **Table 3.** The plotted points represent the mean ± SD (n = 3)
**Figure 20** Active release of F25 (27/203H/A/10/F - 203H/0.2/ND). Formulation composition described in **Table 3.** The plotted points represent the mean ± SD (n = 3).
**Figure 21** Active release of F27 (30/O/PEO200K/A - 202S/0.4/ND). Formulation composition described in **Table 3.** The plotted points represent the mean ± SD (n = 3)
**Figure 22****.** Active release F29 (30/O/PEO8M/A - 202S/0.2/ND). Formulation composition described in **Table 3.** The plotted points represent the mean ± SD (n = 3)
**Figure 23** Active release of dip coated core-shell systems produced via film casting. MF 1-4 are described in **Table 1.** The plotted points represent the mean ± Standard Deviation (SD) (n = 3)
**Figure 24** The release of 5-ALA from PLGA based Cores with 70% drug loading with polymers 757S (C2), and 858S (C5), also 50% drug loading with polymers 757S (C1), 502H (C3), 858S (C4). All formulations (C1-C5) are described in Table.2. The plotted points represent the mean ± SD (n = 3)
**Figure 25** The release of 5-ALA from PLA based Cores. The core has 25% 5-ALA loading with polymer 202S (C9), 30% 5-ALA loading with polymers 203H (C6), 203s (C7), 202S (C8), and 40% 5-ALA loading with 203H (C10). All the formulations from C6-C10 are described in **Table 4.** The plotted points represent the mean ± SD (n = 3)
**Figure 26** Schematic representation of a Franz cell.

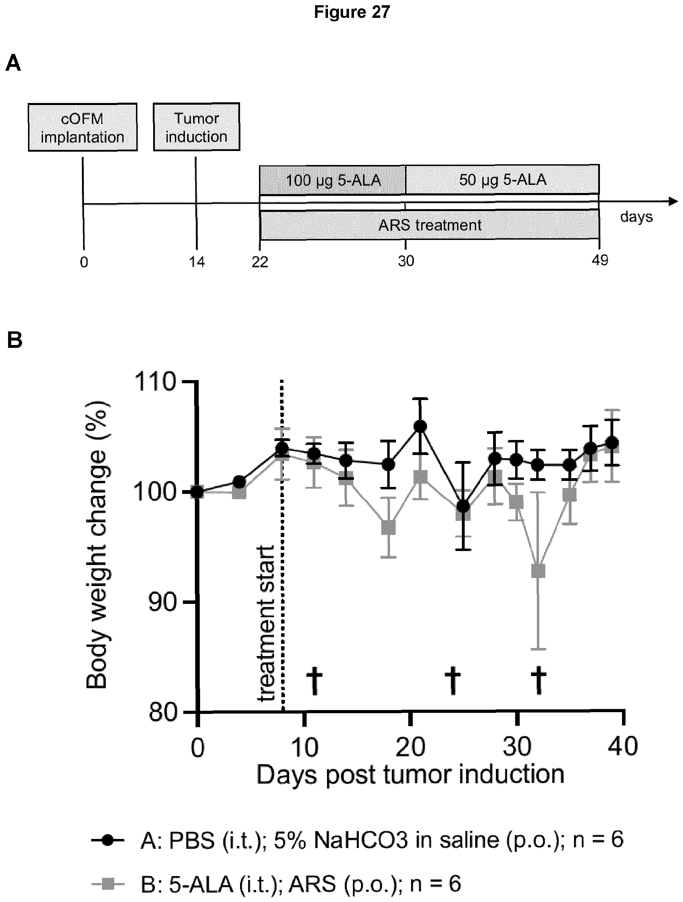

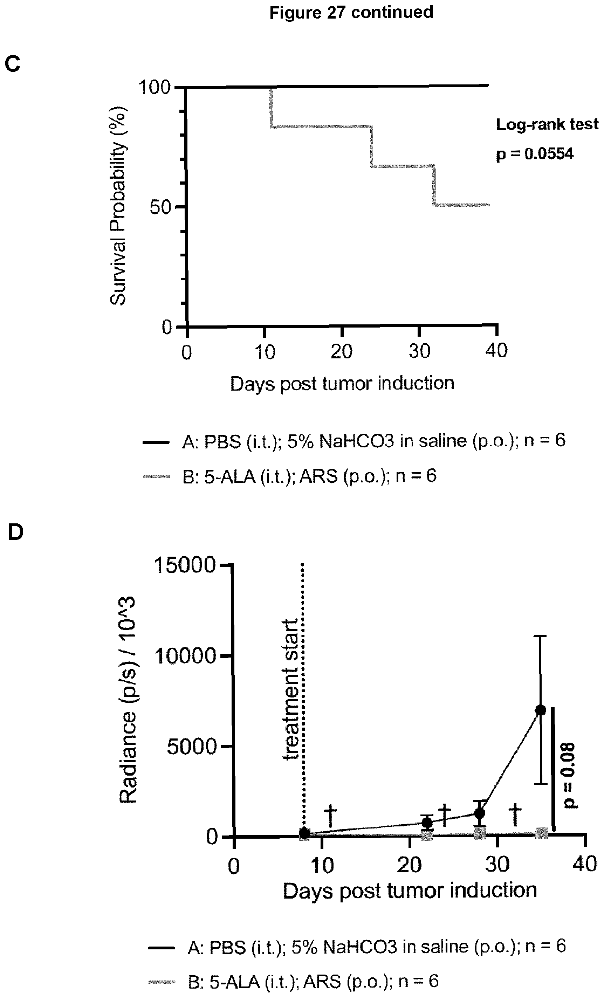

**Figure 27** shows an efficacy study using an orthothopic patient derived xenograft (PDX) model in mice. Patient-derived glioblastoma cells were implanted into the mouse brain via a cerebral open flow microperfusion (cOFM) device. **A,** Schematic representation of the study timeline. 14 days after cOFM implantation VBT529 (patient derived glioblastoma cell line) were implanted into the mouse brain via a cerebral open flow microperfusion (cOFM) device. 5-ALA bolus treatments were performed via the cOFM device and ARS was administered per oral (5x per week). **B,** % body weight changes of mice receiving solvents (Group A: PBS plus 5% NaHCO3 in saline) or 5-ALA plus ARS (Group B). †: premature termination of group B mice due to unexpected adverse effects. Data are presented as mean ± SEM; 2-way ANOVA followed by Bonferroni's multiple comparisons test. **C,** Kaplan-Meier survival curves of control and 5-ALA/ARS treated mice. Data analysis was performed using Log-rank (Mantel-Cox) test. **D**, Quantification of VBT529 brain tumor luminescence signals. †: premature termination of group B mice due to unexpected adverse effects. Data are presented as mean ± SEM; 2-way ANOVA followed by Bonferroni's multiple comparisons test.

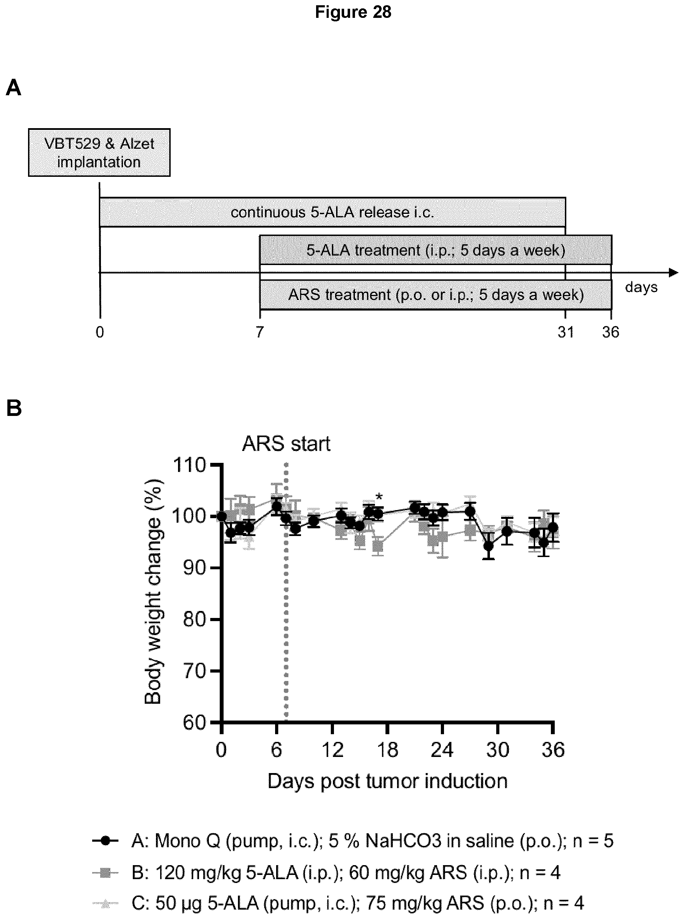

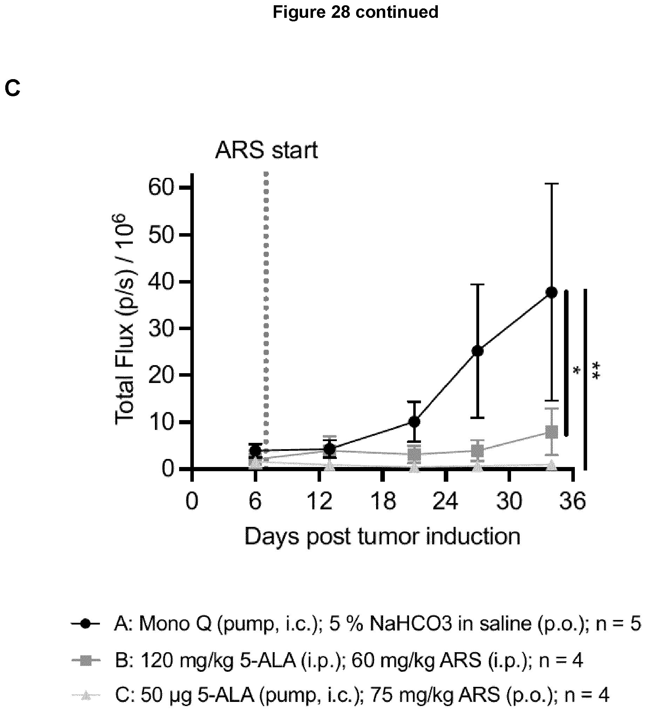

**Figure 28** shows an efficacy study using an orthothopic patient derived xenograft (PDX) model in mice comparing i.p. bolus treatments (5-ALA plus ARS) to an intracerebral slow-release 5-ALA treatment (via Alzet osmotic pump) combined with oral ARS. **A,** Schematic representation of the study timeline. 7 days after VBT529 (patient derived glioblastoma cell line) and Alzet pump implantation, ARS treatment (5 times a week) started. **B,** % body weight changes of mice receiving solvents or 5-ALA plus ARS. Data are presented as mean ± SEM; *p < 0,05 (Group A vs Group B and Group B vs. Group C); 2-way ANOVA followed by Bonferroni's multiple comparisons test. **C,** Quantification of VBT529 brain tumor luminescence signals. Data are presented as mean ± SEM; *p < 0,05; **p < 0,01; 2-way ANOVA followed by Bonferroni's multiple comparisons test.

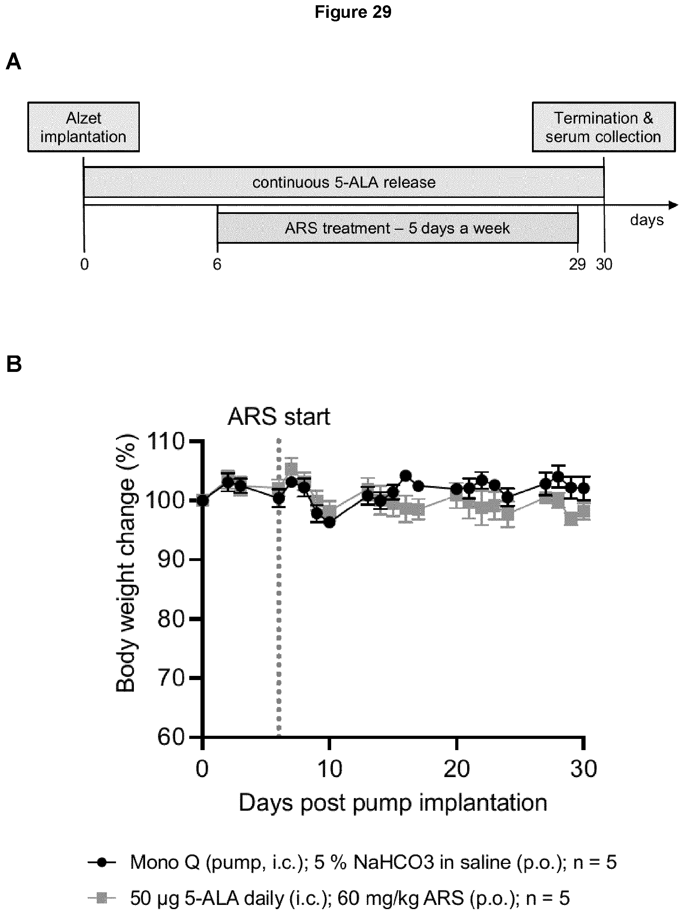

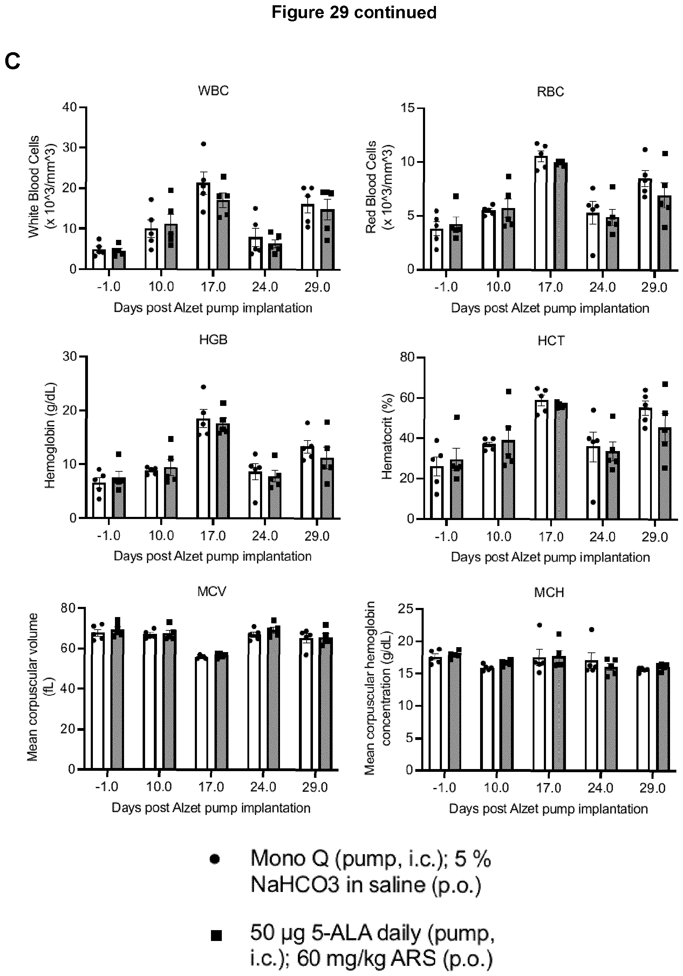

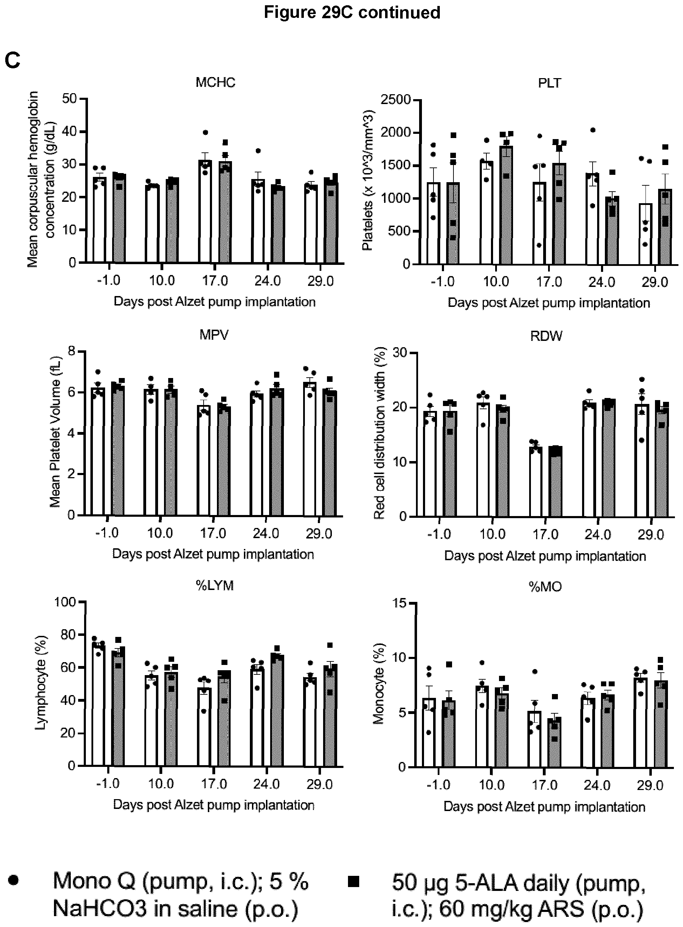

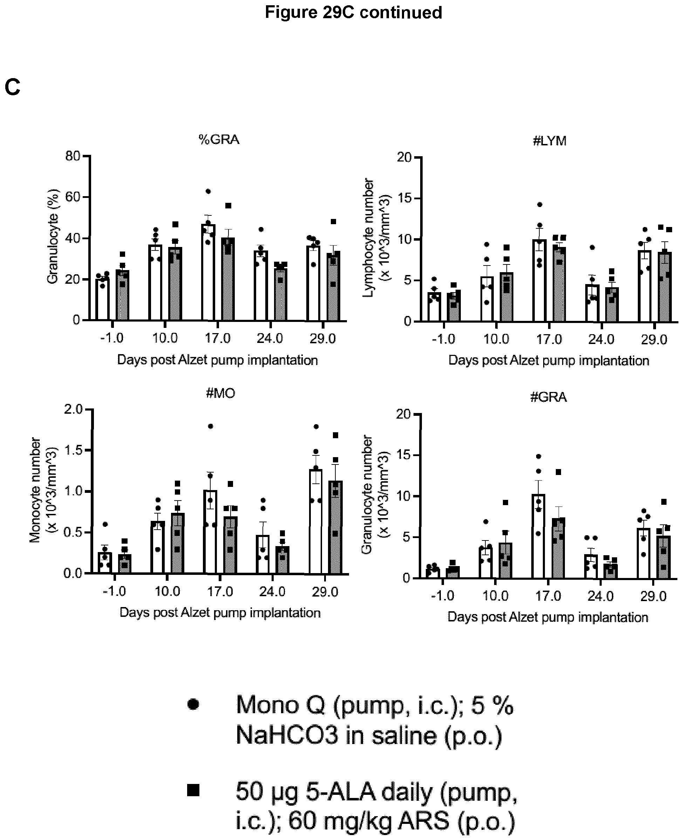

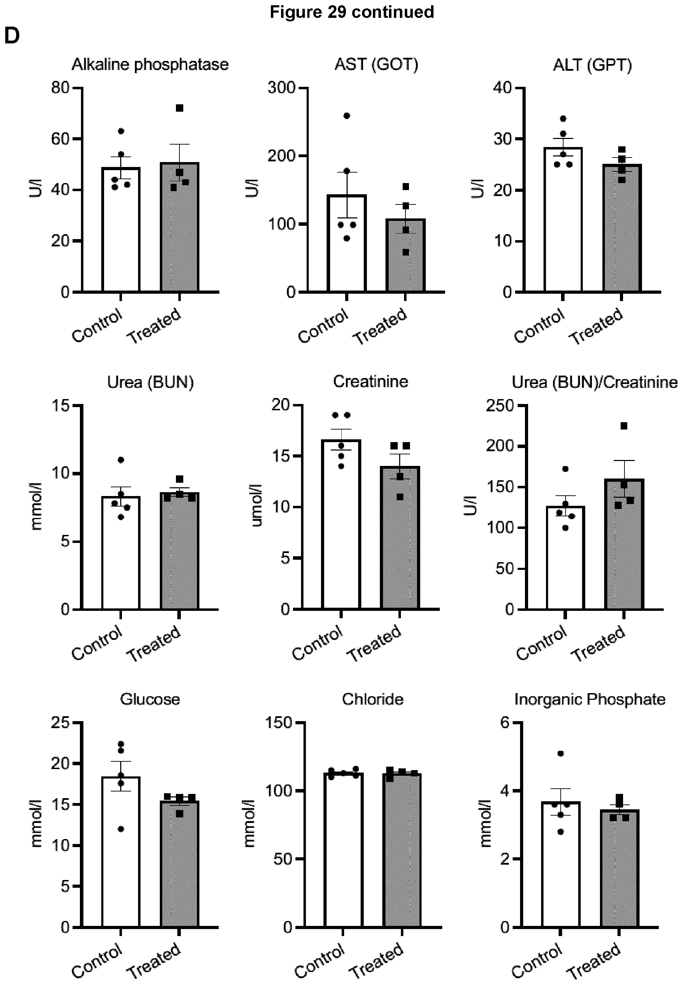

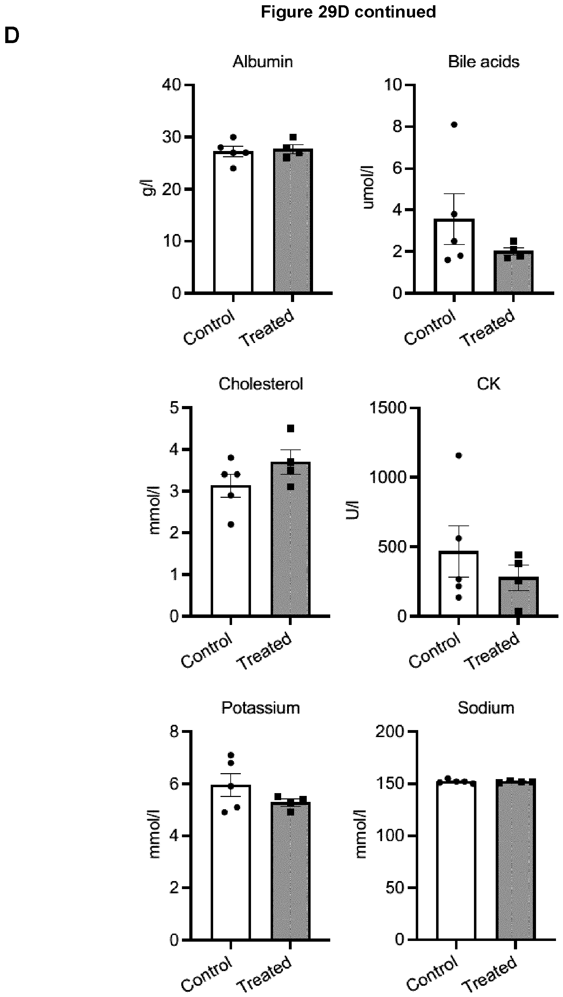

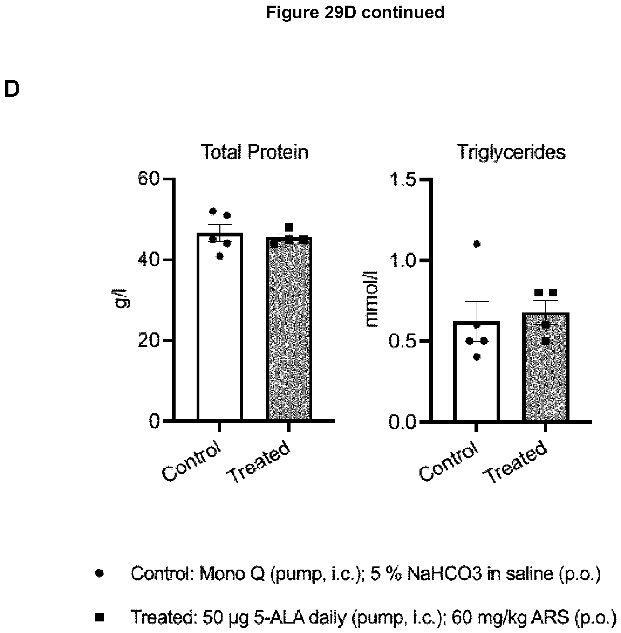

**Figure 29** shows a toxicity study in mice utilizing Alzet osmotic pumps connected to a brain infusion cannula to deliver different doses of 5-ALA directly into the brain via a constant slow-release mechanism. ARS was administered per oral. **A,** Schematic representation of the study timeline. 6 days after Alzet pump implantation, ARS treatment (5 times a week) started. **B,** % body weight changes of mice receiving solvents or 5-ALA plus ARS. Data are presented as mean ± SEM; 2-way ANOVA followed by Bonferroni's multiple comparisons test. **C,** Blood cell counts were performed 1 day before and 10-, 17-, 24- and 29-days post Alzet pump implantation. Each graph represents a distinct blood cell type/parameter as indicated. Data are presented as mean ± SEM; Student's unpaired t-test with Bonferroni's multiple comparisons test. **D,** Mouse sera were harvested 30 days post Alzet pump implantation (23 days of ARS treatment) and an expanded mouse serum tox analysis was performed. Each graph represents a distinct serum parameter as indicated. Data are presented as mean ± SEM; Student's unpaired t-test with Bonferroni's multiple comparisons test.

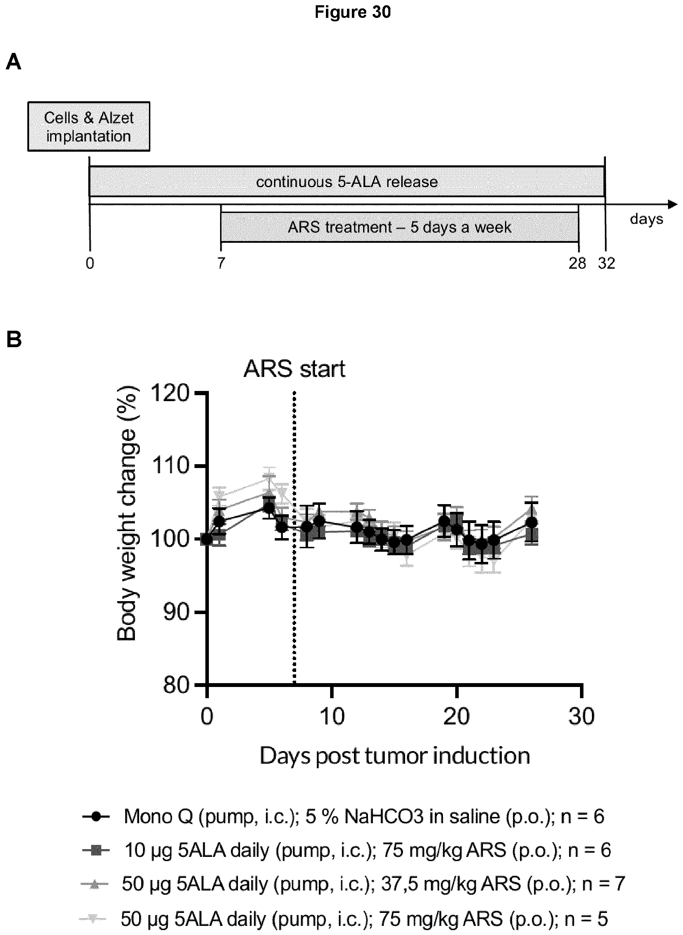

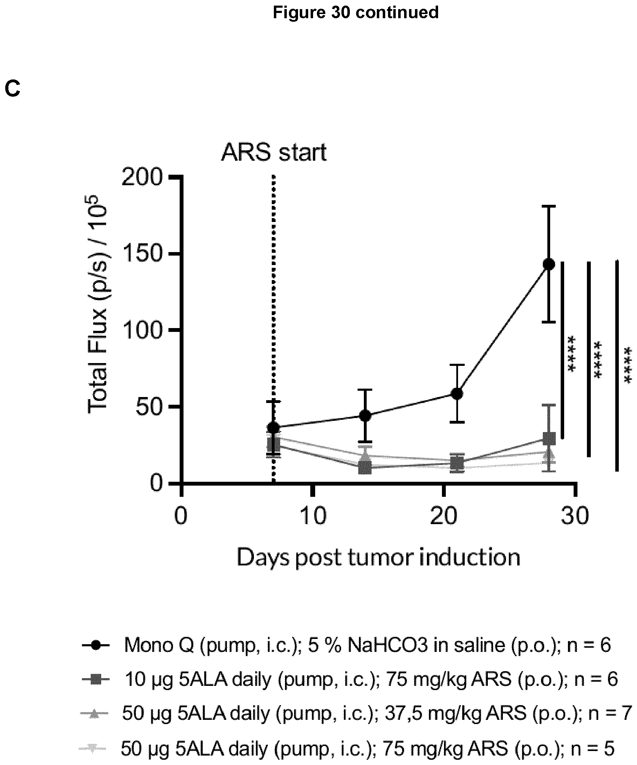

**Figure 30** shows an efficacy study using an orthothopic patient derived xenograft (PDX) model in mice using different doses of intracerebral slow-release 5-ALA plus oral ARS treatment. **A,** Schematic representation of the study timeline. 7 days after VBT529 (patient derived glioblastoma cell line) and Alzet pump implantation, ARS treatment (5 times a week) started. **B,** % body weight changes of mice receiving solvents or different doses of 5-ALA plus ARS. Data are presented as mean ± SEM; 2-way ANOVA followed by Bonferroni's multiple comparisons test. **C,** Quantification of VBT529 brain tumor luminescence signals. Data are presented as mean ± SEM; ****p < 0,0001; 2-way ANOVA followed by Bonferroni's multiple comparisons test.

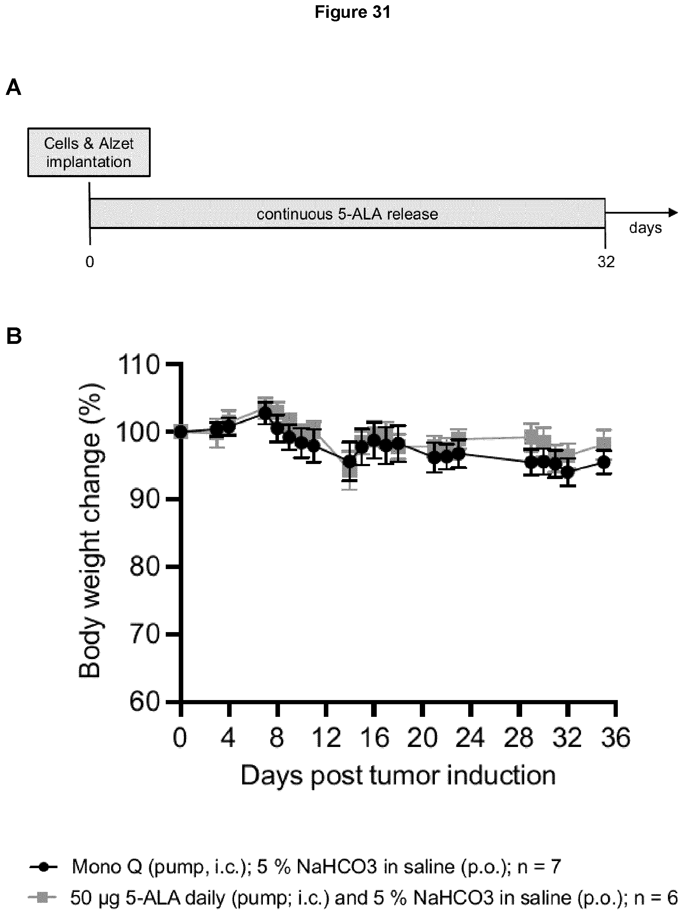

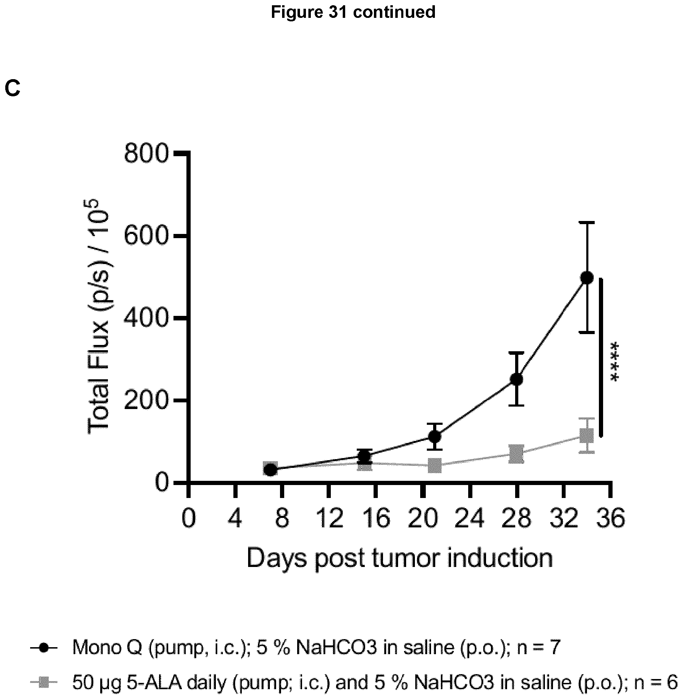

**Figure 31** shows an efficacy study using an orthothopic patient derived xenograft (PDX) model in mice utilizing Alzet osmotic pumps to deliver 5-ALA directly into the brain via a constant slow-release mechanism. **A,** Schematic representation of the study timeline. **B,** % body weight changes of mice receiving slow-release 5-ALA treatment. Data are presented as mean ± SEM; 2-way ANOVA followed by Bonferroni's multiple comparisons test. **C,** Quantification of VBT529 brain tumor luminescence signals. Data are presented as mean ± SEM; ****p < 0,0001; 2-way ANOVA followed by Bonferroni's multiple comparisons test.
**Figure 32** shows an efficacy study using an orthothopic patient derived xenograft (PDX) model in mice utilizing Alzet osmotic pumps to deliver 5-ALA directly into the brain via a constant slow-release mechanism. **A,** Schematic representation of the study timeline. 7 days after VBT529 (patient derived glioblastoma cell line) and Alzet pump implantation, ARS treatment (5 times a week) started. **B** and **C,** Quantification of VBT529 brain tumor luminescence signals. Data are presented as mean ± SEM; **p < 0,01; 2-way ANOVA followed by Bonferroni's multiple comparisons test.

The following examples are included to demonstrate preferred embodiments of the invention. It should be appreciated by those skilled in the art that the techniques disclosed in the examples, which follow represent techniques discovered by the inventor to function well in the practice of the invention, and thus can be considered to constitute preferred modes for its practice. However, those skilled in the art should, in light of the present disclosure, appreciate that many changes can be made in the specific embodiments, which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Experiments

### Example 1: Preperation methods of brain implants

### Device Fabrication Protocol

### 1) Core preparation

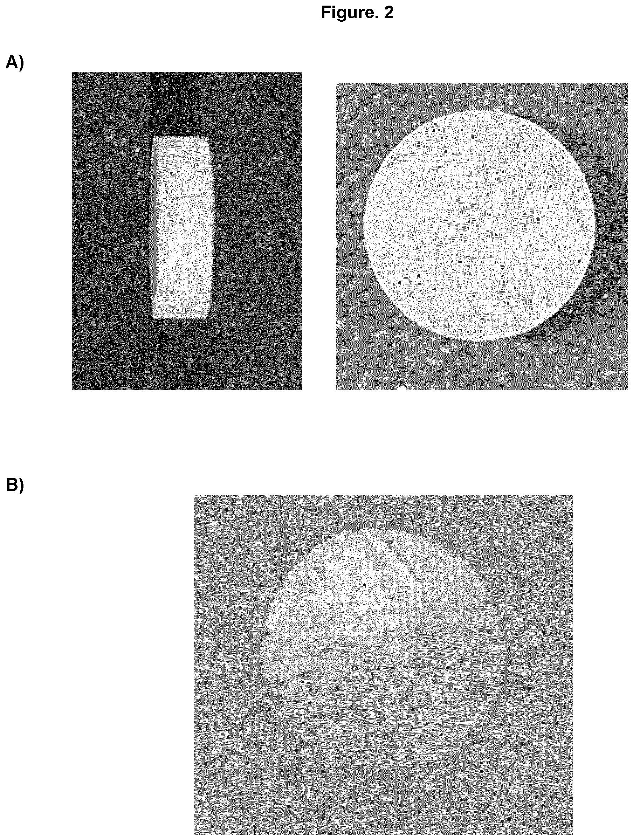

### (See Fig. 2A)

1. Weigh the polymer and 5-aminolevulinic acid (5-ALA) using an analytical balance, according to the specified formula.
2. Mix the polymer and 5-ALA using a mortar and pestle to ensure a homogenous mixture.
3. Transfer the mixture into the PTFE-lined Vapour Control Module (VCM) **D10** mm chamber.
4. Utilize a brush as necessary to minimize powder loss during the transfer process.
5. Insert the base component into the bottom of the main body of the VCM.
6. Position the lid onto the main body from the top and apply firm pressure to secure it.
7. Set the heating plate temperature to 120°C.
8. Place the **D10** mm VCM tool on the heating plate for a duration of 10 minutes.
9. Subsequently, transfer the VCM tool to the cooling plate for an additional 5 minutes.
10. Once fully cooled, carefully peel off the foils from the sample to avoid any damage.
11. Measure both the weight and thickness of the sample accurately.

### 2) Upper membrane Preparation

### (See Fig. 2B)

1. Weigh the polymer using an analytical balance, according to the specified formula.
2. Select the appropriate silicone module to put in the **D20** mm Vapour Control Module (VCM) chamber.
3. Transfer the materials into the VCM chamber with care.
4. Insert the base component into the bottom of the main body of the VCM.
5. Securely position the lid onto the main body from the top, applying firm pressure to ensure a tight seal.
6. Set the temperature of the heating plate to 130°C.
7. Place the **D20** mm VCM tool on the heating plate for a duration of 5 minutes.
8. Subsequently, transfer the VCM tool to the cooling plate for an additional 5 minutes.
9. Once fully cooled, carefully peel off the foils from the sample to avoid any damage.
10. Measure both the weight and thickness of the sample accurately.

### 3) Vessel (spacer and lower membrane) Preparation

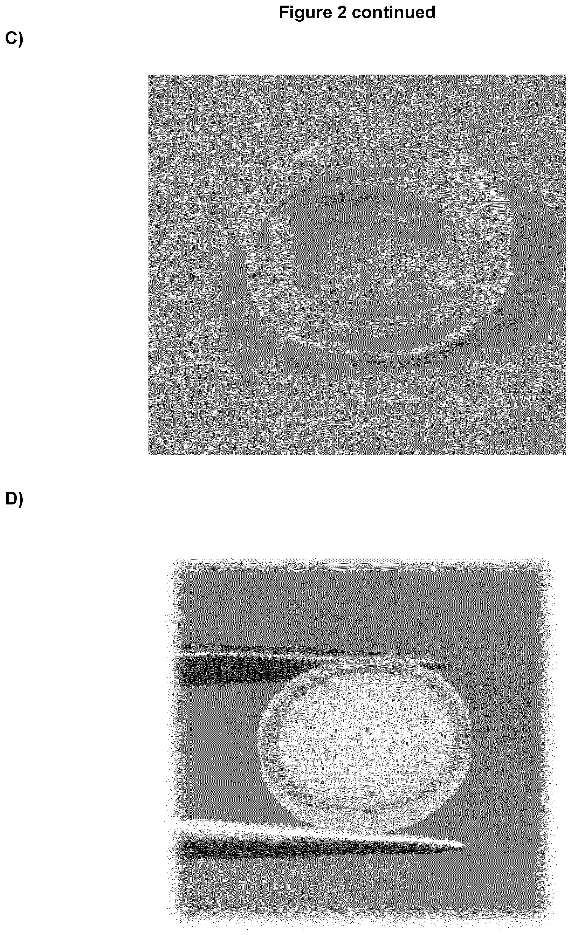

### (See Fig. 2C)

1. Weigh the polymer using an analytical balance, according to the specified formula.
2. Select the appropriate silicone module to put in the D20 mm VCM chamber.
3. Transfer the materials into the VCM chamber with care.
4. Insert the base component into the bottom of the main body of the VCM.
5. Securely position the lid onto the main body from the top, applying firm pressure to ensure a tight seal.
6. Set the temperature of the heating plate to 130°C.
7. Place the **D20** mm VCM tool on the heating plate for a duration of 5 minutes.
8. Subsequently, transfer the VCM tool to the cooling plate for an additional 5 minutes.
9. Once fully cooled, carefully peel off the foils from the sample to avoid any damage.
10. Measure both the weight and thickness of the sample accurately.

### 4) Sealing

### (See Fig. 2D)

1. Place the 0.2 mm silicone upper membrane module into the cylinder first.
2. Position the prepared upper membrane onto the silicone module.
3. Insert the vessel and core into the other 1.5 cm module.
4. Invert the module containing the vessel and core, then gently place it into the cylinder, positioning it on top of the 0.2 mm silicone module with the upper membrane.
5. Insert the base component into the bottom of the main body of the VCM.
6. Securely position the lid onto the main body from the top, applying firm pressure to ensure a tight seal.
7. Set the temperature of the heating plate to 130°C.
8. Set the heating plate temperature to 130°C.
9. Place the **D20** mm VCM tool on the heating plate for a duration of **15** minutes.
10. Subsequently, transfer the VCM tool to the cooling plate for an additional 5 minutes.
11. Once fully cooled, carefully peel off the foils from the sample to avoid any damage.
12. Measure the weight and thickness of the sample.

### Poly(D,L-lactide) RESOMER^{®} R standard polymers

| Polymer name | Inherent viscosity (dl/g) | Composition | Degradation timeframe* | End group |
|---|---|---|---|---|
| RESOMER^{®} R 202 H | 0.16 - 0.24 | Poly(D,L-lactide) | < 6 months | Acid |
| RESOMER^{®} R 203 H | 0.25 - 0.35 | Poly(D,L-lactide) | < 6 months | Acid |
| RESOMER^{®} R 202 S | 0.16 - 0.24 | Poly(D,L-lactide) | < 9 months | Ester |
| RESOMER^{®} R 203 S | 0.25 - 0.35 | Poly(D,L-lactide) | < 12 months | Ester |

| | | | | |
|---|---|---|---|---|
| **Approximate degradation times are intended to guide polymer selection. Actual resorption times are dependent upon the process and application and must be empirically determined.* | | | | |

### Poly(D,L-lactide-co-glycolide) RESOMER^{®} RG standard polymers

| Polymer name | Inherent viscosity (dl/g) | Composition | Degradation timeframe* | End group |
|---|---|---|---|---|
| RESOMER^{®} RG 501 H | 0.08 - 0.16 | Poly(D,L-lactide-co-glycolide) 50:50 | < 3 months | Acid |
| RESOMER^{®} RG 502 | 0.16 - 0.24 | Poly(D,L-lactide-co-glycolide) 50:50 | < 3 months | Ester |
| RESOMER^{®} RG 502 H | 0.16 - 0.24 | Poly(D,L-lactide-co-glycolide) 50:50 | < 3 months | Acid |
| RESOMER^{®} RG 503 | 0.32 - 0.44 | Poly(D,L-lactide-co-glycolide) 50:50 | < 3 months | Ester |
| RESOMER^{®} RG 503 H | 0.32 - 0.44 | Poly(D,L-lactide-co-glycolide) 50:50 | < 3 months | Acid |
| RESOMER^{®} RG 757 S | 0.9 - 1.3 | Poly(D,L-lactide-co-glycolide) 75:25 | < 6 months | Ester |
| RESOMER^{®} RG 858 S | 1.3 - 1.7 | Poly(D,L-lactide-co-glycolide) 85:15 | | Ester |

### Brain Implants of the Invention

### Preparation Methods

### PLGA Core-Shell Systems Via Vacuum Compression Moulding

The implant core comprised a PLGA polymer matrix containing 5-ALA as per Table **2.** The implant core was composed of a PLGA polymer matrix containing 5-ALA. The two components were weighed and placed directly into a mortar and pestle and were ground together until a homogenous mixture was formed. Then, the mixture is transferred into a PTFE-lined D10 mm Vapour Control Module (VCM) (Melt Prep VCM, Austria) chamber, securing the lid and heating at 120°C for 10 minutes. The VCM tool is then cooled for 5 minutes, and the foils are carefully removed to measure the sample's weight and thickness. The shells were manufactured with PLA (203S, 20S and 203H polymers). For cap and vessel preparation, the polymer is weighed, transferred into the D20 mm VCM chamber, which has a silicone cap module with different thickness (0.15/0.2/0.3/0.4/0.45/0.5/0.6) and processed by heating at 120°C for 5 minutes, followed by cooling for another 5 minutes, and the foils are carefully peeled off to measure weight and thickness. For sealing, a 0.2 mm silicone cap module is placed into the cylinder, the prepared cap is positioned, and the vessel and core are inserted into a 1.5 cm module. The assembly is carefully inverted into the cylinder, sealed with the VCM base and lid, and heated at 130°C for 15 minutes, followed by cooling for 5 minutes. After peeling off the foils, the final device weight and thickness are measured. The cores and the shells were assembled to fulfil two types of design prototypes:
**Prototype 1**: The prototype one formulation features a core composed of a polymer matrix and a significant dose of 5-ALA, encased in a cap and supported by a structural vessel, both made from PLA polymer.

**Prototype 2:** The prototype two formulation features a core composed of a polymer matrix and a significant dose of 5-ALA, encased in a cap and supported by a structural vessel, both made from PLA polymer and 5-ALA.

**Table 1 is the list of the core-shell implants developed with PLGA polymer.**

| **F#** | **F name** | **Description** | |
|---|---|---|---|
| **Prototype 1** | | | |
| **F3** | 70/G/PLGA502H/A-203S/0.15/ND | **Core:** | |
| | | | **• Polymer (30%):** The core contains **Poly-lactic glycolic acid (PLGA) 502 H,** with a total of **36 mg.** |
| | | | **• Drug (70%):** The core is primarily composed of **5-ALA,** at **84 mg.** |
| | | **Cap (Upper membrane) (0.15mm thick):** | |
| | | | **•** The cap is made with 10 mg of **203 S Poly (D, L-lactide).** The cap has a thickness of **0.15 mm** and contains no drugs or excipients. |
| | | **Vessel (spacer + lower membrane):** | |
| | | | **•** The vessel is also composed of 100 mg **203 S Poly (D, L-lactide),** serving as the structural component of the system. It does not contain any active drugs or excipients. |
| **F4** | 70/G/PLGA502H/A-202S/0.2/ND | **Core:** | |
| | | | **• Polymer (30%):** The core contains **Poly-lactic glycolic acid (PLGA) 502 H,** with a total amount of **36** |
| | | | **mg. • Drug (70%):** The active ingredient in the core is **5-ALA,** with a concentration of **84 mg.** |
| | | **Cap (Upper membrane) (0.2mm thick):** | |
| | | | **•** The cap is composed of 10 mg **202 S Poly (D, L-lactide).**The cap thickness of **0.2 mm.** No drugs or excipients are present in the cap. |
| | | **Vessel (spacer + lower membrane):** | |
| | | | **•** The vessel, also made from 100 mg **202 S Poly (D, L-lactide),** provides structural support for the system. It does not contain any active ingredients or excipients. |
| **F10** | 70/G/PLGA502H/A-203S/40/E-PEG400/0.2/ND | **Core:** | |
| | | | **• Polymer (30%):** The core is composed of **Poly-lactic glycolic acid (PLGA) 502 H,** with a total weight of **36 mg.** |
| | | | **• Drug (70%):** The core contains **5-ALA (5-Aminolevulinic acid)** at **84 mg.** |
| | | **Cap (Upper membrane) (0.2mm thick):** | |
| | | | **•** The cap is made from 6 mg of **203 S Poly (D, L-lactide),** The thickness of the cap is **0.2 mm** and includes **PEG 400** at 4 **mg** as an excipient. |
| | | **Vessel (spacer + lower membrane):** | |
| | | | **•** The vessel is also constructed from 60 mg **203 S Poly (D, L-lactide),** and 40 mg of **PEG 400** providing structural support for the system without containing any active ingredients or additional excipients. |

| **Prototype 2** | | | |
|---|---|---|---|
| **F11** | 70/G/PLGA502H/A - 203S/0.2/10/D | **Core:** | |
| | | | **• Polymer (30%):** The core consists of **Poly-lactic glycolic acid (PLGA) 502 H,** weighing **36 mg.** |
| | | | **• Drug (70%):** The core includes **5-ALA** at **84 mg,** providing a substantial therapeutic dose that enhances the formulation's efficacy. |
| | | **Cap (Upper membrane) (0.2mm thick):** | |
| | | | **•** The cap is made from 9 mg **203 S Poly (D, L-lactide),** with a thickness of **0.2 mm.** It contains an additional 5-ALA dose of **1mg.** |
| | | **Vessel (spacer + lower membrane):** | |
| | | | **•** The vessel is also constructed from 90 mg **203 S Poly (D, L-lactide)** and includes **10 mg** of **5-ALA (5-Aminolevulinic acid).** |
| **F14** | 70/G/PLGA502H/A - 203S/0.2/6/D | **Core:** | |
| | | | **• Polymer (30%):** The core consists of **Poly-lactic glycolic acid (PLGA) 502 H,** with a total weight of **36 mg.** |
| | | | **• Drug (70%):** The core contains **5-ALA (5-Aminolevulinic acid)** at **84 mg.** |
| | | **Cap (Upper membrane) (0.2mm thick):** | |
| | | | **•** The cap is composed of 9.4 mg **203 S Poly (D, L-lactide)** and has a thickness of **0.2 mm.** It contains an additional **5-ALA** dose of **0.6 mg.** |
| | | **Vessel (spacer + lower membrane):** | |
| | | | **•** The vessel is also made from 94 mg **203 S Poly (D, L-lactide)** and contains **6 mg** of **5-ALA (5-Aminolevulinic acid).** |
| **F15** | 70/G/PLGA502H/A - 203S/0.2/8/D | **Core:** | |
| | | | **• Polymer (30%):** The core consists of **Poly-lactic glycolic acid (PLGA) 502** |
| | | | **H,** with a total weight of **36 mg.** |
| | | | **• Drug (70%):** The core contains **5-ALA (5-Aminolevulinic acid)** at **84 mg.** |
| | | **Cap (Upper membrane) (0.2mm thick):** | |
| | | | **•** The cap is made from 9.2 mg 203 **S Poly (D, L-lactide)** and has a thickness of **0.2 mm.** It includes an additional **5-ALA** dose of **0.8 mg.** |
| | | **Vessel (spacer + lower membrane):** | |
| | | | **•** The vessel is also constructed from 92 mg **203 S Poly (D, L-lactide)** and contains **8 mg** of **5-ALA (5-Aminolevulinic acid).** |

### PLA Core-Shell Systems Via Vacuum Compression Moulding

The implant core was composed of a PLA polymer matrix containing 5-ALA as per Table **2.** The two components were weighed and placed directly into a mortar and pestle and were ground together until a homogenous mixture was formed. Following that, mixture is transferred into a PTFE-lined D10 mm Vapour Control Module (VCM) (Melt Prep VCM, Austria) chamber, securing the lid, and heating at 120°C for 10 minutes. The VCM tool is then cooled for 5 minutes, and the foils are carefully removed to measure the sample's weight and thickness. The shells were manufactured with PLA (203S, 20S and 203H polymers). For cap and vessel preparation, the polymer is weighed, transferred into the D20 mm VCM chamber, which has a silicone cap module with different thickness (0.15/0.2/0.3/0.4/0.45/0.5/0.6) and processed by heating at 120°C for 5 minutes, followed by cooling for another 5 minutes, and the foils are carefully peeled off to measure weight and thickness. For sealing, a 0.2 mm silicone cap module is placed into the cylinder, the prepared cap is positioned, and the vessel and core are inserted into a 1.5 cm module. The assembly is carefully inverted into the cylinder, sealed with the VCM base and lid, and heated at 130°C for 15 minutes, followed by cooling for 5 minutes. After peeling off the foils, the final device weight and thickness are measured. The cores and the shells were assembled to fulfil two types of design prototypes:
**Prototype 1:** The prototype one formulation features a core composed of a polymer matrix and a significant dose of 5-ALA, encased in a cap and supported by a structural vessel, both made from PLA polymer.

**Prototype 2:** The prototype two formulation features a core composed of a polymer matrix and a significant dose of 5-ALA, encased in a cap and supported by a structural vessel, both made from PLA polymer and a small drug concentration.

**Table 2** is the list of the core-shell implants developed with PLA polymer.

**Table 2 Formulations developed with PLA polymer**

| **F#** | **F name** | **Description** | |
|---|---|---|---|
| **Prototype 1** | | | |
| **F19** | 40/L/203H/A - 203H/0.2/ND | **Core:** | |
| | | | **• Polymer (60%):** The core is made up of **203 H Poly-lactic acid (PLA),** with a quantity of **90 mg.** This biodegradable polymer provides the structural framework and controls the drug release rate. |
| | | | **• Drug (40%):** Drug incorporated in the core is **5-ALA,** present at 60 **mg.** |
| | | **Cap (Upper membrane) (0.2mm thick):** | |
| | | | **•** The cap is also made of 10 mg **203 H Poly-lactic acid (PLA),** but it does not contain any drugs or excipients. Its primary function is to regulate the release rate of the drug by providing an additional diffusion barrier. |
| | | **Vessel (spacer + lower membrane):** | |
| | | | **•** The vessel housing the system is also composed of 100 mg **203 H Poly-lactic acid (PLA),** with no drugs or excipients present. |
| **F20** | 30/L/203H/A - 203H/0.2/ND | **Core:** | |
| | | | **• Polymer (70%):** The core is made of **203 H Poly-lactic acid (PLA),** present at **105 mg.** This biodegradable polymer provides structural integrity and enables continuous release of the drug over time. |
| | | | **• Drug (30%):** The active drug in the core is **5-ALA (5-Aminolevulinic acid),** with a dosage of **45 mg.** 5-ALA is used for therapeutic applications and is incorporated into the core for gradual release. |
| | | | **• Excipients:** No additional excipients are included in the core composition. |
| | | **Cap (Upper membrane) (0.2mm thick):** | |
| | | | **•** The cap is composed of 10 mg **203 H Poly-lactic acid (PLA),** serving as a diffusion barrier for the drug release. It does not contain any drugs or excipients. |
| | | **Vessel (spacer + lower membrane):** | |
| | | | **•** The vessel enclosing the system is also made from 100 mg **203 H Poly-lactic acid (PLA),** with no added drugs or excipients. |
| **F21** | 2x-30/L/203H/A - 203H/0.2/ND | **Core:** | |
| | | | **• Polymer (70%):** The core is made of **203 H Poly-lactic acid (PLA),** present at **210 mg.** This biodegradable polymer provides structural integrity and enables continuousrelease of the drug over time. |
| | | | **• Drug (30%):** The active drug in the core is **5-ALA (5-Aminolevulinic acid),** with a dosage of **90 mg.** 5-ALA is used for therapeutic applications and is incorporated into the core for gradual release. |
| | | | **• Excipients:** No additional excipients are included in the core composition. |
| | | **Cap (Upper membrane) (0.2mm thick):** | |
| | | | **•** The cap is composed of 10 mg **203 H Poly-lactic acid (PLA),** serving as a diffusion barrier for the drug release. It does not contain any drugs or excipients. |
| | | **Vessel (spacer + lower membrane):** | |
| | | | **•** The vessel enclosing the system is also made from 130 mg **203 H Poly-lactic acid (PLA),** with no added drugs or excipients. |
| **F22** | 2x-30/L/203H/A - 203H/0.3/ND | **Core:** | |
| | | | **• Polymer (70%):** The core is made of **203 H Poly-lactic acid (PLA),** present at **210 mg.** This biodegradable polymer provides structural integrity and enables continuousrelease of the drug over time. |
| | | | **• Drug (30%):** The active drug in the core is **5-ALA (5-Aminolevulinic acid),** with a dosage of **90 mg.** 5-ALA is used for therapeutic applications and is incorporated into the core for gradual release. |
| | | | **• Excipients:** No additional excipients are included in the core composition. |
| | | **Cap (Upper membrane) (0.3mm thick):** | |
| | | | **•** The cap is composed of 30 mg **203 H Poly-lactic acid (PLA),** serving as a diffusion barrier for the drug release. It does not contain any drugs or excipients. |
| | | **Vessel (spacer + lower membrane):** | |
| | | | **•** The vessel enclosing the system is also made from 130 mg **203 H Poly-lactic acid (PLA),** with no added drugs or excipients. |

### Prototype 1: Poly(ethylene oxide) core-based systems

To try and increase the amount of 5-ALA released from the core poly(ethylene oxide) (PEO) core-based systems were developed as alternatives to PLA and PLGA cores, with varying cap thicknesses, and were assessed for drug release performance through dissolution testing **(Table 3).**

**Table 3 Poly(ethylene oxide) core-based systems**

| | | | |
|---|---|---|---|
| **F26** | 30/O/PEO200K/ A - 202S/0.2/ND | **Core:** | |
| | | | **• Polymer (70%):** The core is made of **Poly-ethylene oxide (PEO)** with a molecular weight of **200,000,** in a quantity of **105 mg.** |
| | | | **• Drug (30%):** The active drug in the core is **5-ALA,** present at **45 mg.** |
| | | **Cap (Upper membrane) (0.2/0.4mm thick):** | |
| | | | **•** The cap is composed of 10 mg **202 S Poly-lactic acid (PLA).** The thickness of the cap is **0.2 mm.** The cap does not contain any drugs or excipients. |
| | | **Vessel (spacer + lower membrane):** | |
| | | The vessel is made from 100 mg **202 S Poly-lactic acid (PLA),** providing structural integrity to the system without any added drugs or excipients. | |
| **F27** | 30/O/PEO200K/ A - 202S/0.4/ND | | **• Polymer (70%):** The core is made of **Poly-ethylene oxide (PEO)** with a molecular weight of **200,000,** in a quantity of **105 mg.** |
| | | | **• Drug (30%):** The active drug in the core is **5-ALA,** present at **45 mg.** |
| | | **Cap (Upper membrane) (0.2mm thick):** | |
| | | | **•** The cap is composed of 40 mg **202 S Poly-lactic acid (PLA).** The thickness of the cap is **0.4 mm.** The cap does not contain any drugs or excipients. |
| | | **Vessel (spacer + lower membrane):** | |
| | | | **•** The vessel is made from 105 mg 202 S **Poly-lactic acid (PLA),** providing structural integrity to the system without any added drugs or excipients. |
| **F29** | 30/O/PEO200K/ A - 202S/0.5/ND | **Core:** | |
| | | | **• Polymer (70%):** The core is made of **Poly-ethylene oxide (PEO)** with a molecular weight of **200,000,** in a quantity of **105 mg.** |
| | | | **• Drug (30%):** The active drug in the core is **5-ALA,** present at **45 mg.** |
| | | **Cap (Upper membrane) (0.5mm thick):** | |
| | | | **•** The cap is composed of 50 mg **202 S Poly-lactic acid (PLA).** The thickness of the cap is **0.5 mm.** The cap does not contain any drugs or excipients. |
| | | **Vessel (spacer + lower membrane):** | |
| | | The vessel is made from 120 mg **202 S Poly-lactic acid (PLA),** providing structural integrity to the system without any added drugs or excipients | |

### PLA core-based systems double thickness

To try and extend the drug release over a four-week period, PLA core thickness was doubled, and the drug release was tested (Table **4).**

**Table 4 PLA double thick core systems**

| | | | |
|---|---|---|---|
| **F21** | 2x-30/L/203H/A - 203H/0.2/ND | **Core:** | |
| | | | **• Polymer (70%):** The core is made of **203 H Poly-lactic acid (PLA),** present at **210 mg.** This biodegradable polymer provides structural integrity and enables continuous release of the drug over time. |
| | | | **• Drug (30%):** The active drug in the core is **5-ALA (5-Aminolevulinic acid),** with a dosage of **90 mg.** 5-ALA is used for therapeutic applications and is incorporated into the core for gradual release. |
| | | | **• Excipients:** No additional excipients are included in the core composition. |
| | | **Cap (Upper membrane) (0.2mm thick):** | |
| | | | **•** The cap is composed of 10 mg **203 H Poly-lactic acid (PLA),** serving as a diffusion barrier for the drug release. It does not contain any drugs or excipients. |
| | | **Vessel (spacer + lower membrane):** | |
| | | | **•** The vessel enclosing the system is also made from 130 mg **203 H Poly-lactic acid (PLA),** with no added drugs or excipients. |
| **F22** | 2x-30/L/203H/A - 203H/0.3/ND | **Core:** | |
| | | | **• Polymer (70%):** The core is made of **203 H Poly-lactic acid (PLA),** present at **210 mg.** This biodegradable polymer provides structural integrity and enables continuous release of the drug over time. |
| | | | **• Drug (30%):** The active drug in the core is **5-ALA (5-Aminolevulinic acid),** with a dosage of **90 mg.** 5-ALA is used for therapeutic applications and is incorporated into the core for gradual release. |
| | | | **• Excipients:** No additional excipients are included in the core composition. |
| | | **Cap (Upper membrane) (0.3mm thick):** | |
| | | | **•** The cap is composed of 30 mg **203 H Poly-lactic acid (PLA),** serving as a diffusion barrier for the drug release. It does not contain any drugs or excipients. |
| | | **Vessel (spacer + lower membrane):** | |
| | | | **•** The vessel enclosing the system is also made from 130 mg **203 H Poly-lactic acid (PLA),** with no added drugs or excipients. |
| **F29** | 30/O/PEO200K/ A - 202S/0.5/ND | **Core:** | |
| | | | **• Polymer (70%):** The core is made of **Poly-ethylene oxide (PEO)** with a molecular weight of **200,000,** in a quantity of **105 mg.** |
| | | | **• Drug (30%):** The active drug in the core is **5-ALA,** present at **45 mg.** |
| | | **Cap (Upper membrane) (0.5mm thick):** | |
| | | | **•** The cap is composed of 50 mg **202 S Poly-lactic acid (PLA).** The thickness of the cap is **0.5 mm.** The cap does not contain any drugs or excipients. |
| | | **Vessel (spacer + lower membrane):** | |
| | | The vessel is made from 120 mg **202 S Poly-lactic acid (PLA),** providing structural integrity to the system without any added drugs or excipients | |

**Table 5. Brain Implants of the invention with percentages of each component**

| | **Core** | | | **Upper membrane** | | | **Vessel** | | | Sum | Poly 1 | Poly 2 | 5-ALA | EXP1 | EXP2 | SUM |
|---|---|---|---|---|---|---|---|---|---|---|---|---|---|---|---|---|
| | Poly 1 | 5-ALA | EXP1 | Poly 2 | 5-ALA | EXP2 | Poly 2 | 5-ALA | EXP2 | | % | % | % | % | % | % |
| | PLGA | | | | | | | | | | | | | | | |
| **F3** | 502H | 5-ALA | | PLA 203S | 5-ALA | | PLA 203S | 5-ALA | | | | | | | | |
| | 36 mg | 84 mg | | 10 mg | 0 mg | | 100 mg | 0 mg | | 230 mg | | | | | | |
| | 15,65% | 36,52% | | 4,35% | | | 43,48% | | | 100,00% | 15,65 | 47,83 | 36,52 | 0,00 | 0,00 | 100,00 |
| | 30,00 | 70,00 | 0,00 | 100,00 | 0,00 | 0,00 | 100,00 | 0,00 | 0,00 | | | | | | | |
| | PLGA | | | | | | | | | | | | | | | |
| **F4** | 502H | 5-ALA | | PLA 202S | 5-ALA | | PLA 202S | 5-ALA | | | | | | | | |
| | 36 mg | 84 mg | | 10 mg | 0 mg | | 100 mg | mg | | 230 | | | | | | |
| | 15,65% | 36,52% | | 4,35% | | | 43,48% | | | 100,00% | 15,65 | 47,83 | 36,52 | 0,00 | 0,00 | 100,00 |
| | 30,00 | 70,00 | 0,00 | 100,00 | 0,00 | 0,00 | 100,00 | 0,00 | 0,00 | | | | | | | |
| | PLGA | | | | | | | | | | | | | | | |
| **F10** | 502H | 5-ALA | | PLA 203S | 5-ALA | PEG400 | PLA 203S | 5-ALA | PEG400 | | | | | | | |
| | 36 mg | 84 mg | | 6 mg | | 4 mg | 60 mg | 0 mg | 40 mg | 230 | | | | | | |
| | 15,65% | 36,52% | | 2,61% | | 1,74% | 26,09% | | 17,39% | 100,00% | 15,65 | 28,70 | 36,52 | 0,00 | 19,13 | 100,00 |
| | 30,00 | 70,00 | 0,00 | 60,00 | 0,00 | 40,00 | 60,00 | 0,00 | 40,00 | | | | | | | |
| | PLGA | | | | | | | | | | | | | | | |
| **F11** | 502H | 5-ALA | | PLA 203S | 5-ALA | | PLA 203S | 5-ALA | | | | | | | | |
| | 36 mg | 84 mg | | 9 mg | 1 mg | | 90 mg | 10 mg | | 230 mg | | | | | | |
| | 15,65% | 36,52% | | 3,91% | 0,43% | | 39,13% | 4,35% | | 100,00% | 15,65 | 43,04 | 41,30 | 0,00 | 0,00 | 100,00 |
| | 30,00 | 70,00 | 0,00 | 90,00 | 10,00 | 0,00 | 90,00 | 10,00 | 0,00 | | | | | | | |
| | PLGA | | | | | | | | | | | | | | | |
| **F14** | 502H | 5-ALA | | PLA 203S | 5-ALA | | PLA 203S | 5-ALA | | | | | | | | |
| | 36 mg | 84 mg | | 9,4 mg | 0,6 mg | | 94 mg | 6 mg | | 230 mg | | | | | | |
| | 15,65% | 36,52% | | 4,09% | 0,26% | | 40,87% | 2,61% | | 100,00% | 15,65 | 44,96 | 39,39 | 0,00 | 0,00 | 100,00 |
| | 30,00 | 70,00 | 0,00 | 94,00 | 6,00 | 0,00 | 94,00 | 6,00 | 0,00 | | | | | | | |
| | PLGA | | | | | | | | | | | | | | | |
| **F15** | 502H | 5-ALA | | PLA 203S | 5-ALA | | PLA 203S | 5-ALA | | | | | | | | |
| | 36 mg | 84 mg | | 9,2 mg | 0,8 mg | | 92 mg | 8 mg | | 230 mg | | | | | | |
| | 15,65% | 36,52% | | 4,00% | 0,35% | | 40,00% | 3,48% | | 100,00% | 15,65 | 44,00 | 40,35 | 0,00 | 0,00 | 100,00 |
| | 30,00 | 70,00 | 0,00 | 92,00 | 8,00 | 0,00 | 92,00 | 8,00 | 0,00 | | | | | | | |
| **F19** | PLA 203H | 5-ALA | | PLA 203H | 5-ALA | | PLA 203H | 5-ALA | | | | | | | | |
| | 90 mg | 60 mg | | 10 mg | | | 100 mg | | | 260 mg | | | | | | |
| | 34,62% | 23,08% | | 3,85% | | | 38,46% | | | 100,00% | 34,62 | 42,31 | 23,08 | 0,00 | 0,00 | 100,00 |
| | 60,00 | 40,00 | 0,00 | 100,00 | 0,00 | 0,00 | 100,00 | 0,00 | 0,00 | | | | | | | |
| **F20** | PLA 203H | 5-ALA | | PLA 203H | 5-ALA | | PLA 203H | 5-ALA | | | | | | | | |
| | 105 mg | 45 mg | | 10 mg | | | 100 mg | | | 260 mg | | | | | | |
| | 40,38% | 17,31% | | 3,85% | | | 38,46% | | | 100,00% | 40,38 | 42,31 | 17,31 | 0,00 | 0,00 | 100,00 |
| | 70,00 | 30,00 | 0,00 | 100,00 | 0,00 | 0,00 | 100,00 | 0,00 | 0,00 | | | | | | | |
| **F21** | PLA 203H | 5-ALA | | PLA 203H | 5-ALA | | PLA 203H | 5-ALA | | | | | | | | |
| | 210 mg | 90 mg | | 10 mg | | | 130 mg | | | 440 mg | | | | | | |
| | 47,73% | 20,45% | | 2,27% | | | 29,55% | | | 100,00% | 47,73 | 31,82 | 20,45 | 0,00 | 0,00 | 100,00 |
| | 70,00 | 30,00 | 0,00 | 100,00 | 0,00 | 0,00 | 100,00 | 0,00 | 0,00 | | | | | | | |
| **F22** | PLA 203H | 5-ALA | | PLA 203H | 5-ALA | | PLA 203H | 5-ALA | | | | | | | | |
| | 210 mg | 90 mg | | 30 mg | | | 130 mg | | | 460 mg | | | | | | |
| | 45,65% | 19,57% | | 6,52% | | | 28,26% | | | 100,00% | 45,65 | 34,78 | 19,57 | 0,00 | 0,00 | 100,00 |
| | 70,00 | 30,00 | 0,00 | 100,00 | 0,00 | 0,00 | 100,00 | 0,00 | 0,00 | | | | | | | |

| | **Core** | | | **Upper** membrane | | | **Vessel** | | | Sum | Poly 1 | Poly 2 | 5-ALA | EXP1 | EXP2 | SUM |
|---|---|---|---|---|---|---|---|---|---|---|---|---|---|---|---|---|
| | Poly 1 | 5-ALA | EXP1 | Poly 2 | 5-ALA | EXP2 | Poly 2 | 5-ALA | EXP2 | | % | % | % | % | % | % |
| **F24** | PLA 203H | 5-ALA | Citric acid | PLA 203H | 5-ALA | | PLA 203H | 5-ALA | | | | | | | | |
| | 105 mg | 45 mg | 16,6 mg | 10 mg | | | 100 mg | | | 276,6 mg | | | | | | |
| | 37,96% | 16,27% | 6,00% | 3,62% | | | 36,15% | | | 100,00% | 37,96 | 39,77 | 16,27 | 6,00 | 0,00 | 100,00 |
| | 63,03 | 27,01 | 9,96 | 100,00 | 0,00 | 0,00 | 100,00 | 0,00 | 0,00 | | | | | | | |
| **F25** | PLA 203H | 5-ALA | Fumaric acid | PLA 203H | 5-ALA | | PLA 203H | 5-ALA | | | | | | | | |
| | 105 mg | 45 mg | 16,6 mg | 10 mg | | | 100 mg | | | 276,6 mg | | | | | | |
| | 37,96% | 16,27% | 6,00% | 3,62% | | | 36,15% | | | 100,00% | 37,96 | 39,77 | 16,27 | 6,00 | 0,00 | 100,00 |
| | 63,03 | 27,01 | 9,96 | 100,00 | 0,00 | 0,00 | 100,00 | 0,00 | 0,00 | | | | | | | |
| **F27** | PEO | 5-ALA | | PLA 202S | 5-ALA | | PLA 202S | 5-ALA | | | | | | | | |
| | 105 mg | 45 mg | | 40 mg | | | 105 mg | | | 295 mg | | | | | | |
| | 35,59% | 15,25% | | 13,56% | | | 35,59% | | | 100,00% | 35,59 | 49,15 | 15,25 | 0,00 | 0,00 | 100,00 |
| | 70,00 | 30,00 | 0,00 | 100,00 | 0,00 | 0,00 | 100,00 | 0,00 | 0,00 | | | | | | | |
| **F29** | PEO | 5-ALA | | PLA 202S | 5-ALA | | PLA 202S | 5-ALA | | | | | | | | |
| | 105 mg | 45 mg | | 50 mg | | | 120 mg | | | 320 | | | | | | |
| | 32,81% | 14,06% | | 15,63% | | | 37,50% | | | 100,00% | 32,81 | 53,13 | 14,06 | 0,00 | 0,00 | 100,00 |
| | 70,00 | 30,00 | 0,00 | 100,00 | 0,00 | 0,00 | 100,00 | 0,00 | 0,00 | | | | | | | |

| | | | | | | | | | | | | | | | | |
|---|---|---|---|---|---|---|---|---|---|---|---|---|---|---|---|---|
| Poly 1 = core polymer Poly 2 = shell polymer EXP1 = excipient in a core EXP2 = excipient in a shell | | | | | | | | | | | | | | | | |

### Reference formulations for comparision to the inventive brain implants

### Manufacturing of Monolithic Systems via Film Casting

The monolithic implants were produced by dissolving 5-ALA in a solvent blend of dichloromethane (DCM) and methanol. This mixture was then combined with PLGA using magnetic stirring (Stuart UC152, Cole-palmer, United Kingdom) to ensure even distribution. The resulting solution was cast into a mould and allowed to evaporate overnight in a fume hood, forming a solid monolithic film. The film underwent cross-sectional analysis using the Selective Electron Microscopy (SEM) (Phenom, ProX, Thermo Fisher) and drug release testing. For SEM observation, all the MNs were mounted on metallic stubs and coated with a 5nm layer of gold using a LUXOR coater (Luxor Tech, Belgium) under vacuum conditions. A list of the fabricated systems is detailed in **Table 6.**

### Manufacturing of Core-shell Systems Via Dip Coating

The dip-coating method involves immersing a PLGA core containing the drug (manufactured as per Section 6.2.1.2) into a PLA polymer solution, then slowly withdrawing it to allow a thin film of the coating to deposit on the surface. The coated sample was left to dry for 10 min, enabling the solvent to evaporate and forming a solid layer. Finally, the sample undergoes post-heat treatment at 30°C overnight in an oven (Cole-Parmer, OVV400, United Kingdom) to enhance adhesion and mechanical properties. After fabrication, all the samples were measured with a vernier calliper for thickness. A list of the fabricated systems is detailed in **Table 6.**

### Manufacturing of Core-Shell System Via Film Casting and Thermal Sealing

To fabricate the core-shell system using thermal sealing, the PLGA-5-ALA (5-ALA-loaded PLGA) core (manufactured as per Section 6.2.1.1) was positioned between two PLA films that were prepared by solvent evaporation method as mentioned in Section 6.2.1.1, two drops of dichloromethane (DCM) solution to promote adequate adhesion. The layered assembly is then compressed using a glass plate to ensure uniform contact, achieving final dimensions of approximately 1.2 cm by 1.2 cm. Following compression, the edges of the PLA layers are sealed using DCM solution. The sealed units were cured in an oven (Cole Parmer OV-400, United Kingdom) at 30°C for 24 h to ensure complete bonding and stabilization of the material layers. After curing, each sample was visual inspected to verify structural integrity, and cross-sectional analysis was conducted using SEM (Phenom, ProX, Thermo Fisher) to confirm proper bonding and assess permeability characteristics. A list of the fabricated systems is detailed in **Table 6.**

**Table 6. List of manually assembled formulations**

| **MF No.** | **Name** | **Description** |
|---|---|---|
| **Monolithic systems via film casting** | | |
| **MF 1** | O/4.8/G/R 203S/A/0.2 | **Composition:** PLGA 203S weighing 300 mg (95.2%) and 15 mg (4.8%) of 5-aminolevulinic acid (5-ALA) as the drug. |
| | | **Solvent Amounts:** DCM: 1.5 mL, Methanol: 1 mL. |
| | | **Blend ratio (PLGA + DCM): (5-ALA + Methanol)** = 3.97:13.97:1. |
| | | **Solvent blend ratio (Methanol: DCM)** = 1:1.51:1.5 |
| **MF 2** | O/4.8/G/R 502H/A/0.2 | **Composition:** PLGA 502H weighing 300 mg (95.2%) and 15 mg (4.8%) of 5-aminolevulinic acid (5-ALA) as the drug. |
| | | **Solvent Amounts:** DCM: 1.75 mL, Methanol: 0.75 mL. |
| | | **Blend ratio (PLGA + DCM) :(5-ALA + Methanol)** = 3.98:1 |
| | | **Solvent blend ratio (Methanol: DCM)** = 1:2.331:2.33 |
| **MF 3** | O/4.8/G/R 757S/A/0.2 | **Composition:** PLGA 757S weighing 300 mg (95.2%) and 15 mg (4.8%) of 5-aminolevulinic acid (5-ALA) as the drug. |
| | | **Solvent Amounts:** DCM: 2.2 mL, Methanol: 0.8 mL |
| | | **Blend ratio (PLGA + DCM) :(5-ALA + Methanol)** = 3.99:1 |
| | | **Solvent blend ratio (Methanol: DCM)** = 1:2.75 |
| **MF 4** | O/4.8/G/R 858S/A/0.2 | **Composition:** PLGA 858S weighing 300 mg (95.2%) and 15 mg (4.8%) of 5-aminolevulinic acid (5-ALA) as the drug. |
| | | **Solvent Amounts:** DCM: 1.8 mL, Methanol: 0.6 mL. |
| | | **Blend ratio (PLGA + DCM) :(5-ALA + Methanol)** = 3.99:1 |
| | | **•Solvent blend ratio (Methanol: DCM)** = 1:31:3. |

| **Core-shell system via dip coating** | | |
|---|---|---|
| **MF 5** | D/50/G/R502H/A - 20/L/203S/6 | **Core:** |
| | | **Polymer (50%):** The core comprises **PLGA 502H,** weighing **60 mg.** |
| | | **Drug (50%):** The core contains 60 mg of 5-aminolovulenic acid |
| | | **Coating layer:** 6 **layers** of coating is achieved by dipping the core in 20% 203S polymer. |
| | | The blend ratio of **(Polymer + Solvent): (Drug)** is approximately **14.17:1,** while the solvent-to-polymer ratio |
| | | **(Methanol: PLGA)** is approximately **13.17:1** |
| **MF 6** | D/50/G/R502H/A - 20/U203S/7 | **Core:** |
| | | **Polymer (50%):** The core comprises **PLGA 502H,** weighing **60 mg.** |
| | | **Drug (50%):** The core contains **60 mg** of 5-aminolovulenic acid |
| | | **Coating layer: 7 layers** of coating is achieved by dipping the core in 20% 203S polymer. |
| | | The blend ratio of **(Polymer + Solvent) : (Drug)** is approximately **14.17:1,** while the solvent-to-polymer ratio |
| | | **(Methanol:PLGA)** is approximately **13.17:1** |
| **MF 7** | D/50/G/R502H/A - 20/L/203S/8 | **Core:** |
| | | **Polymer (50%):** The core comprises **PLGA 502H,** weighing **60 mg.** |
| | | **Drug (50%):** The core contains **60 mg** of 5-aminolovulenic acid |
| | | **Coating layer: 8 layers** of coating is achieved by dipping the core in **20% 203S polymer.** |
| | | The blend ratio of **(Polymer** + **Solvent)** : **(Drug)** is approximately **14.17:1,** while the solvent-to-polymer ratio |
| | | **(Methanol:PLGA)** is approximately **13.17:1** |

| **Core-shell systems via film casting and thermal sealing** | | |
|---|---|---|
| **MF 8** | **H/50/G/R502H/A -** 20/L/203S | **Core:** |
| | | **Polymer (50%):** The core comprises **PLGA 502H,** weighing **60 mg.** |
| | | **Drug (50%):** The core contains **60 mg** of 5-aminolovulenic acid |
| | | **Coating film (0.3mm thick):** The cap is constructed from **PLA 203 S** polymer |
| | | The blend ratio of **(Polymer + Solvent): (Drug)** is approximately **14.17:1,** while the solvent-to-polymer ratio |
| | | **(Methanol: PLGA)** is approximately **13.17:1** |

### Implant 5-ALA Release

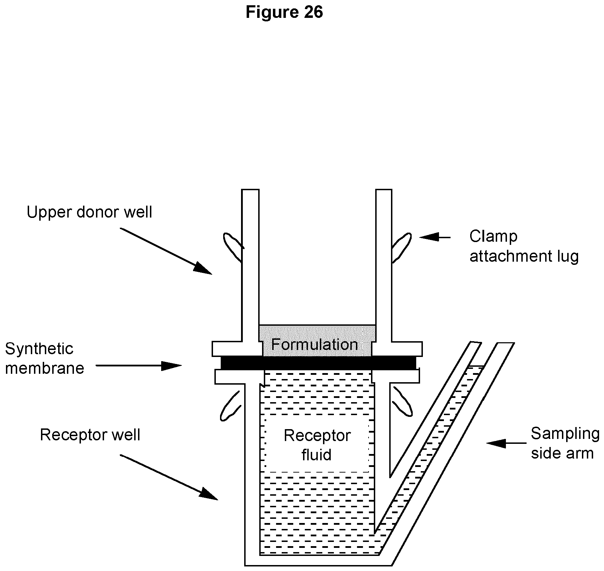

The release of 5-ALA was assessed using the Franz cell as shown in **Figure 26****.** The 5-ALA-loaded films were placed in the Franz cell donor compartments in 1 ml of PBS. The system was placed on an underwater magnetic stirrer (Cowie 12*4.5mm stirrer bars, United Kingdom) maintained at a constant temperature of 37°C using a water bath (Grant OLS 200, United Kingdom) with continuous stirring in the receptor compartments to ensure homogeneity. Samples are collected from the receptor compartment at hourly or daily intervals, with an equal volume of fresh PBS replaced to maintain a consistent receptor volume. Collected samples are stored at room temperature for no longer than a day and analysed using high-performance liquid chromatography (HPLC, Section 6.2.1) to determine the concentration of 5-ALA. The cumulative release of 5-ALA was calculated, accounting for the replacement of the receiver fluid, and the data was plotted to assess drug release over time **(****Figure 26****).**

### Mechanical Properties Assessment

The implant sample was clamped at both ends within the grips of Lloyd instruments TG420 S1038 ensuring proper centring and alignment to avoid bending stresses or uneven force distribution. The testing speed was set according to the material standard (5 mm/min), and the initial dimensions of the sample were inputted into the software for real-time stress-strain calculations. Endpoint criteria, including sample break or maximum elongation, defined before starting the test were measured for each sample. During the test, machine applied a gradual tensile force to the sample and the stress-strain curve was monitored in real time on the software.

### PLGA Core Manufacturing Via Vacuum Compression Method

The implant core was composed of a PLGA polymer matrix containing 5-ALA. The two components were weighed and placed directly into a mortar and pestle; they were ground together until a homogenous mixture was formed. Then, the mixture is transferred into a PTFE-lined D10 mm Vapour Control Module (VCM) (Melt Prep VCM, Austria) chamber, securing the lid and heating at 120°C for 10 minutes. The VCM tool is then cooled for 5 minutes, and the foils are carefully removed to measure the sample's weight and thickness. The details of the different cores are shown in **Table 7.**

**Table 7 PLGA polymer-based cores**

| **MF No.** | **Name** | **Description** |
|---|---|---|
| **C 1** | 50/G/R 757S/A | The system is formulated with **PLGA 757S** weighing **75 mg (50%)** along **with 75 mg (50%)** of 5-aminolovulenic acid as the drug |
| **C 2** | 70/G/R 757S/A | The system is formulated with **PLGA 757S** weighing **105 mg (70%)** along **with 45 mg (30%)** of 5-aminolovulenic acid as the drug |
| **C 3** | 50/G/R 502H/A | The system is formulated with **PLGA 502H** weighing **75 mg (50%)** along **with 75 mg (50%)** of 5-aminolovulenic acid as the drug. |
| **C 4** | 50/G/R 858S/A | The system is formulated with **PLGA 858S** weighing **75 mg (50%)** along **with 75 mg (50%)** of 5-aminolovulenic acid as the drug. |
| **C 5** | 30/G/R 858S/A | The system is formulated with **PLGA 858S** weighing **105 mg (70%)** along with **45 mg (30%)** of 5-aminolovulenic acid as the drug. |

### PLA Core-Shell Systems Via Vacuum Compression Moulding

The implant core was composed of a PLA polymer matrix containing 5-ALA as per Table **8.** The two components were weighed and placed directly into a mortar and pestle and were ground together until a homogenous mixture was formed. Then, mixture is transferred into a PTFE-lined D10 mm Vapour Control Module (VCM) (Melt Prep VCM, Austria) chamber, securing the lid, and heating at 120°C for 10 minutes. The VCM tool is then cooled for 5 minutes, and the foils are carefully removed to measure the sample's weight and thickness. The cores were manufactured using the following machine parameters. **Table 8** is the list of the core for the implants developed with PLA polymer.

**Table 8 PLA polymer-based cores**

| | | |
|---|---|---|
| **C 6** | 30/G/R 203H/A | The system is formulated with **PLGA 203H** weighing **105 mg (70%)** along with **45 mg (30%)** of 5-aminolovulenic acid as the drug |
| **C 7** | 30/G/R 203S/A | The system is formulated with **PLGA 203S** weighing **105 mg (70%)** along with **45 mg (30%)** of 5-aminolovulenic acid as the drug |
| **C 8** | 30/G/R 202S/A | The system is formulated with **PLGA 202S** weighing **105 mg (70%)** along with **45 mg (30%)** of 5-aminolovulenic acid as the drug |
| **C 9** | 25/G/R 202S/A | The system is formulated with **PLGA 202S** weighing **112.5 mg (75%)** along with **37.5 mg (25%)** of 5-aminolovulenic acid as the drug |
| **C 10** | 40/G/R 203H/A | The system is formulated with **PLGA 203H** weighing **90 mg (60%)** along with **60 mg (40%)** of 5-aminolovulenic acid as the drug |

### Example 2: Film Implant Proof-of-Concept Development and Testing

### Analytical Method Development

### 5-ALA Analytical Method

### 5-ALA Analytical Method

The HPLC analytical method for quantifying 5-ALA utilises a Jupiter Proteo 90 Å column (4 µm, 250 x 4.6 mm) with a mobile phase comprising of a buffer at pH 7.0 and methanol in a 90:10 ratio, incorporating octane sulfonate sodium as an ion-pairing agent. The flow rate is 0.5 mL/min, and the injection volume is 10 µL. The column and detector are maintained at 25°C. Calibration standards are prepared in phosphate-buffered saline (PBS) ranging from 10 to 500 µg/mL, establishing a linear calibration curve (R² = 0.999). The method ensures baseline resolution and accurate quantification, with 200 µg/mL 5-ALA as a representative concentration for system suitability testing.

### 5-ALA Extraction Method

Two stock solutions were prepared to prepare the calibration curve for 5-ALA analysis from the implant matrix. First, 15 mg of 5-ALA was dissolved in 10 mL of methanol to create a 1.5 mg/mL 5-ALA stock solution, then 30 mg of PLGA or PLA polymer was dissolved in 20 mL of dichloromethane (DCM) to form a 1.5 mg/mL polymer solution. Seven standard solutions were prepared by mixing specific volumes of 5-ALA and polymer solutions, then concentrating them under a fume hood to remove solvents. Each film was created by combining 1 mL of methanol solution (15 mg ALA) with 1.5 mL of DCM solution (300 mg PLGA) and casting the mixture, a process repeated twice to yield a total of 30 mg ALA and 600 mg PLGA. The blend ratio, calculated as the total mass of ALA divided by the total mass of PLGA, results in a 1:20 (ALA: PLGA) ratio. This ratio optimizes ALA delivery while leveraging PLGA's properties for structural integrity, and continuous 5-ALA delivery. The combination ensures a robust, effective film suitable for therapeutic applications. The dried samples were reconstituted in 5 mL of a 1:1 PBS/DCM mixture, vortexed for 20 seconds, and shaken overnight at 25 °C in a shaking water bath. After complete phase separation, the samples were centrifuged at 4000 rpm for 10 min, filtered, and analysed using HPLC with the 5-ALA LC method (Section 6.2.1.1), with wash solutions inserted between runs. For sample extraction from the polymer implants, the weight of the implant was recorded, and the film was placed in a 7 mL vial with 5 mL of a PBS/DCM mixture, vortexed for 20 s, and shaken in a water bath at 25 °C for 20 min. The solution was left to separate, centrifuged, filtered, and analysed by HPLC, adding wash solutions between runs to maintain system cleanliness.

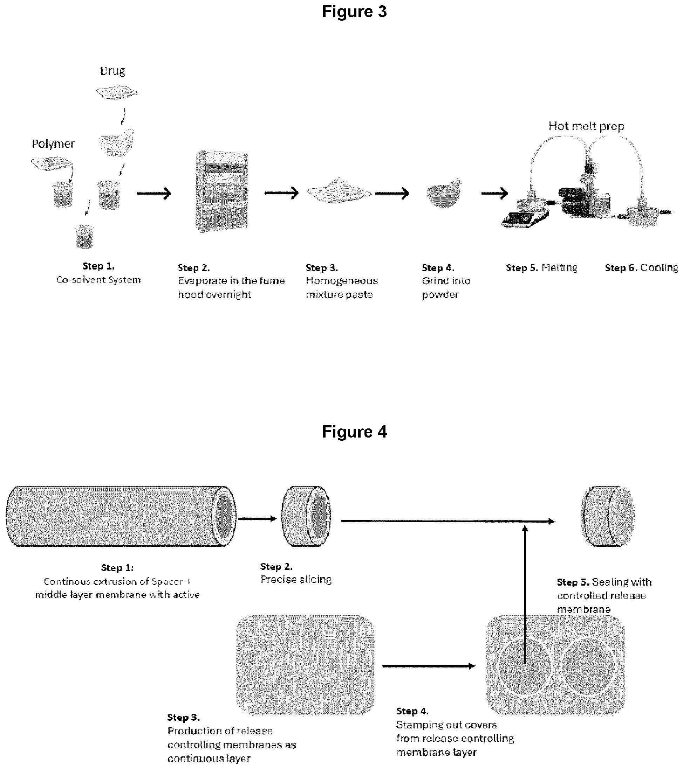

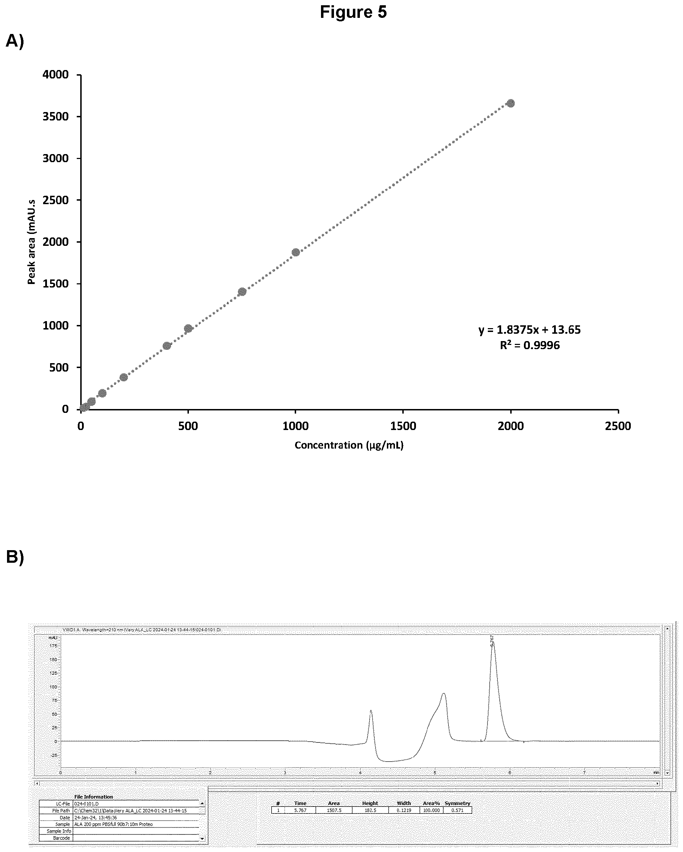

The 5-ALA analytical method was successfully established, demonstrating high linearity within the concentration range of 12.5-2000 µg/mL in PBS, with an R² value of 0.9996 with LOD and LOQ 38.9 µg/mL and 116.6 µg/mL (Figures **4** and **5****).** This method demonstrates high degree of precision and accuracy for 5-ALA quantification (Table 9).

**Table 9 Accuracy and Precision of 5-ALA Analytical Method**

| **Theoretical concentration (µg/mL)** | **Retention time (min)** | **Peak area (mAU.s)** | **Measured concentration (µg/mL)** | **Recovery percentage (%)** |
|---|---|---|---|---|
| 400 | 5.747 | 604.50787 | 403.41 | 100.85 |
| 400 | 5.745 | 604.81622 | 403.61 | 100.90 |
| 400 | 5.743 | 603.98413 | 403.06 | 100.76 |
| 500 | 5.746 | 751.45306 | 501.47 | 100.29 |
| 500 | 5.747 | 750.37164 | 500.74 | 100.15 |
| 500 | 5.744 | 750.06604 | 500.54 | 100.11 |
| 600 | 5.745 | 911.68708 | 608.39 | 101.40 |
| 600 | 5.747 | 909.79718 | 607.13 | 101.19 |
| 600 | 5.747 | 910.49807 | 607.60 | 101.27 |
| **AVERAGE (Accuracy)** | | | | 100.77 |
| **SD** | | | | 0.49 |
| **RSD (Precision)** | | | | 0.48 |

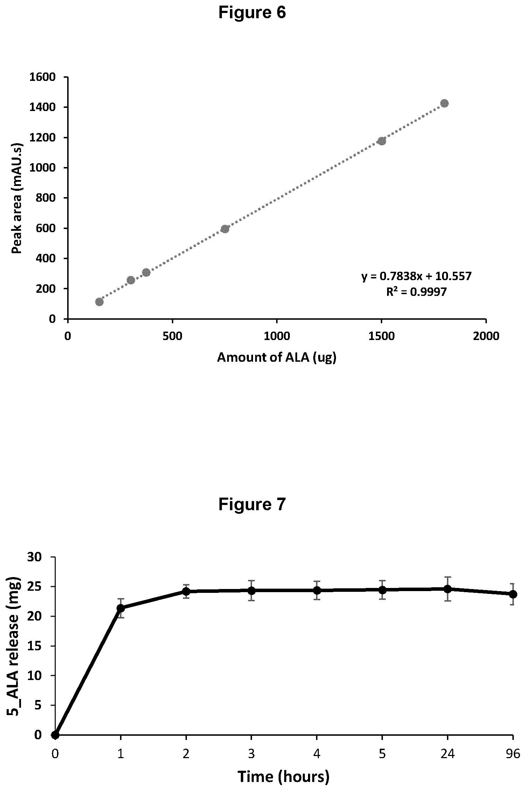

First, **30 mg** of 5-ALA was dissolved in 10 mL of methanol to create a **3 mg/mL** 5-ALA stock solution, second 30 mg of PLGA or PLA polymer was dissolved in 20 mL of dichloromethane (DCM) to form a 1.5 mg/mL polymer solution. The extraction method for 5-ALA gave a linear calibration with an equation **y** = **0.7838x** + **10.557** and an R² value of 0.9997, indicating a good method for 5-ALA (Figure **6****).**

### Preparation of Mixtures:

| **Concentration (ug/mL)** | **ALA solution volume (mL)** | **Polymer solution volume (mL)** | **Total volume (mL)** |
|---|---|---|---|
| **1800** | 1.2 | 0.8 | 2 |
| **1500** | 1 | 1 | 2 |
| **750** | 0.5 | 1.5 | 2 |
| **375** | 0.25 | 1.75 | 2 |
| **300** | 0.2 | 1.8 | 2 |
| **150** | 0.1 | 1.9 | 2 |

### Franz Cell Method Development

A release study of 5-ALA was conducted using a Franz cell setup to evaluate the stability and release profile from a 30 mg 5-ALA sample in a 0.5 ml PBS solution. The results indicated that 5-ALA release reached a plateau within the first five hours, achieving a cumulative amount of approximately 25 mg, with minimal variation observed in subsequent measurements up to 96 h (Figure **7****).**

A Franz cell method was developed to assess the release of 5-ALA from developed implants. To test the retardation of the 5-ALA release through the membrane used in the Franz cell, 30mg of 5-ALA in 0.5 ml of PBS fluid (pH 7.4) was placed in the donor section of six individually calibrated Franz cells (University of Southampton, UK). The receptor compartment was filled with phosphate buffer solution (PBS) at pH 7.4 to mimic physiological conditions. The system was maintained at 37°C to simulate body temperature using a water bath (Grant OLS 200, United Kingdom) and an underwater stirrer (Cowie 12*4.5mm stirrer bars, United Kingdom) to ensure homogeneity of the receiver samples. Samples are collected from the receptor compartment at 1, 2, 3, 4, 5, 24, 96 h using a 10 ml syringe to evaluate 5-ALA's release profile. There was no agitation of the donor chamber to simulate the lack of movement of fluid in the brain cavity (Figure **26****).**

### Chemical Stability Testing

The study aimed to assess the stability of 5-ALA in aqueous solutions at varying pH levels (6, 6.8, and 7.4) over an 8-day period at 37°C. Results indicate that 5-ALA degradation was pH-dependent, with higher pH values accelerating the decomposition rate (Figure **8****).** Specifically, at pH 7.4, 5-ALA concentration decreased more significantly, showing a 12% reduction to 87.98% by the end of the study, compared to more stable concentrations around 95% and 100% at pH 6 and 6.8, respectively. The findings align with de Blois et al.

(2002) research, which also observed increased 5-ALA degradation in aqueous solutions at elevated pH, particularly at room temperature. This data suggests that maintaining 5-ALA in lower pH conditions may enhance its stability.

### Example 3: Studies of Controlled release of brain implants

### F3: 70/G/PLGA502H/A- 203S/0.15/ND

The release profile of 5-ALA showed a rapid and linear increase during the initial 5 days with a rate of 6.15 mg/day, followed by a slower and plateauing release trend over days 5-28, with a maximum release of 47.22 mg (67.45 %) (Figure **9****).**

**Figure 9** shows active release of F3 (70/G/PLGA502H/A- 203S/0.15/ND). Formulation composition described in Table.1. The plotted points represent the mean ± SD (n = 3)

### F4: 70/G/PLGA502H/A- 202S/0.2/ND

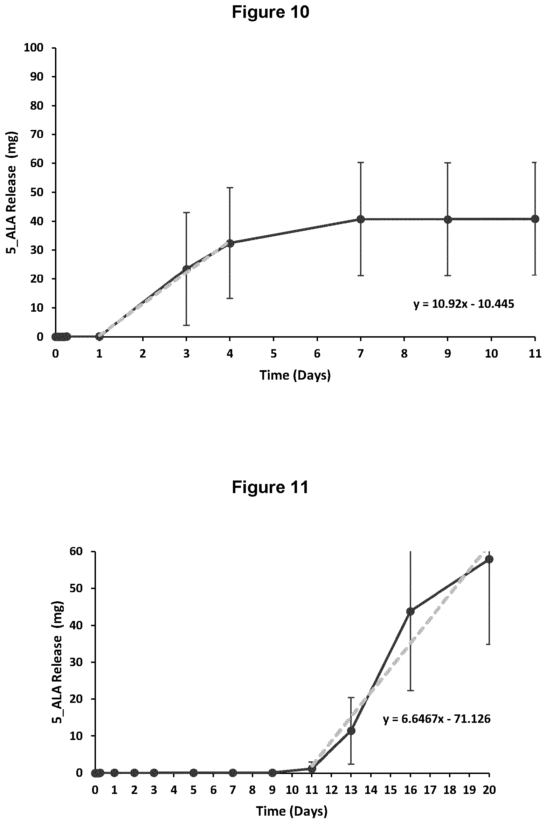

5-ALA showed a linear release from day 1 to 4 at 10.92 mg/day. Following that, the release was slower and plateaued from day 7 with a maximum release extent of 40.87 mg (58.38 %) (Figure **10****)**

**Figure 10** shows active release of F4 (70/G/PLGA502H/A- 202S/0.2/ND). Formulation composition described in Table.1. The plotted points represent the mean ± SD (n = 3).

### F10: 70/G/PLGA502H/A- 203S/40/E-PEG400/0.2/ND

5-ALA showed no release till day 9 followed by a linear release from day 11 to 20 at 6.64 mg/day with a total release of 57.89 (82.7 %). **(****Figure11****).**

**Figure 11** shows active release of F10 (70/G/PLGA502H/A- 203S/40/E-PEG400/0.2/ND). Formulation composition described in Table.1. The plotted points represent the mean ± SD (n = 3).

### F11: 70/G/PLGA502H/A - 203S/0.2/10/D

5-ALA showed a linear release till day 6 at the rate of 4.41 mg/day. The release is slower and plateaued from day 16 with a maximum release extent of 44.27 mg (52.70 %) (Figure **12****).**

**Figure 12** shows active release F11 (70/G/PLGA502H/A - 203S/0.2/10/D) Formulation composition described in Table.1. The plotted points represent the mean ± SD (n = 3).

### F14: 70/G/PLGA502H/A - 203S/0.2/6/D

5-ALA shows a linear release until day 10 at 4.9 mg/day with a release extent of 54.37 mg (42.86 %) (Figure **13****).**

Figure **13** shows active release of F14 (70/G/PLGA502H/A - 203S/0.2/6/D). Formulation composition described in Table.1. The plotted points represent the mean ± SD (n = 3)

### F15: 70/G/PLGA502H/A - 203S/0.2/8/D

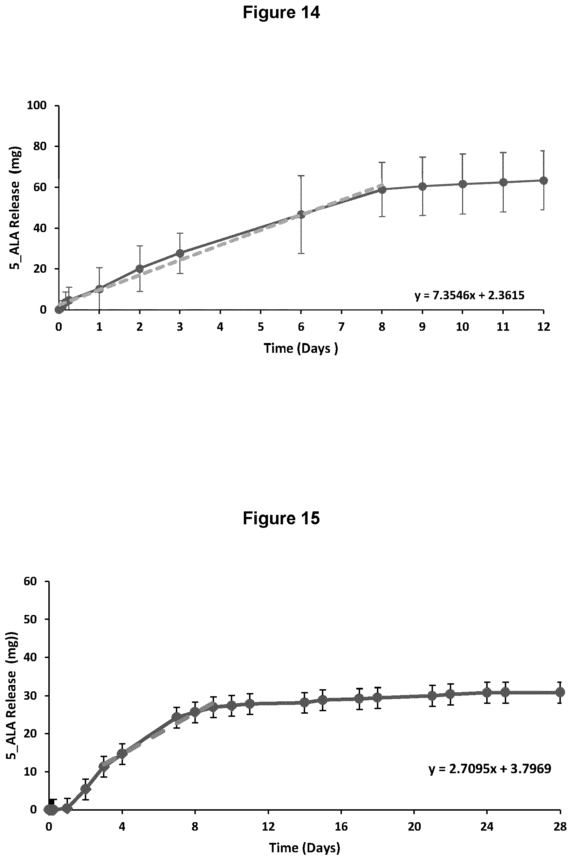

5-ALA showed steady linear release till day 8 at 7.35 mg/day followed by a slow release and plateauing from day 9 with a release extent of 63.41 mg (75.49 %) (Figure **14****).**

**Figure 14** shows active release of F15 (70/G/PLGA502H/A - 203S/0.2/8/D). Formulation composition described in Table.1. The plotted points represent the mean ± SD (n = 3)

### F19: 40/L/203H/A - 203H/0.2/ND

5-ALA showed a linear release till day 9 with a release rate of 2.7 mg/day followed by a slow release leading to plateauing after 2 weeks with a maximum release extent of 30.81 mg (34.34 %) **(****Figure 15****).**

**Figure 15** shows active release of F19 (40/L/203H/A - 203H/0.2/ND). Formulation composition described in Table.2. The plotted points represent the mean ± SD (n = 3)

### F20: 30/L/203H/A - 203H/0.2/ND

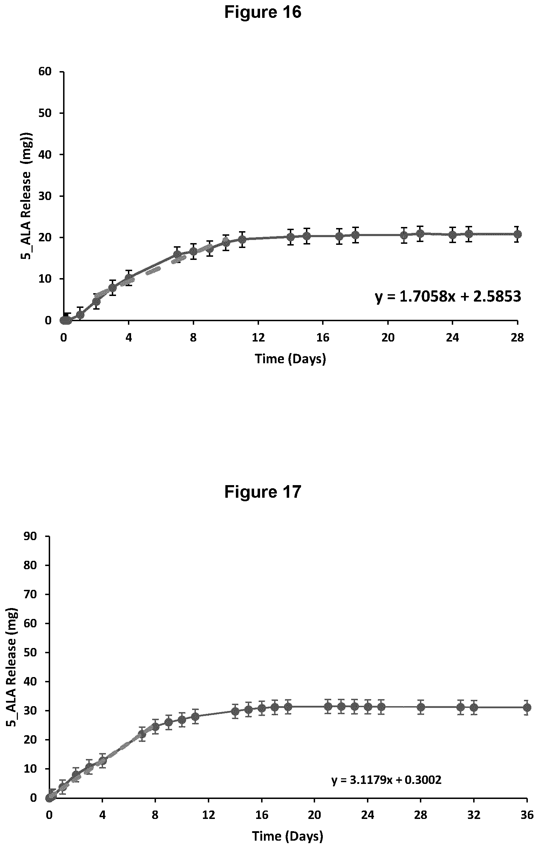

5-ALA showed a linear release till day 10 with a release rate of 1.7 mg/day followed by a slow release leading to plateauing after 2 weeks with a maximum release extent of 20.77 mg (37.94 %) (Figure **16****).**

Figure **16** shows active release of F20 (30/L/203H/A - 203H/0.2/ND). Formulation composition described in Table.2. The plotted points represent the mean ± SD (n = 3)

### Prototpe 1: PLA core-based systems with double thickness

### F21: 2x-30/L/203H/A - 203H/0.2/ND

5-ALA showed a linear release till day 8 with a release rate of 3.11 mg/day followed by a slow release leading to plateauing after 18 days with a maximum release extent of 31.51 mg (34.54 %) (Figure **17****).**

**Figure 17** shows active release of F21 (2x-30/L/203H/A - 203H/0.2/ND). Formulation composition described in Table.3. The plotted points represent the mean ± SD (n = 3)

### F22: 2x-30/L/203H/A - 203H/0.3/ND

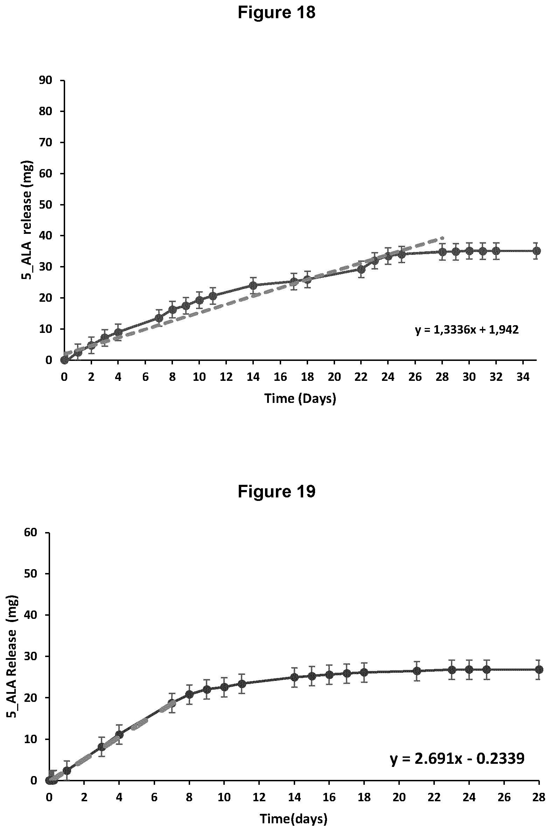

5-ALA shows a linear release till day 25 with a release rate of 1.33 mg/day followed by a slow release leading to plateauing after 28 days with a maximum release extent of 35.15 mg (39.05 %) (Figure **18****).**

**Figure 18** shows active release of F22 (2x-30/L/203H/A - 203H/0.3/ND). Formulation composition described in Table.3. The plotted points represent the mean ± SD (n = 3)

### Formulation Optimisation of Core-Shell Systems Produced Via VCM

After the release results from all the PLGA and PLA formulations, PLA formulations were chosen for further optimisation. Along with that, additionally PEO were selected as an alternative polymer for the core. To increase the release extent of PLA formulations, pH regulators were added to the core as pH influences the ionization state of both the drug and the release medium. Ionized forms of drugs are often more soluble, which increases their diffusion through the implant or material, enhancing the release extent. The other method to improve the release extent by adding more drug into the core. Therefore, a core with double the thickness and the drug were fabricated successfully and was sealed with the cap and vessel with different level of thickness. PEO being a non-ionic polymer, it interacts well with ionic compound such as 5-ALA and stabilizes them, as well as promoting the solubility and diffusion through the matrix. All the formulations were successfully manufactured through vacuum compression method.

### 5-ALA Release Testing from Implant Vacuum Compression Moulding Formulation

### Prototype 1: PLA core-based systems with pH regulators

### F24: 27/203H/A/10/C - 203H/0.2/ND

5-ALA showed a linear release till day 7 with a release rate of 2.69 mg/day followed by a slow release leading to plateauing after 14 days with a maximum release extent of 26.84 mg (59.30 %) (Figure **19****).**

**Figure 19** shows active release of F24 (27/203H/A/10/C - 203H/0.2/ND). Formulation composition described in Table.3. The plotted points represent the mean ± SD (n = 3)

### F25: 27/203H/A/10/F - 203H/0.2/ND

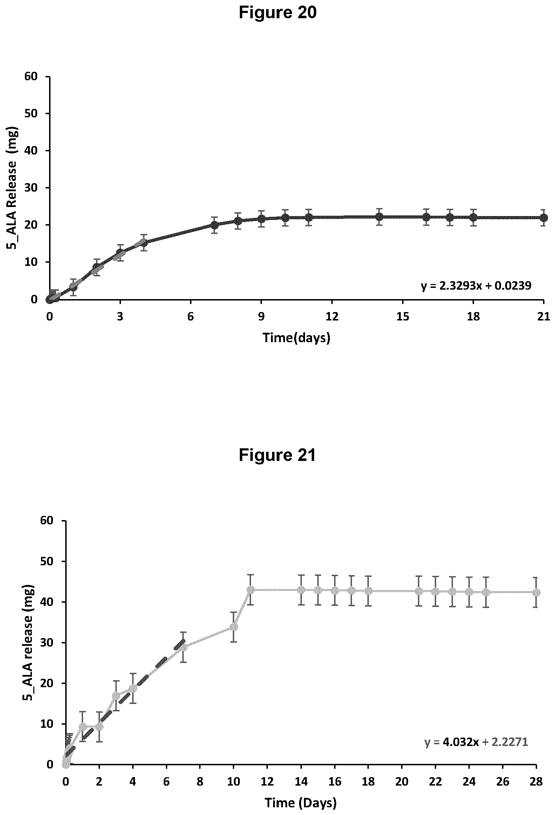

5-ALA shows a linear release till day 4 with a release rate of 2.32 mg/day followed by a slow release leading to plateauing after 9 days with a maximum release extent of 22 mg (41.20 %) (Figure **20****).**

**Figure 20** shows active release of F25 (27/203H/A/10/F - 203H/0.2/ND). Formulation composition described in Table.3. The plotted points represent the mean ± SD (n = 3).

### Prototype 1: Poly(ethylene oxide) core-based systems

### F27: 30/O/PEO200K/A - 202S/0.4/ND

5-ALA shows a linear release till day 7 with a release rate of 4.03 mg/day followed by a slow release leading to plateauing after 12 days with a maximum release extent of 42.40 mg (94.22 %) (Figure **21****).**

**Figure 21** shows active release of F27 (30/O/PEO200K/A - 202S/0.4/ND). Formulation composition described in **Table 3.** The plotted points represent the mean ± SD (n = 3)

### F29: 30/O/PEO200K/A - 202S/0.5/ND

5-ALA shows a linear release till day 21 with a release rate of 0.78 mg/day followed by a slow release leading to plateauing after 7 days with a maximum release extent of 17.88 mg (39.73 %) (Figure **22****).**

**Figure 22** shows active release F29 (30/O/PEO200K/A - 202S/0.5/ND). Formulation composition described in **Table 3.** The plotted points represent the mean ± SD (n = 3)

**Table 10. Summarized Release Rate and Total amount of the drug released for optimised PLA formulations and PEO formulation.**

| **F#** | **F name** | Release rate (mg/day) | Total amount of 5-ALA release (mg) | No. of Days of linear release |
|---|---|---|---|---|
| **F3** | 70/G/PLGA502H/A - 203S/0.15/ND | 6.15 | 47.22 | 4 |
| **F4** | 70/G/PLGA502H/A - 202S/0.2/ND | 10.92 | 40.87 | 3 |
| **F10** | 70/G/PLGA502H/A - 203S/40/E-PEG400/0.2/ND | 6.64 | 57.89 | 8 |
| **F11** | 70/G/PLGA502H/A - 203S/0.2/10/D | 4.41 | 44.27 | 6 |
| **F14** | 70/G/PLGA502H/A - 203S/0.2/6/D | 4.96 | 54.37 | 10 |
| **F15** | 70/G/PLGA502H/A - 203S/0.2/8/D | 7.35 | 63.41 | 8 |
| **F20** | 30/L/203H/A - 203H/0.2/ND | 1.70 | 20.77 | 8 |
| **F21** | 2x-30/L/203H/A - 203H/0.2/ND | 3.11 | 31.51 | 8 |
| **F22** | 2x-30/L/203H/A - 203H/0.3/ND | 1.33 | 35.15 | 25 |
| **F24** | 27/203H/A/10/C - 203H/0.2/ND | 2.69 | 26.84 | 8 |
| **F25** | 27/203H/A/10/F - 203H/0.2/ND | 2.32 | 22 | 4 |
| **F27** | 30/O/PEO200K/A - 202S/0.4/ND | 4.03 | 42.40 | 7 |
| **F29** | 30/O/PEO200K/A - 202S/0.5/ND | 0.78 | 17.88 | 21 |

### Example 4: Controlled release of Reference formulations

Monolithic system release testing was conducted on various formulations (R203 S, R502 H, R757 S, and R858 S) to assess the suitability of PLGA and poly(lactic acid) PLA matrices for 5-ALA delivery (Figure **23****).** Results indicated that the maximum amount of active released from the formulations was **0.30 mg.** Release plateaued within the first 5 h, after which minimal additional drug release was observed. This limited release may be due to 5-ALA becoming trapped within the PLGA structure, as confirmed by SEM micrographs of the cross-sectional structure. Consequently, a simple monolithic PLGA and PLA implant design is not suitable for achieving the desired sustained release of 5-ALA.

**Figure 23** shows active release of dip coated core-shell systems produced via film casting. MF 1-4 are described in Table 1. The plotted points represent the mean ± Standard Deviation (SD) (n = 3)

The fabrication process was successfully completed, with core formed accurately. The core exhibited a uniform mixture of polymer and 5-ALA with consistent weight and thickness measurements.

### PLGA Cores Fabricated Using the Vacuum Compression Method

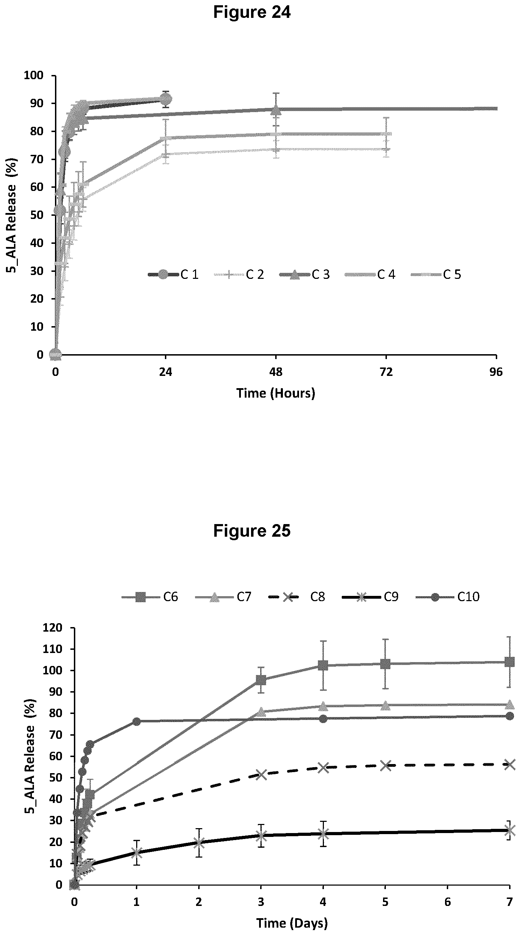

The rates and extents of 5-ALA release from the PLGA cores were analysed and are depicted in the figure below. Initial release profiles demonstrate a rapid burst release phase, observed within the first few hours, followed by a slower, sustained release phase. Among the different samples (C1-C5), variations in release rates were noted, with some formulations achieving near-complete release early on, while others displayed a more gradual and sustained release profile. The release kinetics indicate the influence of polymer composition and core fabrication parameters on the release behaviour (Figure **24****).**

**Figure 24** shows the release of 5-ALA from PLGA based Cores with 70% drug loading with polymers 757S (C2), and 858S (C5), also 50% drug loading with polymers 757S (C1), 502H (C3), 858S (C4). All formulations (C1-C5) are described in Table.2. The plotted points represent the mean ± SD (n = 3)

The release profiles of 5-ALA from the PLA cores (C6, C7, C8, C9, and C10) over a 7-day period show notable differences in both rates and extents of release. C10 exhibits the fastest release, reaching approximately 75% by day 1, followed by a plateau. C6 shows a moderate release, achieving around 100% within 4 days with little change thereafter. In contrast, C8 displays a slower, sustained release, gradually reaching about 50% by day 4 and stabilizing. C9 demonstrates the slowest release, reaching only ~20% by day 7, indicating a more controlled diffusion process. Meanwhile, C7 presents an initial burst release, achieving ~70% within the first 24 hours, followed by a plateau phase. These trends highlight significant differences in release kinetics across the formulations, suggesting that core composition and design influence the rate and extent of 5-ALA release **(****Figure 25****).**

**Figure 25** shows the release of 5-ALA from PLA based Cores. The core has 25% 5-ALA loading with polymer 202S (C9), 30% 5-ALA loading with polymers 203H (C6), 203s (C7), 202S (C8), and 40% 5-ALA loading with 203H (C10). All the formulations from C6-C10 are described in Table.4. The plotted points represent the mean ± SD (n = 3).

### Example 5: Mouse studies

### Materials and Methods

### Patient-derived xenograft (PDX) model.

Patients with malignant brain tumors and/or their legal representatives treated at the Medical University of Vienna gave preoperative informed consent to participate in the study in all cases. The study was approved by the local institutional review board (IRB) of the Medical University of Vienna (EK Nr. 1244/2016, EK Nr: 1616/2020) according to the guidelines of the Helsinki Declaration developed by the World Medical Association and the Department of Health and Human Services Belmont Report. The patient-derived brain tumor model VBT529 was derived from an aggressive glioblastoma and established at the Medical University of Vienna, Austria. VBT529 cells were classified as BSL 1 being tested negative at IDEXX for Hepatitis B/C and HIV 1/2. Cells were transduced with a lentiviral vector containing Luc2 (Luciferase), iRFP (infra-red fluorescent protein) and BlaS (blasticidin resistance gene). Following a two-week selection period on Blasticidin cells were cultivated in RPMI-1640 supplemented with 10 % fetal bovine serum, 1 % L-Glutamin and 1 % PenStep and grown at 37°C and 5% CO₂.

Mice were maintained, examined, and euthanized in accordance with institutional animal care guidelines and ethical animal license protocols approved by the legal authorities. Experimental animals were purchased from Charles River and housed at the Institute of Molecular Biotechnology (IMBA, Vienna, Austria) or the division of Biomedical Research (Medical University of Graz, Austria), in a 12-h light/dark cycle, with food and water ad libitum. Only female Crl:NU(NCr)-Foxn1<nu> (Nude) mice purchased from Charles River group housed in individually ventilated cages were used for all experiments described.

Animals were randomly assigned to experimental groups and checked daily by veterinary staff. For orthotopic PDX, mice were anesthetized and restrained in a stereotaxic frame. The body temperature was monitored with a rectal thermometer and kept constant at 36°C by a heating pad. The skull was exposed, cleaned, and a small hole was drilled. 1,5 x 10⁵ VBT529 cells were injected at coordinates: AP-1, ML+2, DV-3. To prevent backflow, the needle was left at the injection site for 6 min after the injection was finished. Mice were left to recover for at least 1 week after the surgery and during that time their drinking water was supplied with Baytril (Bayer) and Rimadyl (Pfizer). 5-ALA (5-aminolevulinic acid hydrochloride, HY-N0305, MedchemExpress) was dissolved in water, and ARS (Artesunate, PHR2573, Sigma) was dissolved in 5 % NaHCO₃ (8551.1, Roth) and 0.9 % NaCl (3570130, B. Braun). 5-ALA and Artesunate were administered at the concentrations and timepoints indicated in the figure legends. Tumor growth was monitored using the IVIS Spectrum machine (Caliper Life Sciences). Mice were anesthetized using an isoflurane chamber. Visualization of the tumor cells expressing luciferase was achieved by retroorbital injection of 100 µL D-luciferin (15 mg/mL dissolved in PBS; Goldbio, LUCK-1G) and luminescence data were analyzed using the Living Image software.

### Patient-derived xenograft (PDX) model using cOFM probe.

Animals were anesthetized by inhalation narcosis (Isoflurane, 3 % in 1.9 L/h O₂). Pain treatment was maintained by Fentanyl (5 µg/kg, i.p.) and Carprofen (5 mg/kg, s.c.). After shaving the surgical area, animals were placed in stereotaxic equipment and the skull was exposed by a midline incision. Three holes for probe implantation (coordinates: AP 0,6/ML 2/ DV 4) and one hole for the anchor screw were drilled. Dura mater was opened using a hypodermic needle (30 Ga) and the cOFM (cerebral open flow microperfusion) probe was lowered into the brain at approx. 1 mm/min using the stereotaxic equipment. The surgical area was covered by dental cement. Finally, animals were treated with the antibiotic Enrofloacin (7.5 mg/kg, s.c.) and returned to their home cage for recovery.

VBT529 cells were injected on day 14 post cOFM probe implantation. Mice were anesthetized using an isoflurane chamber. A cOFM infusion insert was connected to the pump tubing and the cell suspension was aspired through the cOFM infusion insert (1,5 x 10⁵ VBT529 cells in 3 µl). The cOFM healing dummy was replaced with the cOFM infusion insert and cells were injected at a flow rate of 1 µl/min using a precision pump. After complete infusion of 3 µl cell suspension, the cOFM infusion insert was kept in place for at least 1 additional minute to avoid back flow out of the cOFM cannula after disconnection of the cOFM infusion insert. The cOFM infusion insert was replaced with the cOFM healing dummy and animals were transferred back to their home cage. Animals were closely monitored until wake-up and body temperature was maintained using a warming blanket. For intratumoral drug application, animals were anesthetized using an isoflurane chamber. Compounds were aspired through the cOFM infusion insert. The cOFM healing dummy was replaced with the cOFM infusion insert and compounds were infused at a flow rate of 1 µl/min using a precision pump. After complete infusion the cOFM infusion insert was kept in place for 1 additional minute to avoid back flow. The cOFM infusion insert was replaced by the cOFM healing dummy and animals were transferred back to their home cage. Tumor growth was monitored using the LAGO device (spectral instruments imaging, Bruker). Mice were anesthetized using an isoflurane chamber. Visualization of the tumor cells expressing luciferase was achieved by i.p. injection of 100 µL D-luciferin (15 mg/mL dissolved in PBS; Goldbio, LUCK-1G) and luminescence data were acquired.

**Blood cell counts.** Mouse blood samples were collected into micro tubes containing 1,6 mg/mL EDTA (41.1504.015, Sarstedt) and blood cell counts were performed on scil Vet abc animal blood counter.

**Serum Analysis IDEXX.** Mouse blood samples were collected into Microtainer SST blood collection tubes (365968, Becton Dickinson) and serum samples were sent to IDEXX BioAnalytics (70806 Kornwestheim, Germany) for rodent expanded tox panel analysis.

**Statistical analysis.** All data are expressed as mean ± standard error of the mean (SEM). Statistical significance was tested by 2-way ANOVA followed by Bonferroni's post hoc test; Mantel-Cox test or Student's unpaired t-test followed by Bonferroni's post hoc test. All figures and mouse statistical analyses were generated using Prism 10 (GraphPad). Details of the statistical tests used are stated in the figure legends. In all figures, statistical significance is represented as *p < 0.05, **p < 0.01, ****p < 0.0001.

### Example 5A: Intracranial 5-aminolevulinic acid (5-ALA) bolus treatments combined with oral Artesunate (ARS) show a positive trend on tumor growth restriction but lead to systemic adverse events.

The inventors sought to identify an application scheme of 5-aminolevulinic acid (5-ALA) in combination with Artesunate (ARS) for the effective and safe treatment of brain tumors. Therefore, mice were equipped with a cerebral open flow microperfusion (cOFM) device, which allows frequent intracranial injections. Next, patient derived glioblastoma cells (VBT529) were implanted into the mouse brain via the cOFM device (Fig. 27A). 8 days post implantation, animals were treated with an intracranial 5-ALA bolus (via cOFM device) combined with oral ARS treatment on every weekday for 4 consecutive weeks (Fig. 27A). Body weight and tumor volume (radiance of LAGO imaging) were monitored. While the treatment had a positive, but non-significant antineoplastic effect, the intracranial 5-ALA bolus had to be reduced from 100 to 50 µg per day and the treatment showed adverse effects on body weight and animal survival (Fig. 27B-D).

### Example 5B: Constant intracranial 5-ALA release combined with oral ARS shows strong antitumor activity and is well tolerated.

To improve efficacy and reduce adverse side effects the inventors set out to study a constant low-dose intracranial 5-ALA release method in mouse xenograft models. Patient derived tumor cells (VBT529) were injected into the brain of nude mice and a slow-release osmotic pump (Alzet) loaded with 5-ALA was installed to allow constant release of 5-ALA into the brain over a period of 1 month (Fig. 28A). The continuous 5-ALA treatment was combined with per oral ARS dosing on weekdays and body weight as well as tumor volume were monitored. A third group was treated with 5-ALA and ARS systemically (i.p.) on weekdays for 4 weeks at doses close to the highest tolerated systemic exposures. Both treatment regiments showed strong antineoplastic activity and had no impact on body weights (Fig. 28B,C). Surprisingly, constant intracranial 5-ALA combined with oral ARS treatment showed the strongest antineoplastic effects despite the very low 5-ALA dose of 50 µg per day compared to 120 mg/kg in the systemic group.

### Example 5C: The combination of constant intracranial 5-ALA release combined with oral Artesunate shows no effect on body weight, blood cell parameters and serum tox parameters in healthy mice.

The combination of constant intracranial 5-ALA release combined with oral ARS was tested in healthy animals where no tumor cells were implanted. Animals were treated for 1 month with a constant intracranial 5-ALA release (via Alzet osmotic pump) combined with 23 days of per oral ARS treatment on weekdays (Fig. 29A). Body weight was monitored daily, blood samples for blood cell parameters were collected before- and on days 10, 17, 24, and 29 post treatment start, and serum was analyzed at the end of the study. Continuous intracranial 5-ALA treatment combined with per oral ARS had no effect on body weight, blood cell parameters, and toxicological markers assessed in serum demonstrating the safety of this new application scheme (Fig. 29B-D).

### Example 5D: Different intracranial constant release 5-ALA and oral ARS dose combinations prevent tumor growth in mice, while being well tolerated.

Patient derived tumor cells (VBT529) were injected into the brain of nude mice and slow-release osmotic pumps (Alzet) loaded with 5-ALA were installed to allow constant release of 5-ALA into the mouse brain over a period of 1 month (Fig. 30A). The continuous 5-ALA treatment was combined with per oral ARS dosing on weekdays for 3 weeks, testing two 5-ALA and two ARS concentrations. Body weight as well as tumor volume were monitored. All tested 5-ALA/ARS combinations showed strong antineoplastic activity while not effecting animal body weights (Fig. 30B, C). Surprisingly, even the extremely low 5-ALA dose of 10 µg per day significantly reduced tumor growth in combination with oral ARS treatment.

### Example 5E: Constant intracranial 5-ALA release alone significantly reduces brain tumor growth.

To assess the antineoplastic activity of 5-ALA alone, patient derived tumor cells (VBT529) were injected into the brain of nude mice and a slow-release osmotic pump (Alzet) loaded with 5-ALA was installed to allow constant release of 5-ALA into the brain over a period of 1 month (Fig. 31A). Body weight as well as tumor volume were monitored. The body weight remained unaffected by the 5-ALA treatment, but tumor growth was significantly reduced indicating that constant intracranial 5-ALA release alone has antineoplastic activity (Fig. 31B, C).

### Example 5F: Constant intracranial 5-ALA release alone slightly reduces tumor growth, while the combination with oral ARS shows strong antineoplastic activity.

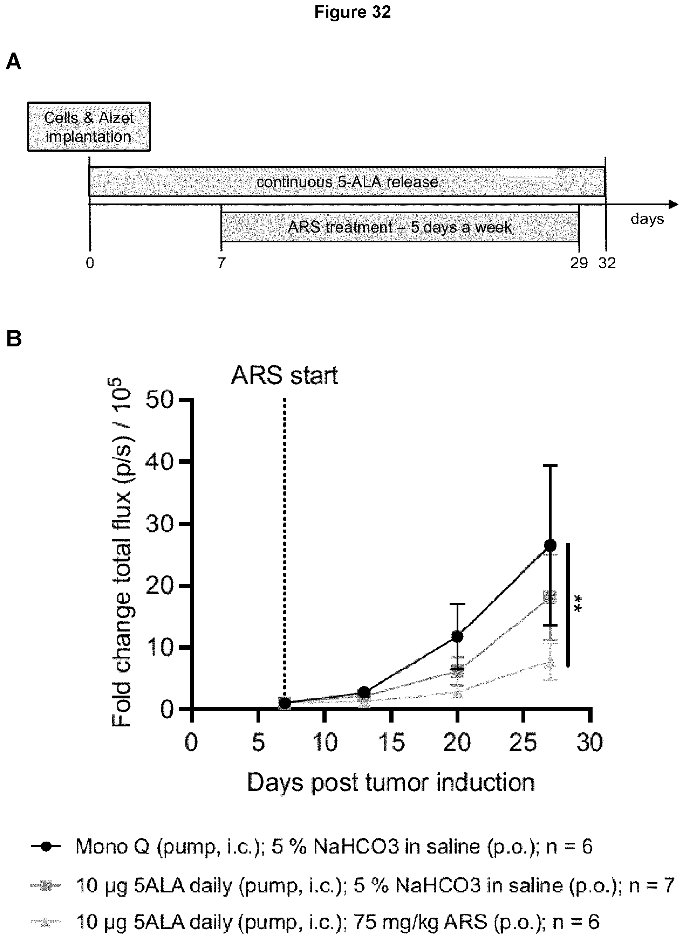

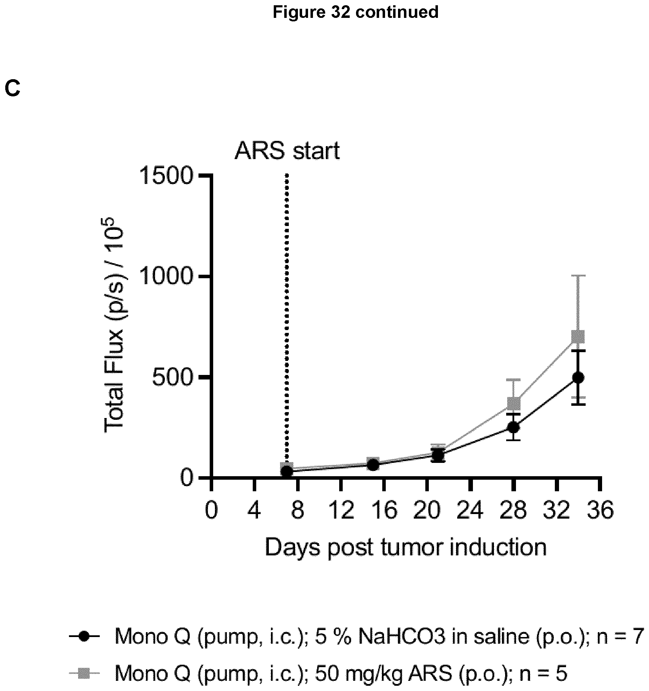

Patient derived tumor cells (VBT529) were injected into the brain of nude mice and slow-release osmotic pumps (Alzet) loaded with 5-ALA were installed to allow constant release of 5-ALA into the brain over a period of 1 month (Fig. 32A). In one group, the 5-ALA treatment was combined with per oral Artesunate treatment. In parallel, the effect of only ARS treatment on tumor growth was tested. Constant, low-dose intracranial 5-ALA release showed mild, but non-significant antitumor activity, which was boosted by the combination with per oral ARS treatment (Fig. 32B). ARS treatment alone did not limit tumor growth in this setup, highlighting the synergistic effects of the combination of constant 5-ALA release with per oral ARS (Fig. 32C).

## Claims

1. A brain implant for linear release of 5-aminolevulinic acid comprising
a) a core comprising:
5-aminolevulinic acid and a core polymer,
wherein the core polymer is selected from the group consisting of poly(lactic acid), poly(ethylene oxide), poly(ethylene glycol), and poly(lactic-co-glycolic acid);
and
b) a shell consisting of an upper membrane, a spacer, and a lower membrane, wherein the upper membrane, the spacer, and the lower membrane each comprise a shell polymer, wherein the shell polymer is poly(lactic acid);
**characterized in that**
the shell polymer is in a range from 20 wt.% to 60 wt.% based on the total weight of the brain implant; and
when the core polymer is poly(lactic acid), poly(ethylene oxide), poly(ethylene glycol), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid), the core contains 5-aminolevulinic acid in a range from 65 wt.% to 75 wt.% and the core polymer in a range from 35 wt.% to 25 wt.% based on the total weight of the core.

2. The brain implant according to claim 1, wherein the shell further comprises 5-aminolevulinic acid in a range from 1 wt.% to 15 wt.% based on the total weight of the shell.

3. The brain implant according to claim 1 or 2, further comprising an excipient in the core, or in the shell, wherein
the excipient in the core is selected from the group consisting of citric acid, fumaric acid, glycolic acid, lactic acid, malic acid, tartaric acid, maleic acid, succinic acid and a mixture of two or more of these acids; and/or
an antioxidant selected from ascorbic acid, acetylcysteine, cysteine, thioglycerol, sodium hydrogen sulfite, butylated hydroxyanisole, butylated hydroxytoluene, α-tocopherol acetate, methionine, citric acid, ethylenediaminetetraacetic acid, tartaric acid, gallic acid and its esters, glutathione, uric acid, carotenoids, and polyphenols; and/or
the excipient in the shell is selected from polyethylene glycol.

4. The brain implant according to claim 3, wherein
the excipient in the core is in a range from 5 wt.% to 15 wt.%, based on the total weight of the core; or
the excipient in the shell is in a range from 35 wt.% to 45 wt.%, based on the total weight of the shell.

5. The brain implant according to any one of claims 1 to 4, wherein the core is in a range from 40 wt.% to 75 wt. %, preferably 45 wt.% to 70 wt.% based on the total weight of the brain implant.

6. The brain implant according to any one of claims 1 to 5, consisting of:
a) the core consisting of:
5-aminolevulinic acid in a range from 25 wt.% to 75 wt.%,
the core polymer in a range from 75 wt.% to 25 wt.%, and
optionally the excipient in the core in a range of 5 wt.% to 15 wt.%, based on the total weight of the core,
wherein the excipient in the core is citric acid, fumaric acid or a mixture thereof; and
b) the shell consisting of:
the upper membrane, the spacer, and the lower membrane each comprising a shell polymer in a range from 55 wt.% to 100 wt.%, and
optionally, 5-aminolevulinic acid in a range from 1 wt.% to 15 wt.%, or
optionally, the excipient in the shell in a range from 35 wt.% to 45 wt.% based on the total weight of the shell,
wherein the excipient in the shell is polyethylene glycol 400.

7. The brain implant according to any one of claims 1 to 6,
wherein
the brain implant consists of:
a) the core consisting of:
65 wt.% to 75 wt.% of 5-aminolevulinic acid, and 35 wt.% to 25 wt.% of poly (lactic-co-glycolic acid), based on a total weight of the core; and
b) the shell consisting of poly(lactic acid);
wherein in the brain implant
poly(lactic acid) is in a range from 40 wt.% to 50 wt.%,
based on a total weight of the brain implant;
or
the brain implant consists of:
a) the core consisting of:
25 wt.% to 45 wt.% of 5-aminolevulinic acid and 75 wt.% to 55 wt.% of poly(lactic acid), based on a total weight of the core; and
b) the shell consisting of poly(lactic acid);
wherein in the brain implant
poly(lactic acid) is in a range from 75 wt.% to 85 wt.%,
based on a total weight of the brain implant;
or
the brain implant consists of:
a) the core consisting of:
25 wt.% to 35 wt.% of 5-aminolevulinic acid, and 75 wt.% to 65 wt.% of poly(ethylene oxide), based on a total weight of the core; and
b) the shell consisting of poly(lactic acid);
wherein in the brain implant
poly(lactic acid) is in a range from 45 wt.% to 55 wt.%,
based on a total weight of the brain implant;
or
the brain implant consists of:
a) the core consisting of:
65 wt.% to 75 wt.% of 5-aminolevulinic acid, and 35 wt.% to 25 wt.% of poly (lactic-co-glycolic acid), based on a total weight of the core; and
b) the shell consisting of:
5 wt.% to 15 wt.% of 5-aminolevulinic acid, and 95 wt.% to 85 wt.% of poly(lactic acid) based on a total weight of the shell;
wherein in the brain implant
poly(lactic acid) is in a range from 35 wt.% to 45 wt.%,
based on a total weight of the brain implant;
or
the brain implant consists of:
a) the core consisting of:
25 wt.% to 30 wt.% of 5-aminolevulinic acid, 5 wt.% to 15 wt.% of citric acid or fumaric acid, and 55 wt.% to 70 wt.% of poly(lactic acid) based on a total weight of the core; and
b) the shell consisting of poly(lactic acid);
wherein in the brain implant
poly(lactic acid) is in a range from 75 wt.% to 85 wt.%,
based on a total weight of the brain implant;
or
the brain implant consists of:
a) the core consisting of:
65 wt.% to 75 wt.% of 5-aminolevulinic acid, and 35 wt.% to 25 wt.% of poly (lactic-co-glycolic acid) based on a total weight of the core; and
b) the shell consisting of:
35 wt.% to 45 wt.% of polyethylene glycol 400, and 65 wt.% to 55 wt.% of poly(lactic acid) based on a total weight of the shell;
wherein in the brain implant
poly(lactic acid) is in a range from 25 wt.% to 35 wt.%,
based on a total weight of the brain implant;
or
the brain implant consists of:
a) the core consisting of:
25 wt.% to 45 wt.% of 5-aminolevulinic acid, and 75 wt.% to 55 wt.% of poly(lactic acid) or poly(ethylene glycol) or poly(ethylene oxide) based on the total weight of the core; or
65 wt.% to 75 wt.% of 5-aminolevulinic acid, and 25 wt.% to 35 wt.% poly(lactic-co-glycolic acid) based on the total weight of the core;
and
b) the shell consisting of poly(lactic acid);
wherein in the brain implant poly(lactic acid) is in a range from 20 wt.% to 60 wt.%, based on a total weight of the brain implant.

8. The brain implant according to any one of the claims 1 - 7, wherein
the upper membrane has a height (h₂) in a range from 0.05 mm to 0.60 mm, preferably 0.10 mm to 0.50 mm; and
the lower membrane has a height (h₃) in a range from 0.05 mm to 0.60 mm, preferably 0.10 mm to 0.50 mm.

9. A brain implant for linear release of 5-aminolevulinic acid **characterized in that** the brain implant releases 5-aminolevulinic acid linearly over at least 3 days in an amount of 1 mg to 30 mg into cavities emerging from a brain surgery.

10. The brain implant according to any one of the claims 1 - 9 for use in the prophylaxis and/or treatment of brain cancer.

11. The brain implant for use according to claim 10, wherein the brain cancer is selected from subtypes proneural, mesenchymal, and classical glioblastoma, pilocytic astrocytoma, pleomorphic xanthoastrocytoma, subependymal giant cell astrocytoma, fibrillary astrocytoma, anaplastic astrocytoma, oligodendrogliomas, anaplastic oligodendroglioma, ependymoma, subependymoma, choroid plexus tumor, choroid plexus papilloma, and choroid plexus carcinoma.

12. The brain implant for use according to claim 10 or 11 in a combination with one of the following artemisinin compounds **(1a** - **1e)** or a pharmaceutically acceptable salt thereof 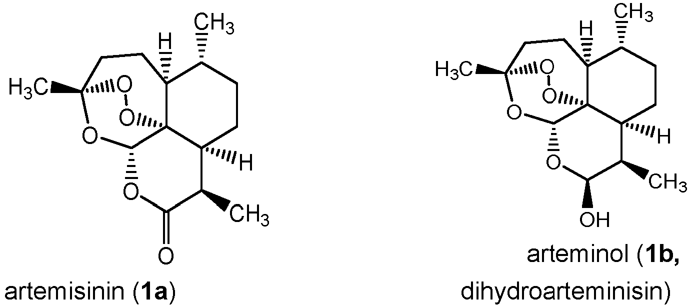 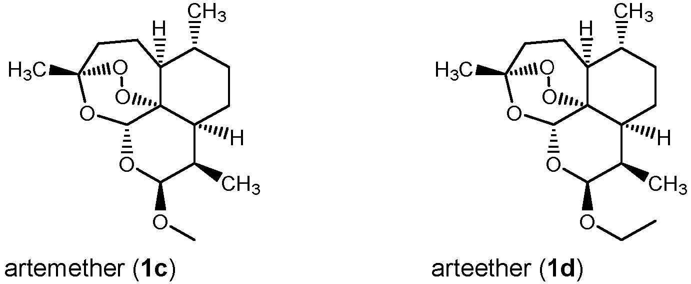 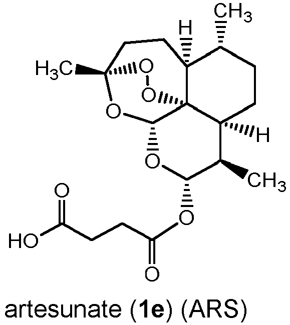 and preferably with artesunate **(1e).**

13. The brain implant for use according to any one of the claims 10 - 12, in combination with a radiotherapy, photodynamic therapy, immunotherapy, electromagnetic field therapy, hyperthermia therapy, chemotherapy, cancer immunotherapy, surgical therapy and/or cellular therapy such as Car-T and TIL, preferably radiotherapy, photodynamic therapy, and chemotherapy.

14. A method for producing the brain implant in a form of an implant according to claim 1, comprising:
Step 1) Preparing a core comprising 5-aminolevulinic acid and a core polymer by using vapour control module D10 mm chamber,
wherein the core polymer is selected from the group consisting of poly(lactic acid), poly(ethylene oxide), poly(ethylene glycol), and poly(lactic-co-glycolic acid);
Step 2) preparing an upper membrane of a shell comprising a shell polymer by using a vapour control module D20 mm chamber and heating the upper membrane at 120°C;
Step 3) preparing a vessel of the shell comprising the shell polymer by using a vapour control module D20 mm chamber and heating the formed vessel at 120°C, wherein the vessel consisting of a spacer and a lower membrane; and
Step 4) inserting the core prepared in the step 1) into the vessel of the shell prepared in the step 3 and closing the core in the vessel with the upper membrane of the shell prepared in the step 2) and applying a predetermined pressure to seal the upper membrane and the vessel of the shell and heating a formed tablet at 130°C,
**characterized in that**
the shell polymer is in a range from 25 wt.% to 55 wt.% based on the total weight of the brain implant; and
when the core polymer is poly(lactic acid), poly(ethylene oxide), poly(ethylene glycol), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid), the core contains 5-aminolevulinic acid in a range from 65 wt.% to 75 wt.% and the core polymer in a range from 35 wt.% to 25 wt.%, based on the total weight of the core;
or
Step 1') preparing a coextruded strand consisting of a core material and a spacer material by using a hot melt co-extrusion device,
wherein the core material comprises 5-aminolevulinic acid and a core polymer; and the spacer material comprises a shell polymer;
wherein the core polymer is selected from the group consisting of poly(lactic acid), poly(ethylene oxide), poly(ethylene glycol), and poly(lactic-co-glycolic acid); and
the shell polymer is poly(lactic acid);
Step 2') cutting the coextruded strand of step 1') to obtain a core coated with a spacer;
Step 3') preparing an upper membrane and a lower membrane from a shell material comprising the shell polymer;
Step 4') sealing the core coated with the spacer of step 2') with the upper membrane and the lower membrane of step 3') by applying a predetermined pressure at a predetermined temperature;
**characterized in that**
the shell polymer is in a range from 25 wt.% to 55 wt.% based on the total weight of the brain implant; and
when the core polymer is poly(lactic acid), poly(ethylene oxide), poly(ethylene glycol), the core contains 5-aminolevulinic acid in a range from 25 wt.% to 45 wt.%, and the core polymer in a range from 75 wt.% to 55 wt.% based on the total weight of the core; or
when the core polymer is poly(lactic-co-glycolic acid), the core contains 5-aminolevulinic acid in a range from 65 wt.% to 75 wt.% and the core polymer in a range from 35 wt.% to 25 wt.%, based on the total weight of the core.

15. The method according to claim 14, wherein
in the step 1)
the core further comprises an excipient in a range from 5 wt.% to 15 wt.% based on the total weight of the core, and
the excipient in the core is selected from the group consisting of citric acid, fumaric acid, glycolic acid, lactic acid, malic acid, tartaric acid, maleic acid, succinic acid and a mixture thereof;
or
in the steps 2) and 3)
the upper membrane and the vessel of the shell each further comprise 5-aminolevulinic acid in a range from 1 wt.% to 15 wt.% based on the total weight of the shell; or
the upper membrane and the vessel of the shell each further comprise an excipient in a range from 35 wt.% to 45 wt.%, based on the total weight of the shell, and
the excipient in the shell is selected from polyethylene glycol;
or
in the step 1')
the core material further comprises an excipient in a range from 5 wt.% to 15 wt.% based on the total weight of the core, and
the excipient in the core is selected from the group consisting of citric acid, fumaric acid, glycolic acid, lactic acid, malic acid, tartaric acid, maleic acid, succinic acid and a mixture thereof;
or
in the step 3')
the shell material further comprise 5-aminolevulinic acid in a range from 1 wt.% to 15 wt.% based on the total weight of the shell material; or
the shell material further comprise an excipient in a range from 35 wt.% to 45 wt.%, based on the total weight of the shell material, and
the excipient in the shell is selected from polyethylene glycol.
